# EUROPEAN PATENT APPLICATION

(11) **EP 1 288 297 A1**
(43) Date of publication of application: **05.03.2003**
(21) Application number: 01930205.8
(22) Date of filing: 18.05.2001
(51) Int. Cl.: C12N 15/12, C12N 5/10, A61K 38/46, A61K 39/395, A61K 48/00, A61P 35/00, A61P 43/00, C07K 14/81, C07K 16/38, C12P 21/02, C12Q 1/02, C12Q 1/37, G01N 33/15, G01N 33/50

(54) **REGULATION OF MT1-MMP ACTIVITY**

(30) Priority: 19.05.2000 JP 2000147739
(71) Applicant: Fujichemico Ltd., Takaoka-shi, Toyama 933-0951 (JP)
(72) Inventor: SEIKI, Motoharu, Shinagawa-ku, Tokyo 142-0061 (JP); OBATA, Ken-ichi, Tonami-shi, Toyama 939-1368 (JP)
(74) Representative: von Kreisler, Alek, Dipl.-Chem.
(86) International application number: JP0104166
(87) International publication number: WO01088131

(57) **Abstract**

It has been considered that an activation of proMMP-2 induced by MT1-MMP is one of the important steps for invasion of tumor cells such as cancers, but its detailed mechanism for an expression of activation has been unknown. Thus, it has been required that methods for preventing/treating various diseases are developed by elucidating the mechanism of activation expression of proMMP-2 induced by MT1-MMP and elucidating various actions potentially involving MT1-MMP in addition to invasion and metastasis of cancers. A formation of a complex formed of multiple MT1-MMP, particularly a homodimer formation of MT1-MMP is crucial for the activation of proMMP-2 induced by MT1-MMP, and the homodimer formation is based on functions of the PEX domain of MT1-MMP. Utilizing such findings, the homodimer formation of MT1-MMP is inhibited and the activation of proMMP-2 is inhibited leading to developing methods to control invasion/metastasis of cancers.

## Description

### Field of the Invention

The present invention relates to substances which inhibit the formation of complexes comprised of a plurality of membrane-type 1 matrix metalloproteinases (MT1-MMPs). Particularly, the present invention relates to substances which inhibit the homophilic dimer formation of MT1-MMP present on a cell surface.

The present invention relates to methods for controlling various physiological and biological processes attributable to the activation of proMMP-2, which comprise inhibiting the homophilic dimer formation of MT1-MMP thereby inhibiting the activation of proMMP-2 and to pharmaceutical drugs therefor. The present invention further provides pharmaceuticals and therapeutic methods for the inhibition of cancer invasion and metastasis based on regulation mechanisms of MT1-MMP activity via the hemopexin-like (PEX) domain of MT1-MMP. More specifically, the present invention relates to MT1-MMP complex formation inhibitors, proMMP-2 activation inhibitors, blockers for cell migration, invasion and/or metastasis, associated with the homophilic dimer formation of MT1-MMP. Further, the present invention relates to methods for inhibiting the homophilic dimer formation of MT1-MMP and related methods, to substances (such as MT1-PEX) having an inhibitory activity on the homophilic dimer formation of MT1-MMP present on a cell surface, to nucleic acids coding for the same and uses of said nucleic acids. The present invention also relates to antibodies against the PEX protein of MT1-MMP or fragments thereof or polypeptides having substantially equivalent activity as compared to the same and uses thereof. The present invention relates to screening methods for inhibitory substances against the homophilic dimer formation of MT1-MMP and reagents therefor.

Matrix metalloproteinases (MMPs) are of the zinc-dependent endopeptidase family which degrade various constitutive proteins at extracellular matrices (ECM) and basement membrane components. These enzymes are involved in rearrangement of connective tissues such as normal embryonic development, bone growth or wound healing and the like (Woessner, J. F., FASEB J., 5:2145-2154, 1991; Matrisian, L. M., BioEssays, 14:455-463, 1992; Birkedal-Hansen, H., Moore, W. G. I., Bodden, M. K., Windsor, L. J., Birkedal-Hansen, B., DeCarlo, A., and Engler, J. A., Crit. Rev. Oral Biol. Med., 4:197-250, 1993). It has become known that these are also involved in various processes of diseases such as atherosclerosis (Henney, A. M., Wakeley, P. R., Davies, M. J., Foster, K., Hembry, R., Murphy, G., and Humphries, S., Proc. Natl. Acad. Sci. USA, 88:8154-8158, 1991), pulmonary emphysema (Hautamaki, R. D., Kobayashi, D. K., Senior, R. M., and Shapiro, S. D., Science, 277:2002-2004, 1997), rheumatoid arthritis (Murphy, G., and Hembry, R. M., J. Rheumatol., 19:61-64, 1992), and invasion/metastasis of cancers (Stetler-Stevenson, W. G., Aznavoorian, S., and Liotta, L. A., Annu. Rev. Cell Biol., 9:541-573, 1993).

To the present, MMPs (MMP-1 (collagenase)); MMP-2 (gelatinase A); MMP-3 (stromelysin-1); MMP-7 (matrilysin); MMP-8 (neutrophil collagenase); MMP-9 (gelatinase B); MMP-10 (stromelysin-2); MMP-11 (stromelysin-3); MMP-12 (macrophage elastase); MMP-13 (collagenase-3); MMP-14 (MT1-MMP); MMP-15 (MT2-MMP); MMP-16 (MT3-MMP); MMP-17 (MT4-MMP); MMP-18 (collagenase-4); MMP-19; MMP-20 (enamelysin); MT5-MMP and the like) analyzed at an amino acid level have been known (Woessner, J. F., FASEB J., 5:2145-2154, 1991; Matrisian, L. M., BioEssays, 14:455-463, 1992; Birkedal-Hansen, H., Moore, W. G. I., Bodden, M. K., Windsor, L. J., Birkedal-Hansen, B., DeCarlo, A., and Engler, J. A., Crit. Rev. Oral Biol. Med., 4:197-250, 1993; Gururajan, R., Grenet, J., Lahti, J. M., and Kidd, V. J., Genomics, 52:101-106, 1998; Velasco, G., Pendas, A. M., Fueyo, A., Knauper, V., Murphy, G., and Lopez-Otin, C., J. Biol. Chem., 274: 4570-4576, 1999). These MMPs are classified into at least 4 types of subfamilies: collagenases, gelatinases, stromelysins and membrane-type MMPs (MT-MMPs) depending on their primary structures, substrate specificity and cellular distribution, and also can be broadly subgrouped into soluble MMPs and membrane-anchored MMPs (membrane-type MMPs, MT-MMPs). Soluble MMPs are believed to be related to ECM degradation in broad areas of tissues. On the other hand, MT-MMPs are believed to be related to ECM degradation surrounding cells because they are connected to the plasma membrane (Seiki, M., Apmis, 107:137-143, 1999). MMPs are composed of preserved domain structures of a pre/propeptide, a catalytic domain, a hinge, and a hemopexin-like domain (Nagase, H., et al., J. Biol. Chem., 274: 21491-21494, 1999).

MT-MMPs subfamilies have been most recently reported as a subclass of MMPs, and five types of members (MT1-MMP, MT2-MMP, MT3-MMP, MT4-MMP and MT5-MMP) have been isolated and identified by degenerated primers for the preserved domains of MMPs and RT-PCR (Sato, H., Takino, T., Okada, Y., Cao, J., Shinagawa, A., Yamamoto, E., and Seiki, M., Nature, 370:61-65, 1994; Will, H., and Hinzmann, B., Eur. J. Biochem., 231: 602-608, 1995; Takino, T., Sato, H., Shinagawa, A., and Seiki, M., J. Biol. Chem., 270:23013-23020, 1995; Puente, X. S., Pendas, A. M., Llano, E., Velasco, G., and Lopez-Otin, C., Cancer Res., 56:944-949, 1996; Japanese Unexamined Patent Publication No. 2000-270874; Pei, D., J. Biol. Chem., 274:8925-8932, 1999; Kajita, M. et al., FEBS Lett., 457: 353-356, 1999).

MT-MMPs are type I membrane proteins which have a single transmembrane domain and a short cytoplasmic tail following the hemopexin domain characteristics of many MMPs. Additionally, an insertion of basic amino acids is commonly present between the propeptide and an active domain.
Cleavage by a furin or furin-like enzyme induces the activation of these membrane proteins (Pei, D., and Weiss, S. J., J. Biol. Chem., 271:9135-9140, 1996; Sato H., Kinoshita, T., Takino, T., Nakayama, K., and Seiki, M., FEBS Lett., 393:101-104, 1996; Cao, J., Rehemtulla, A., Bahou, W., and Zucker, S., J. Biol. Chem., 271:30174-30180, 1996).

When cells migrate, invade and metastasize in the tissue, the degradation of extracellular matrices surrounding them is an essential step. Those playing a pivotal role in the step are the enzyme group called MMPs. Among others, MT1-MMP expressed on the surface of a cell membrane is given importance in its roles in migration, invasion, metastasis and angiogenesis of cancers.

MT1-MMP is the enzyme also called membrane-type 1 matrix metalloproteinase (MT1-MMP) or MMP-14 (MEROPS ID: M10,014), and one reported as the product of a gene occupying a chromosomal locus, 14q11-q12 in humans (Migon, C. et al., Genomics, 28:360-361, 1995) of which a detailed structure and profiles have been demonstrated by successful DNA cloning and recombinant protein expression thereof (Sato, H. et al., Nature, 370:61-65, 1994; Takino, T., et al., Gene, 155:293-298, 1995; Japanese Unexamined Patent Publication Nos. Hei-7-203961, Hei-7-303482; GenBank™ accession number: D26512). The presence of MT1-MMP has been detected in the dogs, goats, rabbits, wild boars, mice, etc., in addition to humans.
cDNA of human MT1-MMP encodes 582 amino acid residues (EMBL accession No. D26512, E09720 and E10297; SWISS-PROT: P50281), of which the structure is composed of a signal peptide followed by a propeptide domain, an insertion sequence composed of 10 specific amino acid residues similar to stromelysin-3 (a potential sequence of a furin-like enzyme recognition site), a core enzyme domain having a potential site with a zinc binding site, a hinge domain, and a hemopexin-like domain encompassing a transmembrane domain.

Thus, MT1-MMP has been thought to have a transmembrane (TM) domain at the C terminus and to exert its biological activity on the cell membrane. To the present, it has been found that MT1-MMP activates a progelatinase A (proMMP-2) which is also a member of the same family and a basement membrane degrading enzyme, on the cell membrane, and further that MT1-MMP per se degrades various ECM molecules such as collagen type I, II and III, fibronectin, laminin, vitronectin and aggrecan. Also, MT1-MMP has been shown to promote the processes of tumor invasion and metastasis (Seiki, M., Apmis, 107:137-143, 1999; Sato, H., et al., Nature, 370:61-65, 1994). MT1-MMP also activates the other MMPs such as proMMP-2 (Sato, H., et al., Nature, 370:61-65, 1994) and procollagenase-3 (proMMP-13) (Knauper, V., et al., J. Biol. Chem., 271:17124-17131, 1996).

Thus, the expression of MT1-MMP is thought to initiate multiple proteinase cascades on a cell surface. And it has been shown that MT1-MMP is involved in not only invasion and metastasis of cancer cells (Seiki, M., Apmis, 107:137-143, 1999; Sato, H., et al., Nature, 370:61-65, 1994), but also other physiological processes such as angiogenesis (Hiraoka, N., et al., Cell, 95:365-377, 1998; Zhou, Z., et al._{,} Proc. Natl. Acad. Sci. USA, 97:4052-4057, 2000) and skeletal development (Zhou, Z., et al., Proc. Natl. Acad. Sci. USA, 97:4052-4057, 2000; Holmbeck, K., et al., Cell, 99:81-92, 1999).

Thus, MT1-MMP is considered to be a tool required for physiological and pathological cellular invasion in tissue. Therefore, it is important to elucidate action mechanisms of MT1-MMP on a cell surface.

MT1-MMP has been shown to promote invasion of cancer cells by activating proMMP-2 and by directly degrading extracellular matrices. The activation of proMMP-2 on a cell surface by MT1-MMP appears to be an important step in tumor invasion. This is because MMP-2 degrades type IV collagen and laminin which are major components of the basement membrane (Stetler-Stevenson, W. G. et al., Annu. Rev. Cell Biol., 9:541-573, 1993). The occurrence of active MMP-2 is also significantly correlated with tumor invasion (Tokuraku, M. et al., Int. J. Cancer, 64:355-359, 1995; Nomura, H. et al., Cancer Res., 55:3263-3266, 1995). Its activation step includes the tri-molecular complex formation of MT1-MMP, TIMP-2 and proMMP-2 (Strongin, A. Y., et al., J. Biol. Chem., 270:5331-5338, 1995). However, the activity expression mechanisms of MT1-MMP on a cell membrane have not been ascertained in detail. Therefore, it has been unknown what should be done to strictly control the activity of MT1-MMP. Thus, there have been problems in that measures to control migration, invasion and metastasis of cells such as cancers cannot be demonstrated.

### Summary of the Invention

The present inventors have conducted an intensive study to uncover the elicitation mechanism of MT1-MMP activity. As a result, the inventors have successfully found that MT1-MMP forms a homo-dimer complex through the hemopexin-like (PEX) domain, and that the complex formation is essential for the activation of proMMP-2 on the cell surface. Furthermore, it has been also found that the expression of PEX without a catalytic site of MT1-MMP on a cell surface directly prevents an association of two enzymes and inhibits the activation of proMMP-2 in a dose-dependent manner. In addition, it has been found that the expression of PEX without the catalytic site of MT1-MMP suppresses invasion and metastasis of tumor cells. As a result, the inventors have completed the present invention.

The present invention provides:
(1) A membrane-type 1 matrix metalloproteinase (MT1-MMP) complex formation inhibitor which inhibits the formation of a complex comprised of plural MT1-MMPs.
(2) A proMMP-2 activation inhibitor which inhibits the formation of a complex comprised of plural MT1-MMPs.
(3) A blocker for cell migration, invasion and/or metastasis which inhibits the activation of proMMP-2 through inhibiting the formation of a complex comprised of plural MT1-MMPs.
(4) The blocker according to the above (3), wherein the cell is a cancer cell.
(5) The inhibitor or blocker according to any of the above (1) through (4), wherein the complex comprised of plural MT1-MMPs is an MT1-MMP homophilic dimer.
(6) The inhibitor or blocker according to any of the above (1) through (5), wherein the complex formation is executed at least with MT1-MMP existing on a cell surface.
(7) A method for inhibiting MT1-MMP complex formation which comprises inhibiting the formation of a complex comprised of plural MT1-MMPs
(8) A method for inhibiting the activation of proMMP-2 which comprises inhibiting the formation of a complex comprised of plural MT1-MMPs.
(9) A method for blocking cell migration, invasion and/or metastasis which comprises inhibiting the activation of proMMP-2 through inhibiting the formation of a complex comprised of plural MT1-MMPs.
(10) The method according to the above (9), wherein the cell is a cancer cell.
(11) The method according to any of the above (7) through (10), wherein the complex comprised of plural MT1-MMPs is an MT1-MMP homophilic dimer.
(12) The method according to any of the above (7) through (11), wherein the complex formation is executed at least with MT1-MMP existing on a cell surface.
(13) A substance which is active in inhibiting the homophilic dimer formation of MT1-MMP that exists on a cell surface.
(14) The substance according to the above (13) which is an MT1-MMP hemopexin-like domain (PEX) protein or a fragment thereof, or a substance which has substantially equivalent activity as compared to the same.
(15) The substance according to the above (14), wherein the substance is from Cys³¹⁹ to Gly⁵³⁵ of Met plus MT1-MMP, or a protein having substantially equivalent activity as compared to the same.
(16) A nucleic acid selected from the group consisting of:
   (i) a nucleic acid coding for MT1-MMP PEX;
   (ii) a nucleic acid coding for a fragment of MT1-MMP PEX;
   (iii) a nucleic acid hybridizable to the nucleic acid as set forth in the above (i); and
   (iv) a nucleic acid having substantially equivalent activity as compared to the nucleic acid as set forth in any of the above (i) through (iii).
(17) The nucleic acid according to the above (16), wherein the nucleic acid is a chimeric molecule in which the transmembrane (TM)/cytoplasmic (CP) domains of MT1-MMP are substituted with nerve growth factor receptor (NGFR) TM/CP, or a molecule having substantially equivalent activity as compared to the same.
(18) The nucleic acid according to the above (16), which is a nucleic acid coding for from Cys³¹⁹ to Gly⁵³⁵ of Met plus MT1-MMP, or a nucleic acid having substantially equivalent activity as compared to the same.
(19) A vector comprising a nucleic acid according to any one of the above (16) through (18).
(20) A transformed cell comprising (i) a nucleic acid according to any of the above (16) through (18) or (ii) a vector according to the above (18).
(21) The transformed cell according to the above (20) which expresses a chimeric molecule in which MT1-MMP TM/CP is substituted with NGFR TM/CP, or a molecule having substantially equivalent activity as compared to the same.
(22) A gene therapeutic agent which comprises (i) a nucleic acid according to any one of the above (16) or (18) or (ii) a vector according to the above (19).
(23) An antibody against (i) an MT1-MMP PEX protein or a fragment thereof, or (ii) a polypeptide having substantially equivalent activity as compared to the same.
(24) A substance capable of binding to MT1-MMP PEX.
(25) The substance according to the above (22) which has MT1PEX activity or substantially equivalent activity as compared to MT1PEX.
(26) A pharmaceutical or veterinary drug comprising the inhibitor or blocker according to any of the above (1) through (6), or the substance, nucleic acid, vector, transformed cell, agent or antibody according to any of the above (13) through (16), (18) through (20), and (22) through (25).
(27) The pharmaceutical or veterinary drug according to the above (26) for suppressing and/or blocking cell migration, invasion and/or metastasis.
(28) The pharmaceutical or veterinary drug according to the above (26), wherein the cell is a cancer cell.
(29) A screening which comprises screening with using, as a marker, the formation of a complex comprised of plural MT1-MMPs for a substance which inhibits the formation of said complex comprised of said plural MT1-MMPs
(30) The screening according to the above (29), wherein the complex comprised of the plural MT1-MMPs is an MT1-MMP homophilic dimer.
(31) The screening according to the above (29) or (30), wherein a chimeric molecule is used in which MT1-MMP transmembrane (TM)/cytoplasmic (CP) domains are substituted with nerve growth factor receptor (NGFR) TM/CP.
(32) A substance which (i) inhibits the formation of a complex comprised of plural MT1-MMPs and (ii) is obtained by the screening according to any of the above (29) through (31).

The above objectives and other objectives, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts anc other parts of the specification without departing from the spirit and scope thereof as disclosed herein. All of the patent publications and reference documents cited herein for illustrative purposes are hereby incorporated by reference into the present disclosure.

As used herein, the term "and/or" means that both (1) combined conjunction and (2) selective conjunction are present. For instance, in the case of "invasion and/or metastasis", it is used to mean that both (1) "invasion and metastasis" and (2) "invasion or metastasis" are included. In other cases, the term "and/or" is used to mean that both (1) combined conjunction and (2) selective conjunction are included in the same way.

### Brief Description of the Drawings

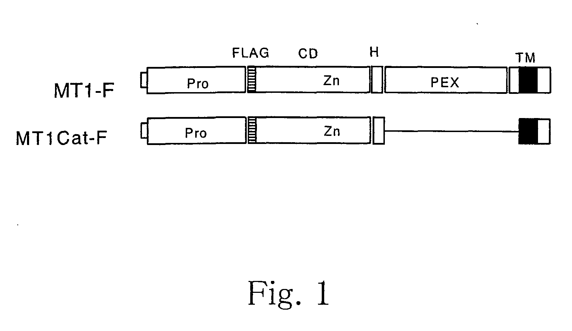

Fig. 1: A schematic illustration of MT1-MMP mutant constructs as used herein.

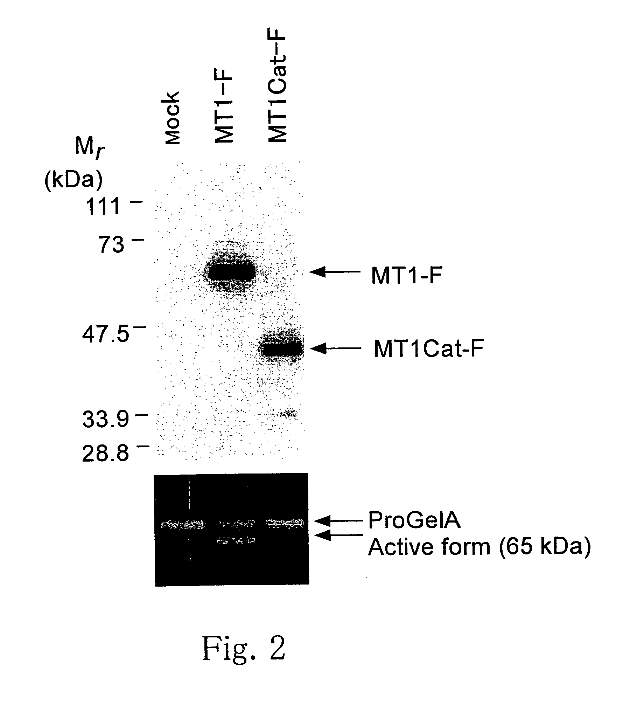

Fig. 2: Tests for the activation of proMMP-2 by MT1-F on a cell surface. COS1 cells were transfected with expression vectors for MT1-MMP mutants; MT1-F or MT1Cat-F or a vector alone (Mock), and then cultured in a serum-free culture medium in the presence of purified proMMP-2 (0.25 µ g/ml) at 37°C for 18 hrs. The resultant cell lysates were examined for the expression of mutants, and the culture supernatants were examined for the activation of proMMP-2. The upper panel indicates the Western blotting analysis with monoclonal anti-FLAG M2 antibody for cell lysates obtained from the transfected cells. The lower panel indicates the gelatin zymographic patterns for culture media.

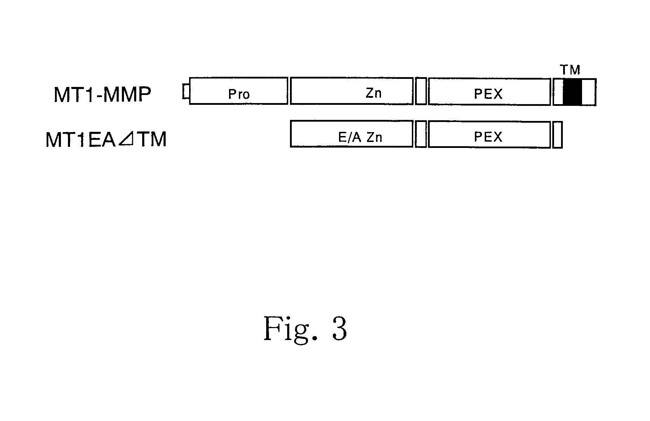

Fig. 3: A schematic illustration of MT1EAΔ TM expressed in E. coli. E/A indicates that Glu²⁴⁰ is mutated with Ala.

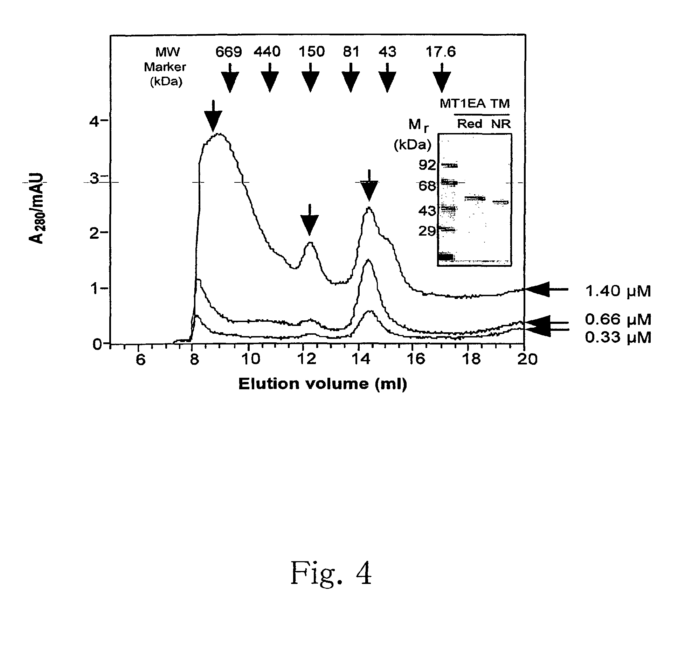

Fig. 4: Tests for a homophilic complex formation by the ectodomain of MT1-MMP. The gel permeation analysis profiles of MT1EA Δ TM at three different concentrations. The inset shows the SDS-PAGE analysis of MT1EAΔ TM under reducing and non-reducing conditions.

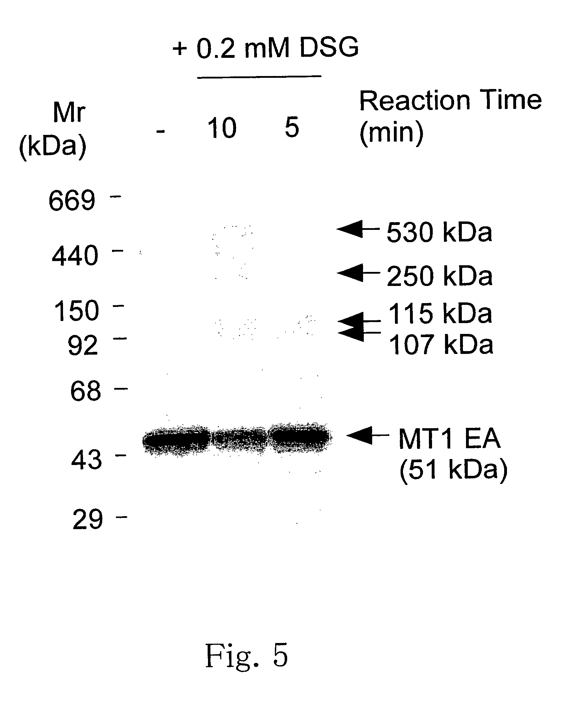

Fig. 5: Tests for the formation of the homophilic complex of the ectodomain of MT1-MMP. The products obtained by crosslinking MT1EAΔ TM with a chemical coupler, DSG, were examined by SDS-PAGE analysis.

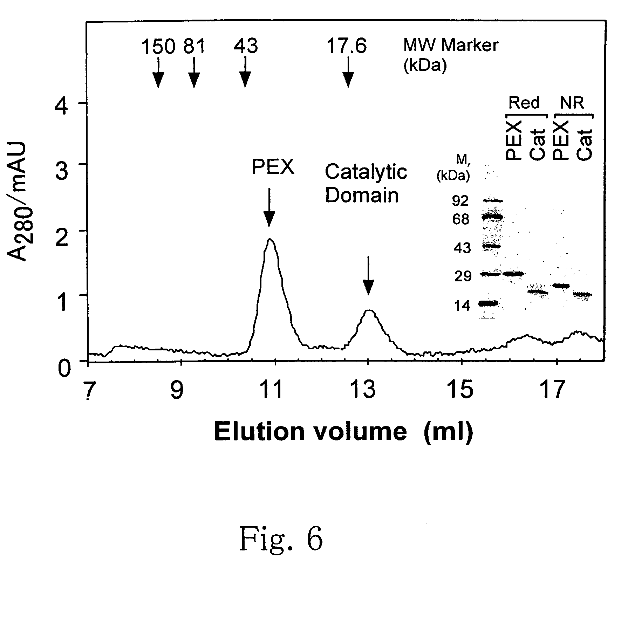

Fig. 6: In regard to the formation of a homodimer complex of MT1-MMP PEX, MT1EA Δ TM(50 µ g/ml) was treated with trypsin, leading to a cleavage between a catalytic domain and PEX, and then the samples were applied on a Superdex 75 gel permeation column. The results are shown in this drawing. The inset indicates purified PEX and catalytic domain (Cat) positions on SDS-PAGE analysis under reducing and non-reducing conditions.

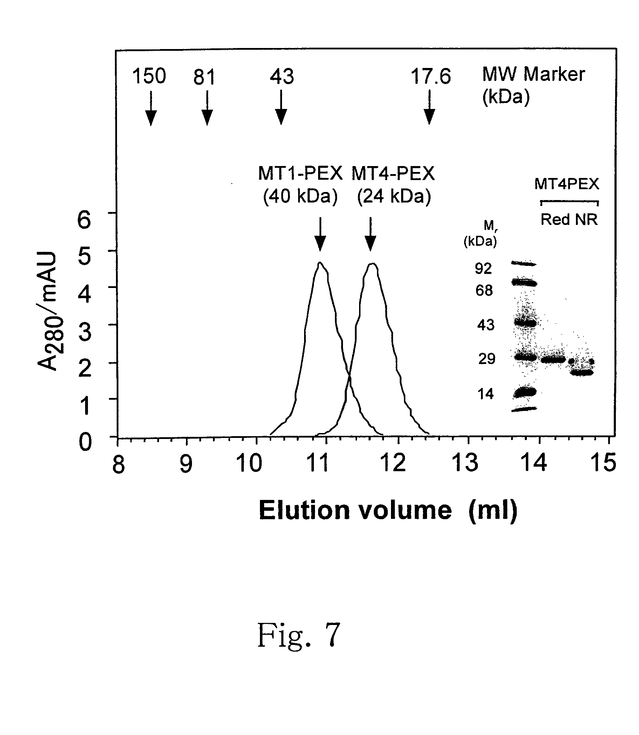

Fig. 7: Superdex 75 gel permeation column profiles of MT1-MMP PEX and mouse MT4-MMP PEX in regard to the homodimer complex formation of MT1-MMP PEX. The inset indicates mouse MT4-MMP PEX on SDS-PAGE analysis under reducing and non-reducing conditions.

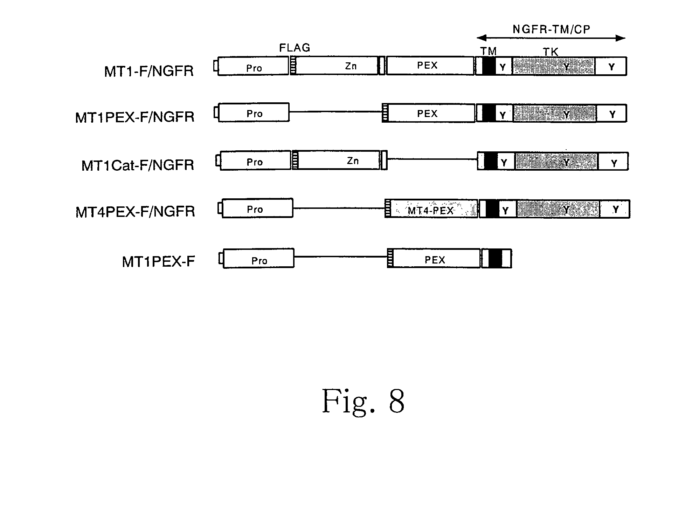

Fig. 8: A schematic illustration of various mutant MT1-MMPs constructed.

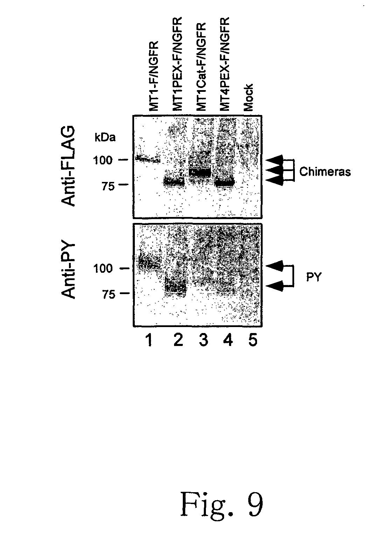

Fig. 9: Tests for a dimer formation by the ectodomain of MT1-MMP on the cell surface. COS1 cells were transfected with the expression vectors for MT1-F/NGFR, MT1PEX-F/NGFR, MT1Cat-F/NGF or MT4PEX-F/NGFR, or a vector alone (Mock). The cell lysates were analyzed by Western blotting using a monoclonal anti-FLAG M2 antibody (upper panel: Anti-FLAG) and an anti-phosphotyrosine PY20 monoclonal antibody (lower panel: Anti-PY).

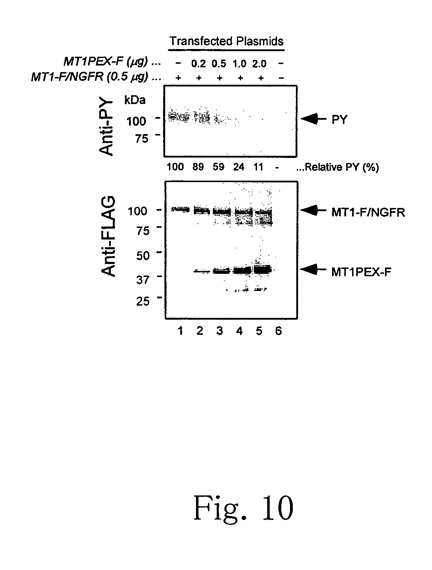

Fig. 10: Tests for a dimer formation by the ectodomain of MT1-MMP on the cell surface. COS1 cells were co-transfected with vectors for MT1-F/NGFR and MT1PEX-F at the indicated DNA ratio. The cell lysates were analyzed by Western blotting using anti-FLAG M2 (Anti-FLAG) and anti-phosphotyrosine PY20 (Anti-PY). The relative intensity of the bands detected with PY20 is shown.

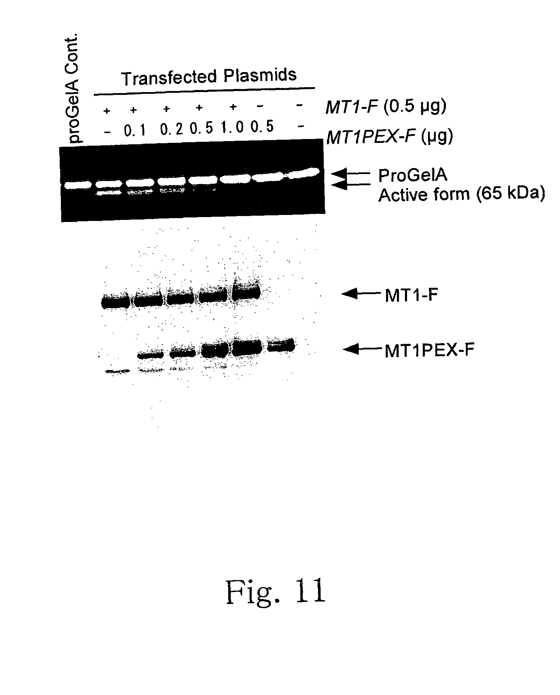

Fig. 11: The efficacy of MT1PEX-F on the proMMP-2 activation by MT1-F on the cell surface. COS1 cells were co-transfected with vectors for MT1-F and MT1PEX-F at the indicated DNA ratio. Then, the above cells were reacted with purified proMMP-2 (0.25 µ g/ml) in a serum-free medium at 37°C for 18 hrs. MMP-2 in the culture supernatants was analyzed by gelatin zymography (upper panel), and the cell lysates were analyzed by Western blotting using a monoclonal anti-FLAG M2 antibody (lower panel).

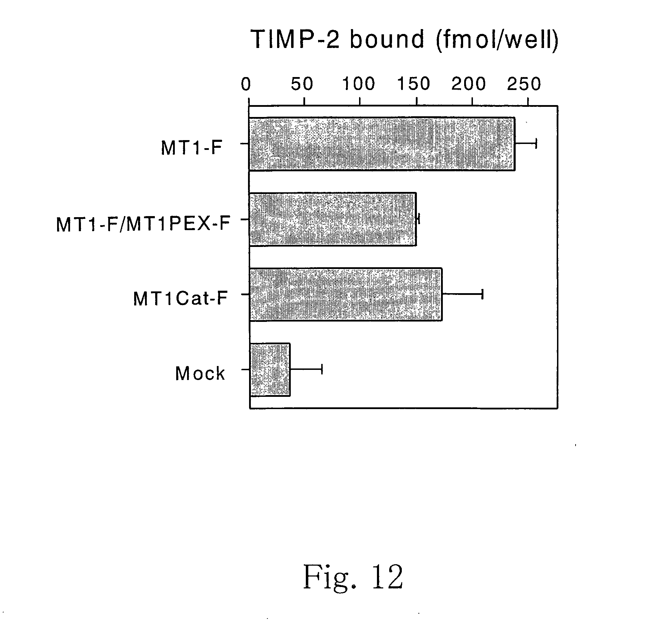

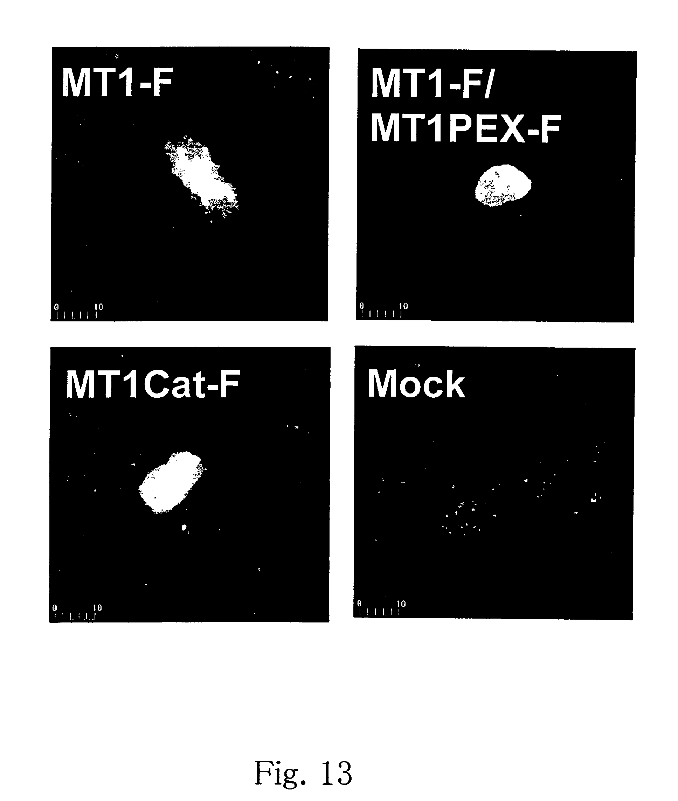

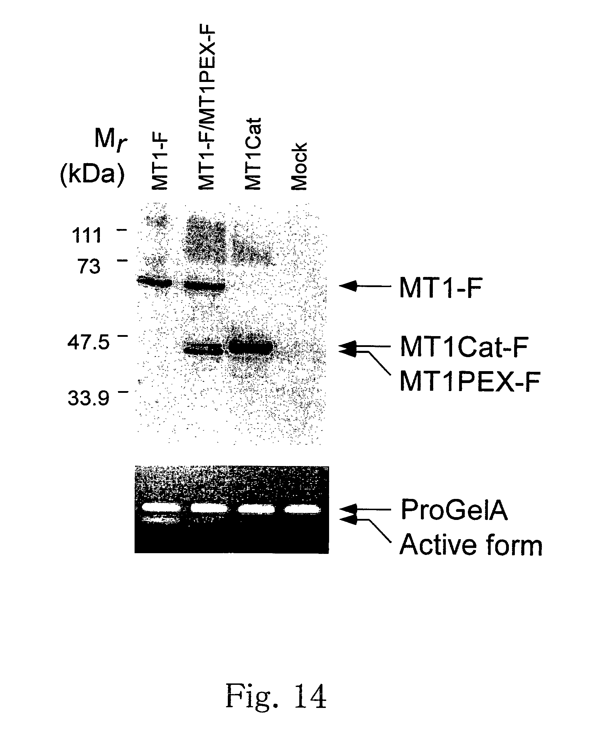

Fig. 12: The efficacies of MT1PEX-F on the proteolytic activity and TIMP-2 binding ability of MT1-F expressed on a cell surface are shown. As is shown in Fig. 12 through Fig. 14, CHO-K1 cells were transfected with the expression vectors for MT1-F, MT1PEX-F or MT1Cat-F or a vector alone (Mock). The cells were then treated with trypsin, and seeded on DQ™-gelatin coated cover slips for proteolytic activity tests (Fig. 13) and in 24-well plates for TIMP-2 binding assays (Fig. 12) and for proMMP-2 activation (Fig. 14). Results are all from the same transfection experiment. This figure indicates the following TIMP-2 binding assay: The transfected cells in the 24-well plates were incubated with 25 nM of ¹²⁵I-labeled TIMP-2 in the presence or absence of BB-94 (50 µ M) at 0°C for one hour. The MT1-MMP specific binding was obtained by subtracting the data in the presence of BB-94 (50 µ M) from each experiment.
Fig. 13: As in the case with Fig. 12, the effects of MT1PEX-F on proteolytic activity and TIMP-2 binding ability of MT1-F expressed on the cell surface are shown. The transfected cells on DQ^{TM} -gelatin coated cover slips were incubated in the medium at 37°C for 18 hrs. Generated fluorescence was analyzed by confocal microscopy.
Fig. 14: As in the case with Fig. 12, the efficacies of MT1PEX-F on the proteolytic activity and TIMP-2 binding ability of MT1-F expressed on a cell surface are shown. The same transfected cells in the 24-well plates were reacted with purified proMMP-2 in the serum-free medium at 37°C for 18 hrs. The cells and culture supernatants were analyzed by Western blotting using a monoclonal anti-FLAG M2 antibody (upper panel) and by gelatin zymography (lower panel), respectively.

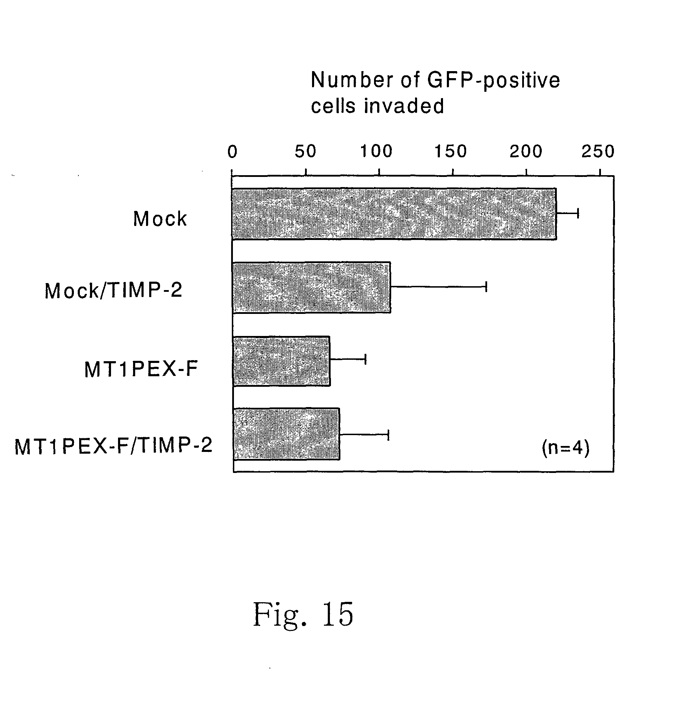

Fig. 15: The efficacy of MT1PEX-F on the matrigel invasive action of HT1080 cell is shown. HT1080 cells were co-transfected with the vectors for MT1PEX-F and GFP or transfected with the expression vector for GFP alone. Then, the cells were subjected to matrigel invasive analysis by the modified Boiden-chamber method as described in "Examples" herein. TIMP-2 at 0.5 µ g/ml was added to an upper and lower chambers. GFP-positive cells on the surface of the lower chamber were counted using fluorescent microscopy. The numbers of GFP-positive cells among Mock and MT1PEX-F transfected cells were 1.31 x 10⁴ and 1.12 x 10⁴ /1 x 10⁵, respectively.

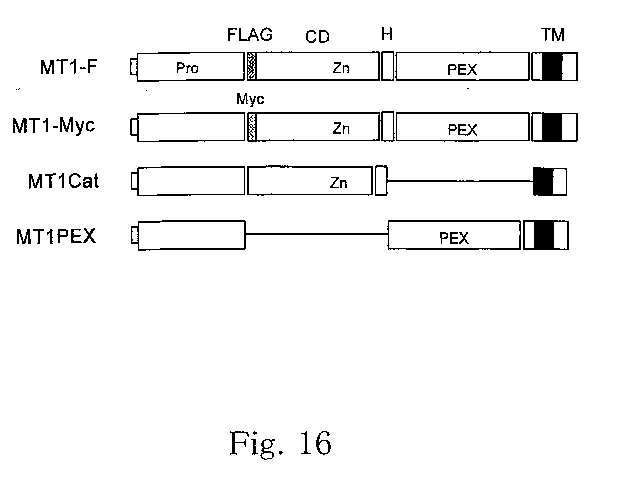

Fig. 16: A schematic illustration of MT1-MMP mutants as used for a homophilic complex formation by an interaction with the PEX domain of MT1-MMP. Each symbol indicates as follows: Pro: propeptide; FLAG: Flag epitope; Myc: c-Myc epitope; CD: catalytic domain; H: hinge domain; PEX: hemopexin-like domain; and TM: transmembrane domain.

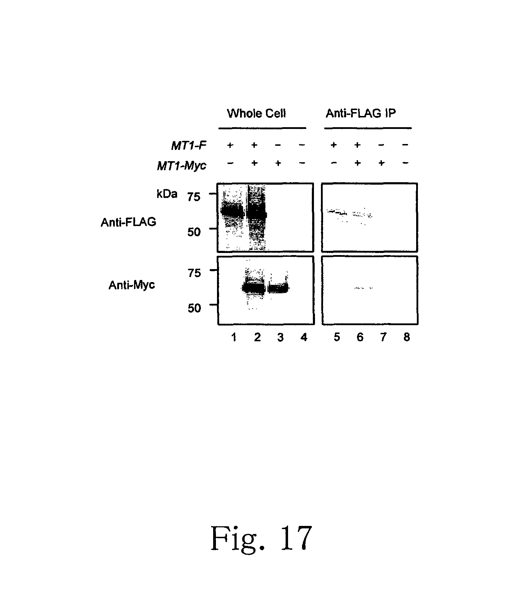

Fig. 17: As in the case with Fig. 16, COS1 cells were transfected with the vectors for MT1-F and/or MT1-Myc. The cells were lysed, and subjected to immunoprecipitation using anti-FLAG.M2 antibody-conjugated beads. Whole cell lysates (Whole Cell) and immunoprecipitates (Anti-FLAGIP) were analyzed by Western blotting using an anti-FLAG M2 antibody (Anti-FLAG) or an anti-c-Myc antibody (Anti-Myc).

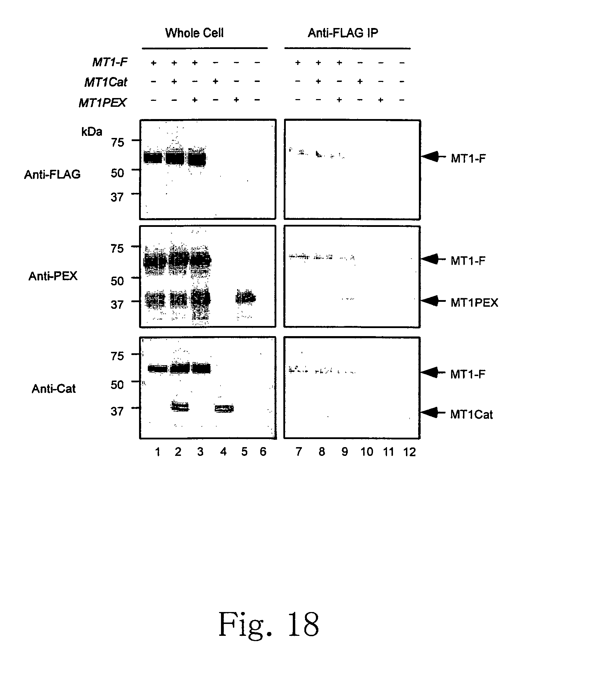

Fig. 18: As in the case with Fig. 16, the expression plasmids for MT1-F, MT1Cat and/or MT1PEX were transfected into COST cells. The cells were lysed, and subjected to immunoprecipitation using anti-FLAG M2 antibody-conjugated beads as in the case with Fig. 17. The samples were analyzed by Western blotting using an anti-FLAG M2 antibody (Anti-FLAG), anti-MT1-MMP PEX (Anti-PEX) or anti-MT1Ca (Anti-Cat).

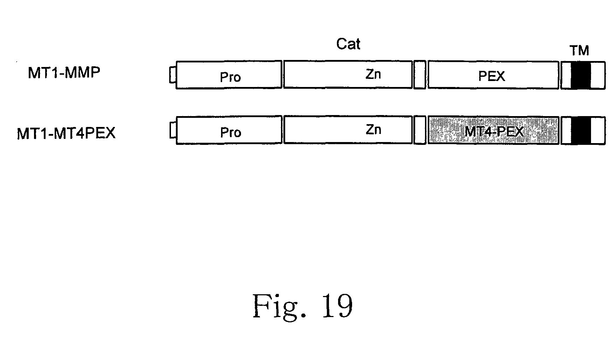

Fig. 19: A schematic illustration of MT1-MT4PEX as used for the study of MT1-MT4PEX on the proMMP-2 activation on the cell surface. The PEX domain of MT4-PEX is derived from MT4-MMP.

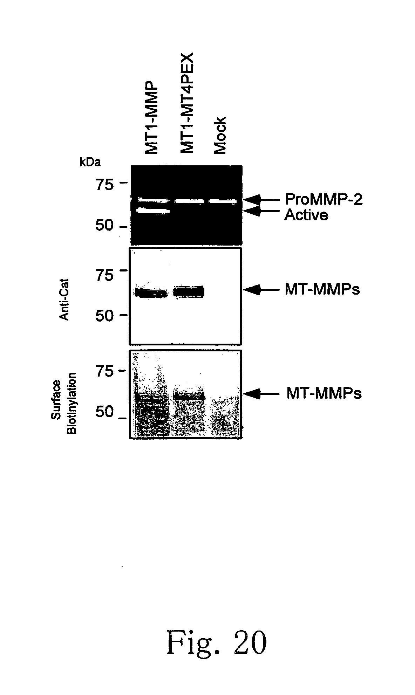

Fig. 20: As in the case with Fig. 19, the activation of proMMP-2 by MT1-MMP is shown. COS1 cells were transfected with the expression plasmids for MT1-MMP, MT1-MT4PEX or a vector alone (Mock), and reacted with proMMP-2 in the serum-free medium. The culture media were analyzed for proMMP-2 processing by zymography (upper panel). The cell lysates were analyzed by Western blotting using an anti-MT1Cat (mid panel). The transfected cells were subjected to surface biotinylation, and analyzed by Western blotting using an anti-MT1Cat (lower panel, Surface Biotinylation).

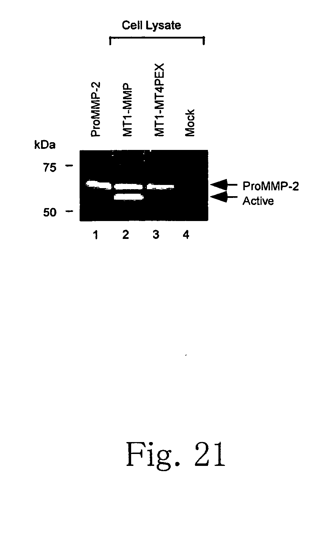

Fig. 21: As in the case with Fig. 19, the binding of proMMP-2 to the MT1-MT4PEX-expressing cells was examined. The samples were analyzed by gelatin zymography.

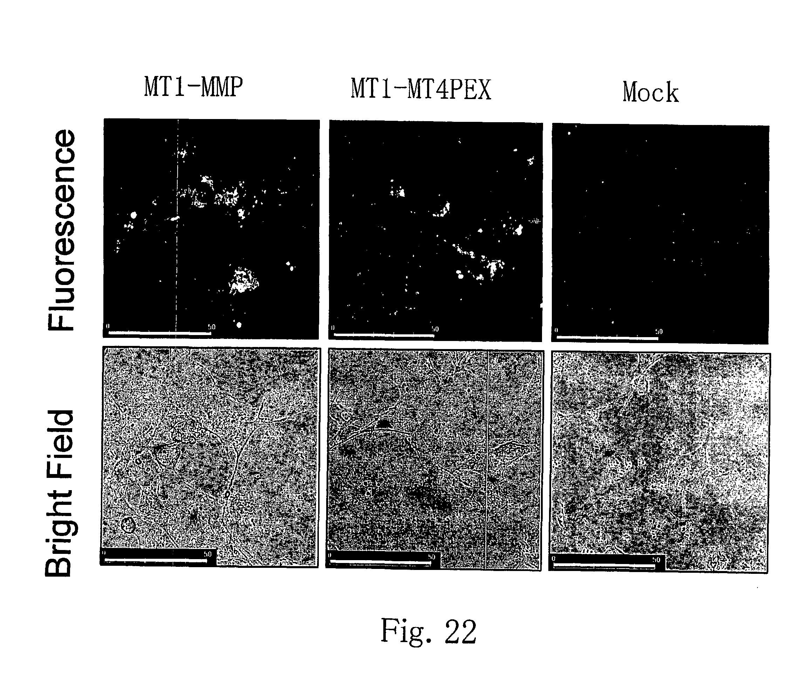

Fig. 22: As in the case with Fig. 19, the MT1-MT4PEX-expressing cells were cultured on DQ gelatin coated cover slips and then examined for their in situ gelatin degrading activity. The gelatin degrading activity was visualized as fluorescent areas. The bright-field image is also shown. Bar: 50µ m.

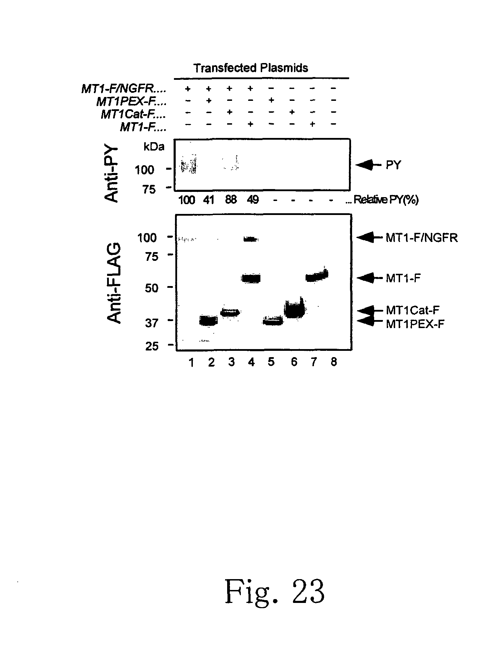

Fig. 23: Tests for the dimer formation by the ectodomain of MT1-MMP on the cell surface. COS1 cells were transfected with vectors for MT1-F/NGFR and/or MT1PEX-F, MT1Cat-F or MT1-F. The cells were subjected to Western blotting using anti-FLAG M2 (Anti-FLAG) or anti-PY20 (Anti-PY). The relative intensity of the bands detected by PY20 was shown by analyzing with an NIH image.

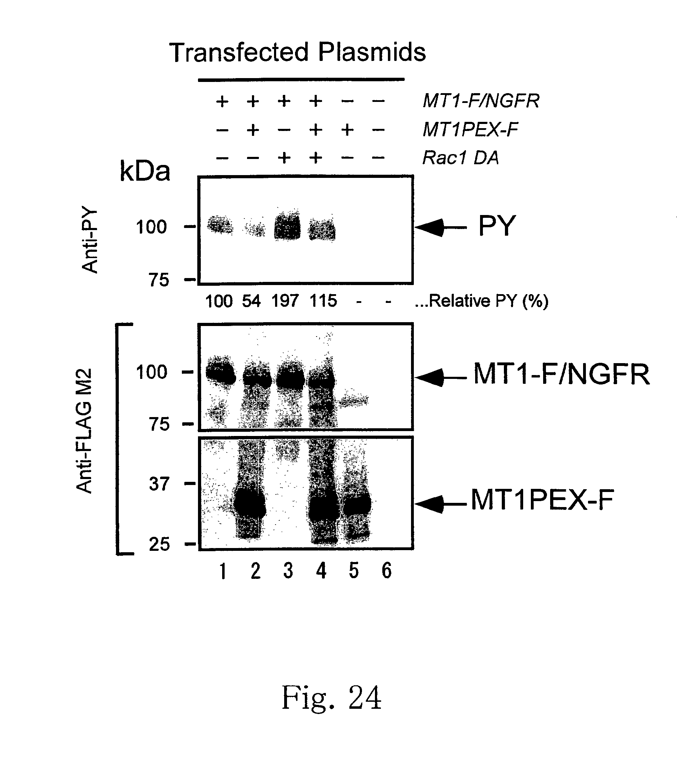

Fig. 24: In regard to the action of constitutively active Rac1 (Rac1DA) and the potential activation of proMMP-2 on the dimer formation of MT1-MMP, the efficacy of Rac1DA on the dimer formation of MT1-F/NGFR is shown. COS1 cells were transfected with the expression plasmids for MT1-F/NGFR, MT1PEX-F and/or Rac1DA. The cell lysates were subjected to Western blotting using PY20 (Anti-PY) and an anti-FLAG M2 antibody (Anti-FLAG). The relative intensity of the bands detected by PY20 was shown by analyzing with NIH image.

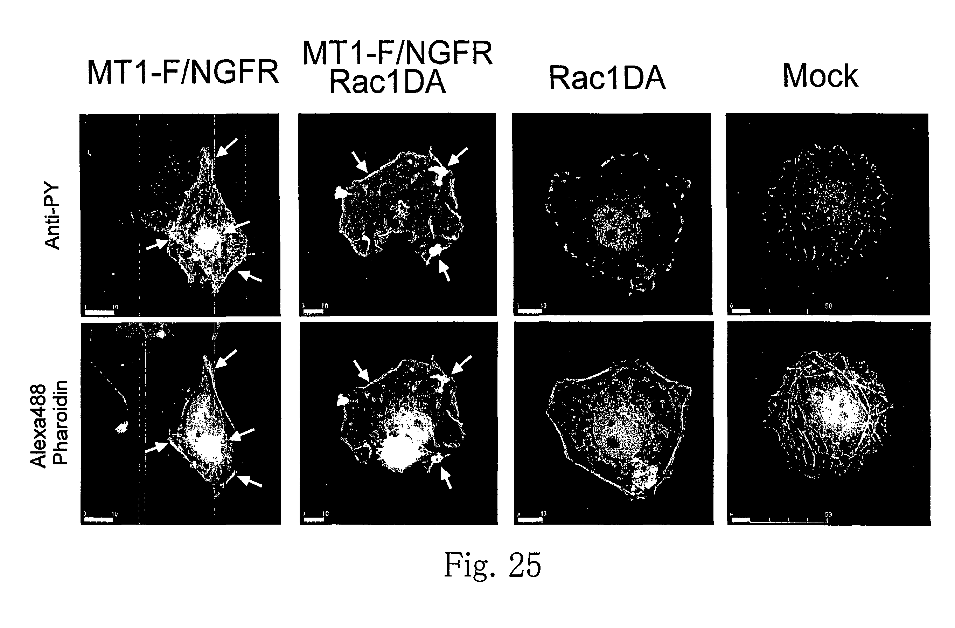

Fig. 25: As in the case with Fig. 24, the transfected COS1 cells were stained with PY20 (Anti-PY). F-actin was also stained with Alexa488-conjugated phalloidin (Alexa488 phalloidin). A white arrow indicates a site where F-actin and a PY signal were co-localized. Bar: 10 µ m.

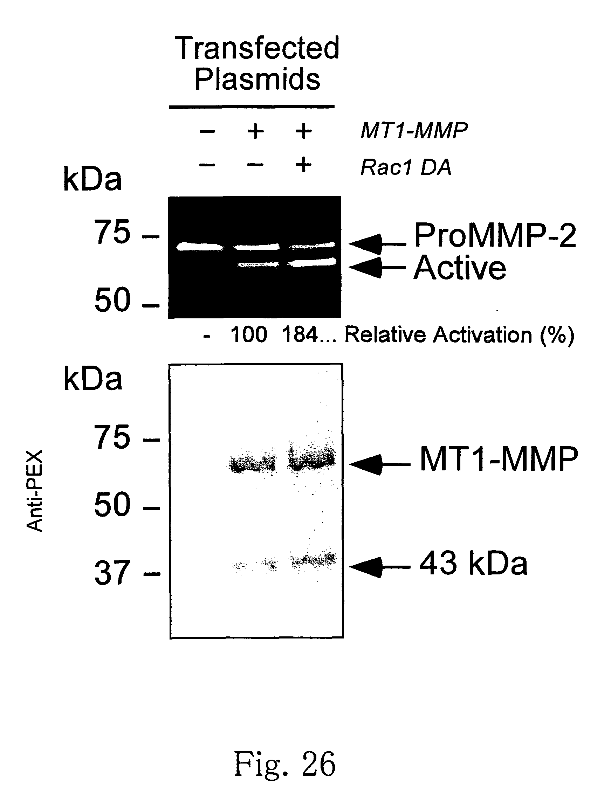

Fig. 26: As in the case with Fig. 24, COS1 cells were transfected with the expression plasmids for MT1-MMP and Rac1DA and reacted with purified proMMP-2. The culture media were analyzed for proMMP-2 processing by zymography (upper panel). The cell lysates were analyzed by Western blotting using an anti-MT1PEX antibody (lower panel). Relative activation of proMMP-2 to its active form was estimated by measuring the intensity of proMMP-2 bands with an NIH image.

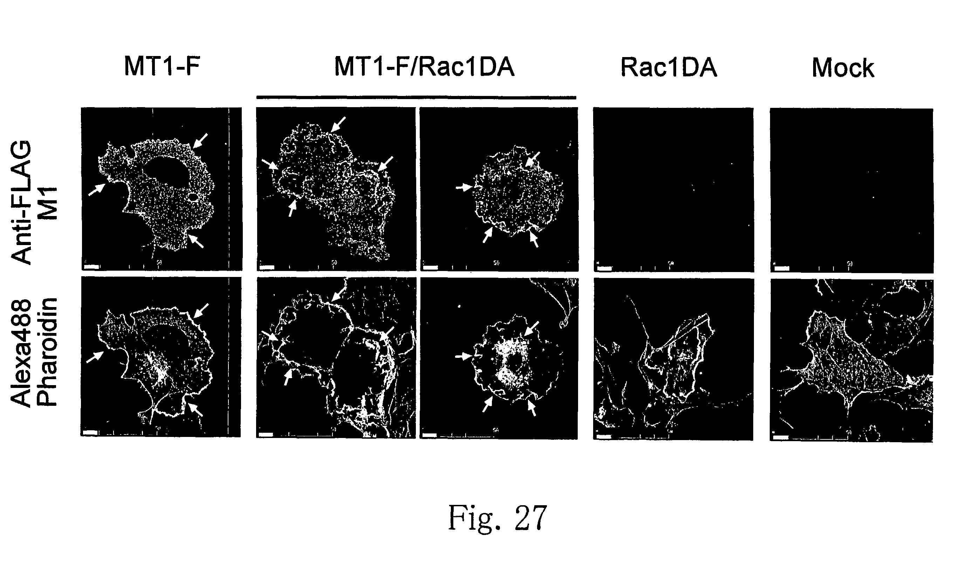

Fig. 27: As in the case with Fig. 24, the transfected COS1 cells were stained with an anti-FLAG M1 antibody. F-actin was also stained with Alexa488-conjugated phalloidin (Alexa488 phalloidin). A white arrow indicates a site where F-actin and a FLAG signal were co-localized. Bar: 10 µ m.

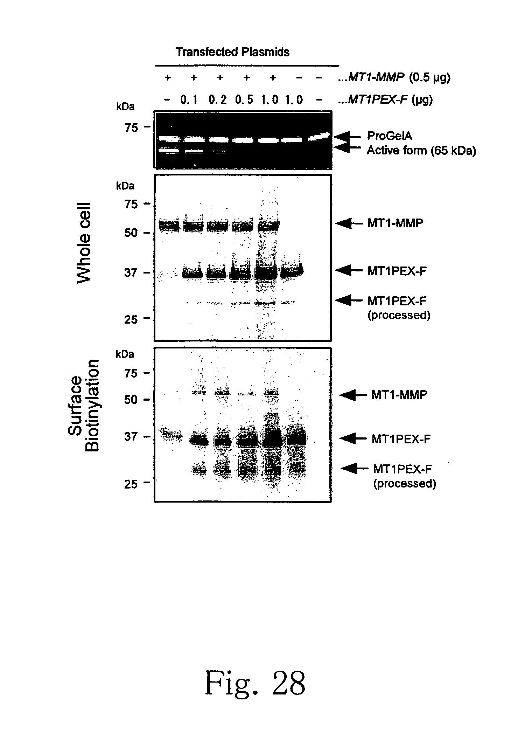

Fig. 28: In regard to the efficacy of MT1PEX-F on the proMMP-2 activation by MT1-MMP on the cell surface, COS1 cells were co-transfected with the vectors for MT1-MMP and MT1PEX-F at the indicated DNA ratio, and reacted with the purified proMMP-2. The culture supernatant was analyzed for MMP-2 by gelatin zymography (upper panel), and the cell lysates were analyzed by Western blotting using an anti-MT1-PEX antibody (mid panel, Whole cells). The transfected cells were subjected to surface biotinylation, and analyzed by Western blotting using an anti-MT1-PEX (lower panel, Surface Biotinylation).

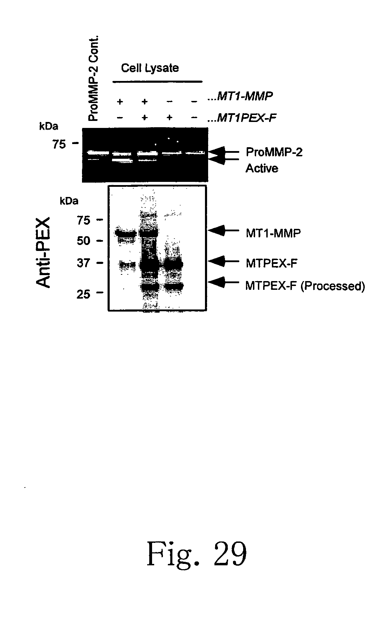

Fig. 29: As in the case with Fig. 28, the binding of proMMP-2 to the transfected cells was examined. The samples were analyzed by gelatin zymography.

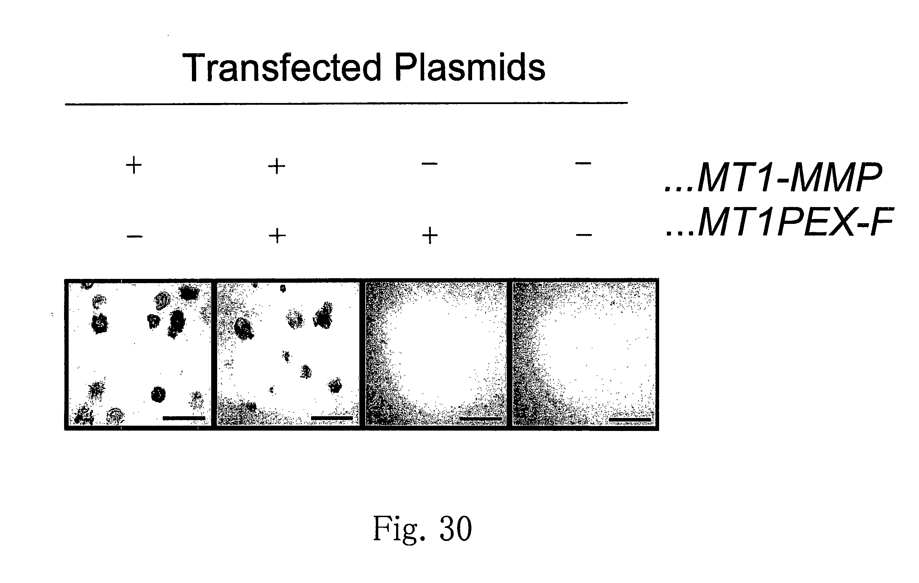

Fig. 30: As in the case with Fig. 28, the transfected cells were cultured on each slide glass coated with Alexa488-labeled gelatin and then examined for their in situ gelatin degrading activity. The gelatin degrading activity was visualized as the dark non-fluorescent area. Bar: 50 µ m.

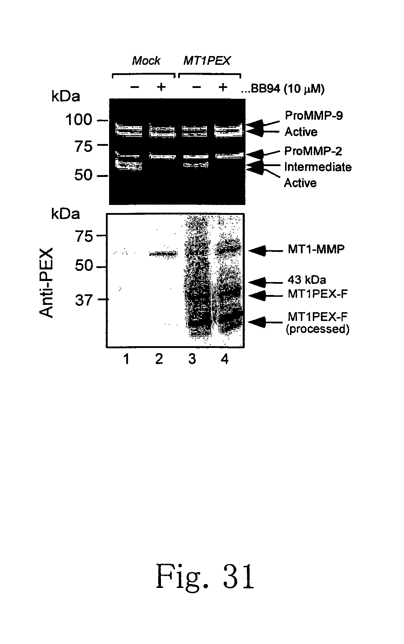

Fig. 31: In regard to the efficacy of MT1PEX-F on the matrigel invasive action of HT1080 cells, HT1080 cells were transfected with the stable expression plasmids for MT1PEX-F and the empty vector (Mock). Hygromycin resistant cells were collected and re-seeded. The culture medium was changed to a serum-free medium in the presence or absence of BB94, and the cells were further cultured. The culture media and the cell lysates were analyzed by gelatin zymography and Western blotting, using an anti-MT1PEX antibody, respectively.

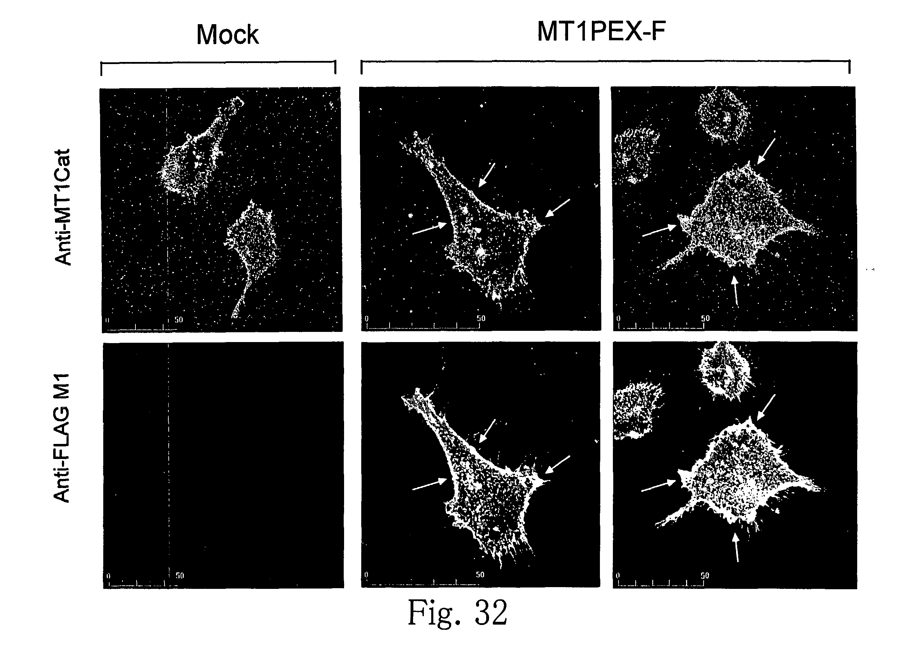

Fig. 32: As in the case with Fig. 31, HT1080 cells were transfected with the expression plasmids for MT1PEX-F or the empty vector. Endogenous MT1-MMP and MT1PEX-F in the transfected cells were stained immunologically using a rabbit anti-MT1Cat antibody and an anti-FLAG M1 antibody. A white arrow indicates a site where signals for endogenous MT1-MMP (Anti-MT1Cat and MT1PEX-F (Anti-FLAG M1) were co-localized. Bar: 50 µ m.

### Best Modes for Carrying Out the Invention

According to the present invention, inhibitory methods for the activation of proMMP-2 relying on inhibiting the formation of a complex comprised of plural MT1-MMPs, for example, the formation of a MT1-MMP homodimer wherein said MT1-MMP is present on the cell surface and substances each having activity therefor can be provided. Further, screening methods for such active substances and pharmaceutical products useful in such applications are provided.

Particularly, the PEX domain of MT1-MMP plays an important role in the homodimer formation of MT1-MMP. For instance, PEX lacking the catalytic domain of MT1-MMP shows an in vivo and in vitro inhibitory activity on the homodimer formation of MT1-MMP. Therefore, it is promising as an inhibitor or pharmaceutical drug, and enables development of various inhibitors against the homodimer formation of MT1-MMP based on this fact. In another aspect, the present invention provides techniques for monitoring the homodimer formation of MT1-MMP. Furthermore, it also provides an outlook for gene therapy.

The present invention relates to antibodies, for example, monoclonal antibodies which specifically recognize the PEX domain of MT1-MMP, and provides immunological assay reagents, pharmaceuticals and further various assay techniques using said antibody.

The term "PEX" or "PEX protein" used herein may cover MT1-MMP PEX domain proteins or fragments thereof [for example, from amino acid residues 316 to 508, selected from the amino acid sequence of MT1-MMP (SWISS-PROT accession number: P50281), and in some cases, from amino acid residues 319 to 535 of said MT1-MMP]. They may also be mutants, analogs and derivatives derived or constructed as described herein below. It is not particularly limited as long as it possesses a binding activity to the PEX domain of MT1-MMP. Such substances include those having an inhibitory activity on the formation of complexes comprised of multiple MT1-MMPs, and preferably those having an inhibitory activity on the homodimer formation of MT1-MMP present on the cell surface. The representatives include, but are not limited to, those which lack the sequence from Ser² to Ile³¹⁸, selected from the amino acid sequence from Met¹ to Gly⁵³⁵ of MT1-MMP, i.e., MT1PEX. The PEX protein may include corresponding PEX proteins with mutations (such as mutated PEX proteins wherein one or more amino acid residues are appropriately substituted, deleted, inserted, rearranged or added) introduced into the naturally occurring amino acid sequence of proteins each having an amino acid sequence corresponding to the PEX domain of MT1-MMP as long as the proteins have a desired biological activity (for instance, an inhibitory activity against the homodimer formation of MT1-MMP or an inhibitory activity against the proMMP-2 activation via inhibition of MT1-MMP homodimer formation, or further a suppressing or inhibiting activity on cell migration, invasion and/or metastasis resulting from the homodimer formation of MT1-MMP, etc.).

Such mutations, conversions and modifications include chemical techniques or those applied with the standard gene engineering methods based on the nucleotide sequence of the MT1-MMP gene (GenBank™ /EMBL accession number: D26512). For example, they are those described in "Zoku-Seikagaku Jikken Koza 1, Idenshi Kenkyuhou II" Japanese Biochemical Society ed., p. 105 (Susumu Hirose), Tokyo Kagaku-dojin Publishing Co., Inc. (1986); "Shin-Seikagaku Jikken Koza 2, Kakusan III (Recombinant DNA technique)" Japanese Biochemical Society ed., p. 233 (Susumu Hirose), Tokyo Kagaku-dojin Publishing Co., Inc. (1992); "Methods in Enzymology" Vol. 154, pp. 350 & 367, R. Wu, L. Grossman ed., Academic Press, New York (1987); "Methods in Enzymology" Vol. 100, pp. 457 & 468, R. Wu, L. Grossman ed., Academic Press, New York (1983); J. A. Wells, et al., Gene, 34:315, 1985; T. Grundstroem et al., Nucleic Acids Res., 13: 3305, 1985; J. Taylor et al., Nucleic Acids Res., 13: 8765, 1985; "Methods in Enzymology" Vol. 155, p. 568, R. Wu ed., Academic Press, New York (1987); A. R. Oliphant et al., Gene, 44:177, 1986 etc. For example, included are methods such as the site-directed mutagenesis method (site specific mutagenesis method) utilizing synthetic oligonucleotides (Zoller et al., Nucl.- Acids Res., 10:6487, 1987; Carter et al., Nucl. Acids Res., 13:4331, 1986), the cassette mutagenesis method (Wells et al., Gene, 34:315, 1985), restriction selection mutagenesis method (Wells et al., Philos. Trans. R. Soc. London Ser A, .317: 415, 1986), the alanine scanning method (Cunningham & Wells, Science, 244: 1081-1085, 1989), PCR mutagenesis method, Kunkel method, dNTP[α S] method (Eckstein), the region directed mutagenesis method using sulfurous acid and nitrous acid and the like.

Moreover, in the proteins obtained, amino acid residues contained therein can be modified by chemical techniques, and for example, can be modified and partially decomposed to make derivatives thereof using enzymes such as peptidases, e.g., pepsin, chymotrypsin, papain, bromelain, endopeptidase, exopeptidase and the like. Also, the protein can be glycosylated or deglycosylated, or glycosylation sites can be altered in vivo or in vitro (WO 87/05330; Apin and Wriston, CRC Crit. Rev. Biochem., 259-306, 1981; Hakimuddin et al., Arch. Biochem. Biophys., 259:52, 1987; Edge et al., Anal. Biochem., 118:131, 1981; "Methods in Enzymology" Vol. 138, p. 350, Academic Press, New York, 1987, etc.).

In the proteins of the present invention, the C-terminal end is typically a carboxyl group (-COOH) or a carboxylate (-COO), but the C-terminal end may be an amide form (-CONH₂) or an ester form (-COOR). For said ester, R includes C₁ to C₆ alkyl groups such as methyl, ethyl, n-propyl, isopropyl and n-butyl, C₃ to C₈ cycloalkyl groups such as cyclopentyl and cyclohexyl, C₆ to C₁₂ aryl groups such as phenyl and α-naphthyl, phenyl-C₁ to C₂ alkyl groups such as benzyl and phenethyl, C₇ to C₁₄ aralkyl groups including α -naphthyl-C₁ to C₂ alkyl groups such as α-naphthylmethyl, as well as a pivaloyloxymethyl group widely used as an ester in oral use. When the proteins of the present invention have a carboxyl group (or carboxylate) at the site other than the C-terminal end, those in which the carboxyl group can be amidated or esterified are included in the proteins of the present invention. As the ester in this case, for example, the C-terminal ester and the like described above are used.

Additionally, the proteins of the present invention may be those having a methionine residue at N-terminus in the above proteins, and further include those in which an amino group of the methionine residue is protected with a protecting group (for example, C₁ to C₆ acyl groups including C₁ to C₅ alkyl-carbonyl groups such as formyl and acetyl), those in which the N-terminus is cleaved in vivo and the resultant glutamyl group is pyroglutamylated, those in which substituents (for example, -OH, -COOH, amino, imidazole, indole, guanidino groups and the like) on side chains of the intramolecular amino acids are protected with appropriate protecting groups (for example, C₁ to C₆ acyl groups such as formyl and acetyl groups), or conjugated proteins (such as so-called glycoproteins) in which saccharide chains are linked. And, the proteins may be expressed as fusion proteins when made by gene recombination methods, and may convert or process into those having substantially equivalent biological activity as compared to those which naturally occur in vivo or in vitro. The production methods by fusion usually used in gene engineering can be used. Such fusion proteins can be purified by an affinity chromatography taking advantage of their fusion moieties.

Such fusion proteins include those fused to a histidine tag, or those fused to the amino acid sequence of maltose-binding protein (MBP), glutathione S-transferase (GST) or thioredoxin (TRX). Similarly, the polypeptide can be added with a tag of heterogenous epitope, and can be isolated/purified by an immunoaffinity chromatography using an antibody specifically binding to the epitope.
The representatives include poly histidine (poly-His) or a polyhistidine-glycine (poly-His-Gly) tag, flu HA tag and the antibody thereto, 12CA5, c-Myc tag and the antibodies thereto, 8F9, 3C7, 6E10, G4, B7 and 9E10, Herpes Simplex virus glycoprotein D (gD) tag and the antibody thereto, FLAG peptide, KT3 epitope peptide, α-tubulin epitope peptide, T7 gene 10 protein peptide tags and the like (Field et al., Molecular and Cellular Biology 8: 2159-2165, 1988; Evans et al., Molecular and Cellular Biology 5: 3610-3616, 1985; Paborsky at al., Protein Engineering 3(6): 547-553, 1990; Hopp et al., BioTechnology 6: 1204-1210, 1988; Martin et al., Science 255: 192-194, 1992; Skinner et al., J. Biol. Chem., 266: 15163-15166, 1991; Lutz-Freyermuth et al., Proc. Natl. Acad. Sci. USA, 87: 6393-6397, 1990). The expression and purification of such fusion proteins can be performed using commercially available kits suitable for these techniques, and can also be carried out according to protocols as instructed by manufacturers or distributors of the kits.

Modifications and alterations of the protein structures can be performed in reference to "Shin-Seikagaku Jikken Koza 1, Protein VII, Protein Engineering" Japanese Biochemical Society ed., (Tokyo Kagaku-dojin Publishing Co., Inc., 1993) using the methods described therein or the methods described in the references cited therein, and, further, the methods substantially equivalent thereto. Their biological activities as described herein below may include an immunological activity, for example, an antigenicity. The modification and alteration may be deamination, hydroxylation, phosphorylation, methylation, acetylation, ring-opening, cyclization, alteration of contained saccharide chains to different types, increasing or decreasing the number of contained saccharide chains, substitution to D-amino acid residues, etc. Those methods are known in the art (for example, T. E. Creighton, Proteins: Structure and Molecular Properties, pp. 79-86, W. H. Freeman & Co, San Francisco, USA, 1983, etc.).

Thus, the proteins may be those of which one or more amino acid residues are different from those which naturally occur in terms of identity or those of which sites of one or more amino acid residues are different from those which naturally occur. The proteins include deletion analogs where one or more (for example, from 1 to 190, preferably from 1 to 50, more preferably from 1 to 20, still preferably from 1 to 10 and especially from 1 to 5) amino acid residues characteristic of PEX are deleted, substituted analogs where one or more (for example, from 1 to 100, preferably from 1 to 50, more preferably from 1 to 20, still preferably from 1 to 10 and especially from 1 to 5) amino acid residues characteristic of PEX are substituted with other residues, and additional analogs where one or more (for example, from 1 to 100, preferably from 1 to 50, more preferably from 1 to 20, still preferably from 1 to 10 and especially from 1 to 5) amino acid residues are added. The mutants as the above are all included in the present invention as long as the domain structure or active center structure characteristic of native PEX is retained and the resistance is retained against the dimer formation of MT1-MMP present on a cell surface described above. Also, it is thought that the proteins may include those having parts of primary structure conformation substantially equivalent to that of native PEX. Furthermore, it is also thought that the proteins may include those having biological activity substantially equivalent to native PEX except for being resistant to the dimer formation of MT1-MMP present on a cell surface.

Moreover, the protein can be one of the mutants which naturally occur. The proteins include those having more than 50% homology, and more preferably 70% or more or 90% or more of an homologous amino acid sequence for the amino acid sequence ranging from the 316th amino acid residue P to the 508th amino acid residue C (and/or the amino acid sequence ranging from the 319th to 535th amino acid residues) in the amino acid sequence of MT1-MMP. The fragments derived from the protein are parts of peptides of the PEX protein (i.e., partial peptides of the PEX protein), and may be any of those as long as they have activity substantially equivalent to the above PEX protein. For example, the partial peptides of the protein of the present invention include peptides having at least two or more consecutive, preferably 5 or more, more preferably 10 or more, still preferably 20 or more, still more preferably 50 or more, and, in some cases, 100 or more amino acid sequences in the constitutive amino acid sequence ranging from the 316th to the 508th amino acid residues (and/or the 319th to the 535th amino acid residues) of the amino acid sequence of MT1-MMP. For example, included are those having the same homology as described above in regard to the homology to the region corresponding to the amino acid sequence of MT1PEX.

The term "substantially equivalent" used herein means that the protein activity, for example, the resistance to proMMP-2 activation by the homodimer formation of MT1-MMP, the physiological activity or biological activity thereto is substantially equivalent. Furthermore, the meanings of that term may include a case having substantially homogenous activity. The substantially homogenous activity can include, for example, a predisposition to inhibit the dimer formation, and the activity to suppress and/or inhibit cell migration, invasion and/or metastasis associated with the activation of proMMP-2. The substantially homogenous activity indicates that these activities are qualitatively homogenous, and, for example, that they are physiologically, pharmacologically or biologically homogenous. For instance, it is preferred that the activities to inhibit the dimer formation are equivalent (for example, from about 0.001 to 1000 fold, preferably from about 0.01 to 100 fold, more preferably from about 0.1 to 20 fold, and still preferably from about 0.5 to 2 fold), but quantitative elements such as the extents of these activities, molecular weights of the proteins etc. may be different.

Next, the substitution, deletion or insertion of amino acids does not often cause a great alteration in physiological or chemical properties of a polypeptide.
In such a case, a polypeptide with substitution, deletion or insertion will be considered to be substantially identical to a polypeptide without such substitution, deletion or insertion. Substantially identical substituents of amino acids in the amino acid sequence can be selected from other amino acids in the class to which the amino acid belongs. For instance, non-polar (hydrophobic) amino acids include alanine, phenylalanine, leucine, isoleucine, valine, proline, tryptophan, methionine and the like, polar (neutral) amino acids include glycine, serine, threonine, cysteine, tyrosine, asparagine, glutamine and the like, amino acids having positive charge (basic amino acids) include arginine, lysine, histidine and the like, and amino acids having a negative charge (acidic amino acids) include aspartic acid, glutamic acid and the like.

The methods known in the peptide synthetic art, for example, chemical synthetic methods such as liquid phase synthetic methods, and solid phase synthetic methods can be used for the synthesis of the proteins and partial peptides thereof of the present invention (Stewart et al., Solid-Phase Peptide Synthesis, W. H. Freeman Co., San Francisco, CA, USA, 1969; Merrifield, J. Am. Chem. Soc., 85:2149-2154, 1963; J. Org. Chem., 37:3404, 1972; G. B. Fields (ed), "Methods in Enzymology", Vol. 289 (Solid-Phase Peptide Synthesis), Academic Press, New York, 1997). In such methods, using a resin for synthesis of proteins or peptides, an appropriately protected amino acid is sequentially attached to the desired amino acid sequence on the resin by various condensation methods as known in the art. Various activation reagents as known in the art are preferably used for the condensation reactions. For example, carbodiimides such as dicyclohexylcarbodiimide can be preferably used as such reagents. An objective reagent can be obtained by appropriately eliminating a protecting group when a product has the protecting group.

The proteins and partial peptides thereof of the present invention can be converted to salts thereof according to the methods known in the art or methods based thereon when they are obtained as free types. Also, when they are obtained as salt types, they can be converted to free types or other salt types according to the methods known in the art or methods based thereon.

The proteins of the present invention (including the protein fragments having the given consecutive regions of the amino acid sequence present in MT1-MMP and the fragments derived therefrom, the proteins and the fragments derived therefrom which are obtained from those having a deletion of one selected from the group consisting of a signal peptide domain, propeptide domain, core enzymatic domain, hinge domain, and transmembrane domain of MT1-MMP) can be obtained by applying gene engineering techniques based on the base sequence of human MT1-MMP gene (GenBank™/EMBL accession number:
D26512). Nucleic acids having the base sequence of the human MT1-MMP gene, for example the DNA molecule can be obtained by PCR or ligase chain reaction (LCR) amplification of the desired sequence by designing and synthesizing appropriate primers based on the MT1-MMP base sequence, preferably using mRNA or cDNA prepared from the mRNA derived from the animal which is an origin of the human MT1-MMP base sequence. In some cases, the DNA molecule can be obtained by screening human genomic DNA libraries or cDNA libraries derived from humans constructed from various human tissues or cultured cells using the resultant DNA fragments as probes.

First, sense and antisense primers are synthesized based on the base sequence of the MT1-MMP. The primers used for the PCR method are not particularly limited as long as they can amplify the DNA fragments containing the desired site. The sense primer can be synthesized preferably by selecting the 5'- terminal exon site of the gene, and the antisense primer can be synthesized preferably by selecting the 3'-terminal exon site of the gene, and more preferably can be selected from the sites other than the exon site utilized for synthesis of the sense primer. It is preferred that the 5'-terminal primer is selected to contain at least an initiation codon or to be able to amplify including the initiation codon and that the 3'-terminal primer is selected to contain at least a stop codon or to be able to amplify including the stop codon. Representatively, the primers can use (a) oligonucleotides having base sequences corresponding to the given 5'-terminal site of the base sequence of human MT1-MMP: GenBank™/EMBL accession number: D26512, and (b) oligonucleotides having complementary base sequences for the given 3'-terminal site of the base sequence of the human MT1-MMP.

It may be targeted to obtain cDNA for the gene as its full length at a time. However, plural primers can be designed and synthesized with utilization of the given exon sites (plural exon sites) and plural PCRs may be designed and conducted, whereby DNA fragments may be obtained. The target cDNA of said gene can be obtained from DNA fragments cloned on the basis of said resultant DNA fragments. The primers include oligonucleotides preferably composed of 5 or more nucleotide bases, for example, oligonucleotides composed of 10 to 50, more preferably 15 to 35, and still preferably 18 to 25 nucleotide bases. Synthesis of the primers can be carried out by methods known in the art, and, for example, the primers can be synthesized by the phosphodiester, the phosphotriester, and the phosphoamidite methods using, for example, an automatic DNA synthesizer, e.g., model 381A DNA synthesizer, Applied Biosystems.

mRNA samples can be isolated from various human tissues and cultured cells known to express human MT1-MMP (in particular, human cells obtained from a human heart, brain, placenta, lung, liver, skeletal muscle, kidney, pancreas and the like, tumor cell lines such as human lung epidermoid cancer cell lines and the like). Isolation of mRNA can be carried out by the methods known in the art or methods substantially equivalent thereto or modified methods thereof, but can also be performed by methods described in J. Sambrook et al., "Molecular Cloning", 2nd ed., Chapter 7, Cold Spring Harbor Laboratory, Cold Spring Harbor, N.Y., 1989; D. M. Glover et al., ed., "DNA Cloning", 2nd ed., Vol. 1, (The Practical Approach Series), IRL Press, Oxford University Press, 1995; L. Grossman et al. ed., "Methods in Enzymology", Vol. 12, Part A & B, Academic Press, New York, 1968; S. L. Berger et al. ed., "Methods in Enzymology", Vol. 152, p.33 & p.215, Academic Press, New York, 1987; Biochemistry, 18:5294-5299, 1979 and the like, for example, the guanidine cesium chloride method, the thiocyanate guanidine method, the phenol method and the like. Kits used for the isolation of mRNA include those commercially available from Pharmacia, Stratagene, Gibco-BRL etc. Poly (A)⁺ mRNA can be obtained by purifying the obtained total RNA using an oligo (dT) cellulose column, spin column, oligo (dT) binding magnetic beads and the like, if necessary.

The cDNA is made using this mRNA and reverse transcriptase (RNA dependent DNA polymerase). An oligo (dT) primer can also be used in the reverse transcription reaction. Those preferably having 12 to 18 T residue can be used as the oligo (dT) primers. It is also preferred that a synthetic oligonucleotide primer where a restriction enzyme site is ligated to the 5' side of the 12 to 18 T residue when directional cloning is carried out. Examples of such primers include Xba I oligo (dT) primer adapter and the like. When random hexamer primers are used, a probability to obtain the 5'-terminal site of mRNA is increased, and the random hexamer primer can be used alone or in combination with the oligo (dT) primer. In particular, it is also preferred that oligonucleotide primers synthesized based on the base sequence of the above human MT1-MMP gene (GenBank™/EMBL accession number: D26512) are used. A RNA inhibitor can also be added in the reverse transcription reaction if necessary. The synthesis of cDNA using mRNA and the reverse transcriptase can be carried out by methods known in the art or methods substantially equivalent thereto and modified methods thereof, which include methods described in H. Land et al., Nucleic Acids Res., 9:2251, 1981; U. Gubler et al., Gene, 25:263-269, 1983; S. L. Berger et al. ed., "Methods in Enzymology", Vol. 152, p.307, Academic Press, New York, 1987.

Polymerase chain reaction (PCR) is carried out using the resultant cDNA as well as the aforementioned sense and antisense primers to amplify the desired region of the cDNA.

The term "polymerase chain reaction" or "PCR" used herein usually refers to techniques described in US Patent No. 4,683,195. For example, the PCR is an in vitro method for the enzymatic amplification of desired specific nucleotide sequences. In general, the PCR includes repetitive series of cycles wherein a primer elongation synthesis is constructed usin two oligonucleotide primers capable of preferentially hybridizin with a template nucleic acid. Typically, the primers used in PCR may include those which are complementary to the internal nucleotide sequence of interest in the the template.
For example, preferable primer pairs as used herein may be those which are complementary to both ends of said nucleotide sequence to be amplified, or flanking regions adjacent to said nucleotide sequence.

A template (for example, DNA synthesized using, as a template, mRNA) and primers synthesized according to designs on said gene are mixed with a 10 x reaction buffer (attached with a Taq DNA polymerase kit), dNTPs (deoxyribonucleoside triphosphates; dATP, dGTP, dCTP and dTTP mix), Taq DNA polymerase and deionized distilled water. The mixture is subjected to 25 to 60 cycles of amplification using an automated thermal cycler such as GeneAmp 2400 PCR system, Perkin-Elmer/Cetus under general PCR cycle conditions. The number of amplification cycles can be suitably set to an appropriate value depending on purposes. The PCR cycle includes, for example, denaturation at 90 to 95°C for 5 to 100 sec, annealing at 40 to 60°C for 5 to 150 sec and extension at 65 to 75°C for 30 to 300 sec, and preferably denaturation at 94°C for 15 sec, annealing at 58°C for 15 sec and extension at 72°C for 45 sec. For the annealing temperature and reaction time, an appropriate value is suitably selected by experimentation. For the denaturation and extension time, an appropriate value suitably varies according to the strand length of expected PCR products. In general, the time of annealing preferably varies depending on the Tm value of primer-template DNA hybrids. The time period of extension usually is set with the aim of getting about 1 min per 1000 bp in strand length, but it may be possible to select a shorter time period in some cases.

The resultant PCR products are typically subjected to electrophoresis on 1 to 2% agarose gels. Specific bands are cut out from the gel, and DNA is extracted with a commercially available kit, e.g., Gene clean kit (Bio 101) and the like. The extracted DNA is cleaved with appropriate restriction enzymes and purified if necessary. Then, the 5'-end is, if necessary, phosphorylated with T4 polynucleotide kinase, etc. and subsequently the DNA is ligated into an appropriate plasmid vector including a pUC vector such as pUC18, and transformed into suitable competent cells. The cDNA library can also be constructed based on the produced DNA fragments using phage vectors, plasmid vectors etc. Transformation of host cells such as E. coli can be carried out by methods known in the art such as the calcium method, the rubidium/calcium method, the calcium/manganese method, the TFB high efficiency method, the FSB frozen competent cell method, the rapid colony method, the electroporation and the like or methods substantially equivalent thereto (D. Hanahan, J. Mol. Biol., 166:557, 1983, etc.).

Nucleic acids having an entire or partial base sequence of the gene can also be obtained by chemical synthesis. In such cases, these may be made by chemically synthesizing the fragments and ligating them with enzymes. Also, the desired sequence can be obtained using the chemically synthesized fragments as primers or probes.

The PCR reactions can be carried out by methods known in the art or methods substantially equivalent thereto and modified methods thereof, and can be performed according to methods described, for example in Saiki, R., et al., Science, 230:1350, 1985; Saiki, R., et al., Science, 239:487, 1988; Erlich, H. A. ed., PCR Technology, Stockton Press, 1989; 'Glover, D. M., et al. ed., "DNA Cloning", 2nd ed., Vol. 1, (The Practical Approach Series), IRL Press, Oxford University Press, 1995; Innis, M. A. et al. ed., "PCR Protocols: Guide to Methods and Applications", Academic Press, New York, 1990; McPherson, M. J., Quirke, P. and Taylor, G. R. ed., PCR: Practical Approach, IRL Press, Oxford, 1991; Frohman, M. A. et al., Proc. Natl. Acad. Sci. USA, 85:8998-9002, 1988, and modified or altered methods thereof. The PCR methods can also be performed using commercially available kits suitable therefor, and can also be carried out according to protocols described by manufacturers or distributors of the kits.

The term "oligonucleotide(s)" used herein refers to a relatively short single-stranded polynucleotide or double-stranded polynucleotides, or preferably polydeoxynucleotide(s), They can be chemically synthesized by known methods as described in Angew, Chem. Int. Ed. Engl., 28: 716-734, 1989, including the triester method, the phosphite method, the phosphoamidite method, the phosphonate method and the like. It has been typically known that the synthesis can be conveniently carried out on modified solid supports. For example, the synthesis can be carried out using an automated synthesizer and such a synthesizer is commercially available. The oligonucleotide may contain one or more modified nucleotide bases and, for example, it may contain a nucleotide base which does not naturally occur, such as inosine, or a tritylated nucleotide base.

The DNA fragments encoding the desired human MT1-MMP can be yielded by cloning the resultant PCR product and identifying the resultant PCR product. Also, the target DNA can be isolated by screening various cDNA libraries using the DNA fragment as a probe. Commercially available plasmid vectors such as p-Direct (Clontech), pCR-Script™ SK(+) (Stratagene), pGEM-T (Promega), PAmP™ (Gibco-BRL) etc., can be used for cloning of the PCR products.

In order to yield the full length gene sequence containing the full open reading frame, the cDNA libraries constructed from various human tissues or cultured cells as mentioned above are screened using the DNA fragments obtained as described above as probes, clones hybridizing to the probes are selected, an insertion base sequence of cDNA in the clone is sequenced, and the DNA fragment composing the gene is identified if necessary. Of course, the insertion sequence of cDNA in the clone can be subcloned if necessary. To label the probe and the like with radioisotopes and the like, it can be carried out using commercially available kits such as a random primed DNA labeling kit (Boehringer Mannheim) and the like.

Reverse transcription PCR (polymerase chain reaction coupled reverse transcription; RT-PCR) and RACE (rapid amplification of cDNA ends) can be applied to isolate the target DNA. RACE can be carried out according to, for example, the methods described in Innis, M. A., et al., ed., "PCR Protocols" (Frohman, M. A., "A Guide to Methods and Applications"), pp.28-38, Academic Press, New York, 1990 etc. RT-PCR products can be cloned into plasmid vectors, which can be introduced into highly efficient competent cells.

Furthermore, cDNA can also be PCR-amplified by utilizing the method where mRNA can be isolated and purified from a trace quantity of tissue or cells, for example, the method by utilizing commercially available kits such as the REX kit (United States Biochemical), Glass MAX™ RNA spin cartridge system (Gibco-BRL) and the like, reversely transcripting the resultant mRNA using an oligo (dT) primer to synthesize the first strand DNA, then attaching a homopolymer tail (for example, G residue) to the 3'-terminus of the first strand DNA or attaching an adapter to the DNA, and subsequently using the oligo (dT) primer and an oligo (dC) primer or the adapter primer. The commercially available kits suitable for this include SuperScript™ pre-amplification system (Gibco-BRL), cDNA Cycle™ kit (Invitrogen) and the like.

Hybridization is carried out by transferring a sample (for example, gene libraries prepared from human cells and tissues, representatively, those obtained by constructing into a phage such as λ gt10 and the like, which is infected to host E. coli such as the E. coli C600hfl strain to form plaques) on a membrane such as a nylon filter, performing denaturation, fixation and washing treatments if necessary, and subsequently reacting the sample transferred on the membrane with a labeled probe DNA fragment denatured, if necessary, in the buffer for hybridization.

The hybridization treatment is carried out usually at about 35°C to about 80°C, more suitably from about 50°C to about 65°C for about 15 min to about 36 hrs, more suitably from about 1 hr to about 24 hrs, but can be carried out by selecting an optimal condition appropriately.
For instance, the hybridization treatment is carried out at about 55°C for about 18 hrs. As buffers for the hybridization, those that can be used are selected among those usually used in the art, for example, Rapid hybridization buffer (Amersham) and the like can be used. The denaturation treatment of the transferred membrane includes the method using an alkali denaturation solution, and it is preferred that treatment is carried out with a neutralization solution or buffer after the treatment. The fixation treatment of the membrane is carried out by baking usually at about 40°C to about 100°C, more suitably from about 70°C to about 90°C for about 15 min to about 24 hrs, more suitably from about 1 hr to about 4 hrs, but can be carried out by selecting an optimal condition appropriately. For instance, the fixation is carried out by baking the filter at about 80°C for about 2 hrs. The washing treatment of the transferred membrane can be carried out by washing with a washing solution which is usually used in the art, for example 50 mM Tris-HCl buffer, pH 8.0 containing 1M NaCl, 1 mM EDTA and 0.1% sodium dodecyl sulfate (SDS), and the like. As for membranes such as a nylon filter, those that can be used are those usually selected from the art, and can include, for example, the nylon filter [Hyborid-N, Amersham] and the like.

The above alkali denaturation solution, neutralization solution and buffer can be used by selecting those usually used in the art. The alkali denaturation solution can include, for example, the solution containing 0.5 M NaOH and 1.5 M NaCl and the like, the neutralization solution can include, for example, 0.5 M Tris-HCl buffer, pH 8.0 containing 1.5 M NaCl and the like, and the buffer can include, for example, 2xSSPE (0.36 M NaCl, 20 mM NaH₂PO₄ and 2 mM EDTA) and the like.
It is preferred that prehybridization treatment is performed for the transferred membrane prior to the hybridization treatment, if necessary, to prevent non-specific hybridization reactions. The prehybridization treatment can be carried out, for example, by immersing in the prehybridization solution [50% formamide, 5xDenhardt's solution (0.2% bovine serum albumin, 0.2% polyvinyl pyrrolidone), 5xSSPE, 0.1% SDS, 100µ g/ml heat denatured salmon sperm DNA] and reacting from about 35°C to about 50°C, preferably at about 42°C for about 4 to 24 hrs, preferably from about 6 to 8 hrs, but those skilled in the art can determine more preferable conditions by appropriately repeating experiments for these conditions. The denaturation of the labeled probe DNA fragments used for the hybridization can be carried out by, for example, heating at about 70°C to 100°C, preferably at 100°C for about 1 min to about 60 min, preferably for about 5 min. The hybridization can be carried out by methods known in the art or methods following thereto. Herein, a stringent condition indicates, for example, the condition where the concentration of sodium is about 15 to 50 mM, preferably about 19 to 40 mM, more preferably about 19 to 20 mM, and the temperature is about 35 to 85°C, preferably about 50 to 70°C, more preferably about 60 to 65°C.

After completion of the hybridization, the filter is thoroughly washed to remove labeled probes other than the labeled probe DNA fragments having the specific hybridization reaction. The washing of the filter can be carried out by selecting among those usually used in the art and, for example, can be performed by washing with a 0.5xSSC solution (15 mM citrate buffer, pH 7.0 containing 0.15 M NaCl) containing 0.1% SDS.

Hybridized plaques can be detected representatively by autoradiography, but those appropriately selected among the methods used in the art can also be used for detection of plaques. The target recombinant phages are obtained from cultured E. coli by suspending the plaques corresponding to the detected signals in the appropriate buffer, e.g., SM solution (50 mM Tris-HCl buffer, pH7.5 containing 100 mM NaCl and 10 mM MgSO₄), diluting the phage suspension moderately to infect the E. coli, and culturing the obtained E. coli. The treatment can be repeatedly carried out if necessary in which the target recombinant phages are screened in genes or cDNA libraries by the hybridization treatment using the above probe DNA. The target recombinant phages can be yielded from the cultured E. coli by conducting an extraction, centrifugation treatments etc.

The yielded phage particles can be isolated and purified by methods usually used in the art and, for example, can be purified by the glycerol gradient ultracentrifuge method (Maniatis, T. ed., Molecular Cloning, A Laboratory Manual, Cold Spring Harbor Laboratory, 2nd ed., 78, 1989) etc. The DNA can be isolated and purified from the phage particles by methods usually used in the art and, for example, is obtained by suspending the yielded phages in TM solution (50 mM Tris-HCl buffer, pH 7.8 containing 10 mM MgSO₄), treating with DNase I and RNase A and the like, then adding a mixture solution of 20 mM EDTA, 50 µ g/ml proteinase K and 0.5% SDS, incubating at about 65°C for about one hour, extracting with phenol and diethylether, precipitating DNA by ethanol precipitation, subsequently washing the yielded DNA with 70% ethanol, and allowing to dry and dissolving in TE solution (10 mM Tris-HCl buffer, pH 8.0 containing 10 mM EDTA). Also, the target DNA can be obtained in a large amount by subcloning and, for example, the subcloning can be carried out by using E. coli as the host using plasmid vectors and the like. The obtained DNA by such a subcloning can also be isolated and purified similarly as in the above by methods such as centrifugation, phenol extraction, ethanol precipitation etc.

Chimera DNA and the like containing human MT1-MMP DNA, TIMP-2 DNA, MMP-2 DNA, nerve growth factor receptor (NGFR) DNA and parts thereof are obtained in the above ways. Others such as nucleic acids for DNA encoding the desired polypeptides can be obtained if necessary. According to the present invention, obtained are the DNA such as the DNA of human MT1-MMP with the addition of a FLAG epitope with deletion of a catalytic region, the DNA of human MT1-MMP with the addition of the FLAG epitope with deletion of PEX, chimera mutants of human MT1-MMP DNA and NGFR DNA with the addition of the FLAG epitope, chimera mutants of human MT1-MMP DNA and NGFR DNA with the addition of the FLAG epitope with deletion of the catalytic region, chimera mutants of human MT1-MMP DNA and NGFR DNA with the addition of the FLAG epitope with deletion of PEX, the DNA encoding from Cys³³⁶ to Gly⁵⁵⁰ of Met plus MT1-MMP and the like. The representative chimera mutants include those in which TM/CP of human MT1-MMP is substituted with TM/CP of NGFR.

The nucleic acids obtained in the present invention are single chain DNA, double chain DNA, RNA, DNA:RNA hybrids, synthetic DNA and the like, and may be either of human genomic DNA, human genomic DNA libraries, cDNA derived from human tissues and cells and synthetic DNA. The nucleic acids may be those which hybridize to those having the characteristic sequence specifically disclosed herein in the stringent condition. The base sequence of the nucleic acids is modified (e.g., addition, elimination, substitution etc.) by the methods known by those skilled in the art such as the gene recombination techniques as mentioned above such that it encodes the desired protein.

The gene recombination techniques can be carried out by the methods described in, for example, Sambrook, J., Fritsch, E. F. & Maniatis, T., "Molecular Cloning: A Laboratory Manual (2nd ed.)", Cold Spring Harbor Laboratory Press, Cold Spring Harbor, New York, 1989; Glover, D. M. et al. ed., "DNA Cloning", 2nd ed., Vol. 1 to 4 (The Practical Approach Series), IRL Press, Oxford University Press, 1995; "zoku-Seikagaku Jikken Koza 1, Idenshi Kenkyuhou II" ed., by Japanese Biochemical Society, Tokyo Kagaku-dojin Publishing Co., Inc., 1986; "Shin-Seikagaku Jikken Koza 2, Kakusan III (Recombinant DNA technique)" ed., by Japanese Biochemical Society, Tokyo Kagaku-dojin Publishing Co., Inc., 1992; Wu, R., ed., "Methods in Enzymology", Vol. 68 (Recombinant DNA), Academic Press, New York, 1980; Wu, R. et al., ed., "Methods in Enzymology", Vol. 100 (Recombinant DNA, Part B) & 101 (Recombinant DNA, Part C), Academic Press, New York, 1983; Wu, R. et al., ed., "Methods in Enzymology", Vol. 153 (Recombinant DNA, Part D), 154 (Recombinant DNA, Part E) & 155 (Recombinant DNA, Part F), Academic Press, New York, 1987; Miller, J. H., ed., "Methods in Enzymology", Vol. 204, Academic Press, New York, 1991; Wu, R. et al., ed., "Methods in Enzymology", Vol. 218, Academic Press, New York, 1993; Weissman, S., ed., "Methods in Enzymology", Vol. 303, Academic Press, New York, 1999; Clorioso, J. C., ed., "Methods in Enzymology", Vol. 306, Academic Press, New York, 1999 etc., or by methods described in the references cited therein or methods substantially equivalent thereto or modified methods thereof (the descriptions therein are incorporated in the disclosure of the present invention as reference).

The determination of the base sequence can be carried out using the dideoxy methods such as the M13 dideoxy method, Maxam-Gilbert method and the like and, for example, can be carried out using commercially available sequencing kits such as the Taq dye primer cycle sequencing kit, Sequenase v 2.0 kit and the like, or using an automatic sequencer apparatus, such as a fluorescence DNA sequencer apparatus and the like. Polymerases used for the dideoxy method include, for example, a Klenow fragment of DNA polymerase, AMV reverse transcriptase, Taq DNA polymerase, T7 DNA polymerase, modified T7 DNA polymerase and the like.

The DNA fragments obtained in the present invention can be incorporated into an appropriate vector, e.g., plasmid pEX, pMAMneo, pKG5, pET3a (Stratagene) and the like as described in detail below, and can be expressed in appropriate host cells, e.g., E. coli, yeast, CHO cells, COS cells and the like as described in detail below. The DNA fragments can be introduced into animal cells intact, as DNA fragments with the addition of an appropriate control sequence or by being incorporated into an appropriate vector. Transgenic animals which express the given gene can be produced. The animals include mammalian animals, and include, for example, mice, rats, rabbits, guinea pigs, cattle etc. Preferably, the transgenic animal can be produced by introducing the DNA fragments into fertilized eggs of an animal such as a mouse. The methods can be carried out according to methods known by those skilled in the art, for example, the methods described in US Patents Nos. 4,736,866 and 4,870,009.

Identification of the given gene product can be carried out using animal cells including cells derived from tumors such as COS1 cells, HT1080 cells and the like suitable therefor into which the gene has been transfected. The methods where the foreign gene is introduced into animal cells of mammals can be carried out by methods known in the art or methods substantially the same thereto, and include, for example, the calcium phosphate method (e.g., Graham, F. L. et al., Virology, 52:456, 1973, etc..), the DEAE-dextran method (e.g., Warden, D. et al., Gene Virol., 3:371, 1968, etc.), the electroporation method (e.g., Neumann, E. et al., EMBO J., 1: 841, 1982, etc.), the microinjection method, the liposome method, the viral infection method, the phage particle method and the like. The gene product produced by the animal cells transfected with the given gene in such ways can also be analyzed. Immunoprecipitation experiments or Western blotting using antibodies such as monoclonal antibodies can be used for the analysis.

Plasmids into which the given gene is incorporated are any of those which can express the DNA in the host cells usually used in gene engineering (e.g., prokaryotic hosts such as E. coli, Bacillus subtilis, etc., eukaryotic hosts such as yeast, CHO cells, COS cells etc., insect cell hosts such s Sf21 etc.) In such sequences, for example, the sequence can be modified into codons suitable for expressing in the selected host cells, restriction enzyme sites can be inserted, the sequences can be contained which regulate linkers, adapters serving to bind the target gene, and antibiotic resistance, which regulate metabolism, and which are useful for selection.

Preferably, appropriate promoters such as the tryptophan promoter (trp), lactose promoter (lac), tryptophan-lactose promoter (tac), lipoprotein promoter (lpp), λ phage P_{L} promoter and the like can be used for plasmids of which hosts are E. coli, promoters such as SV40 late promoter, MMTV LTR promoter, RSV LTR promoter, CMV promoter, SRα promoter and the like can be used for the plasmids of which hosts are animal cells, and promoters such as GAL1 and GAL10 promoters and the like can be used for the plasmids of which hosts are yeast. Moreover, regulation systems such as CYC1, HIS3, ADH1, PGK, PHO5, GAPDH, ADC1, TRP1, URA3, LEU2, ENO, TP1, AOX1 and the like can also be used.

An enhancer can be inserted into the vector to facilitate the transcription of DNA encoding the desired polypeptide, and such enhancers include those of elements having an action to facilitate the transcription acting on the promoter and typically having a cis action of approximately 10 to 100 bp. Many enhancers have been known in mammalian genes such as globin, elastase, albumin, α-fetoprotein, insulin genes and the like. Representatively, the enhancers obtained from eukaryotic infectious viruses can be suitably used, and include, for example, an SV40 enhancer located in the late region of the replication origin (100-270 bp), an enhancer of the early promoter of cytomegalovirus, enhancer located in the late region of the replication origin in polyoma, an enhancer of adenovirus and the like. A signal sequence fitting for the host can be added if necessary, and those known by those skilled in the art can be used.

The plasmids of which hosts are E. coli include, for example, pBR322, pUC18, pUC19, pUC118, pUC119, pSP64, pSP65, pTZ-18R/18U, pTZ-19R/19U, pGEM-3, pGEM-4, pGEM-3Z, pGEM-4Z, pGEM-5Zf(-), pBluescript KS™ (Stratagene) and the like. The plasmid vectors suitable for the expression in E. coli also include pAS, pKK223 (Pharmacia), pMC1403, pMC931, pKC30, pRSET-B (Invitrogen) and the like. The plasmids of which hosts are animal cells include the SV40 vector, polyoma viral vector, vaccinia viral vector, retroviral vector and the like, and include, for example, pcD, pcD-SRα , CDM8, pCEV4. pME18S, pBC12BI, pSG5 (Stratagene) and the like. The plasmids of which hosts are yeast include YIp, YEp, YRp, YCp type vectors and the like; and, for example, pGPD-2 and the like. The host cells which are E. coli include those derived from the E. coli K12 strain and, for example, NM533., XL1-Blue, C600, DH1, DH5, DH11S, DH12S, DH5 α , DH10B, HB101, MC1061, JM109, STBL2, BL21(DE3)/pLYsS and the like. The host cells which are yeast include, for example, Saccharomyces cerevisiae, Schizosaccharomyces prombe, Pichia pastoris, Kluyveromyces strains, Candida, Trichoderma reesia and the other yeast strains. The host cells which are animal cells include, for example, COS-7 cells, COS-1 cells, CV-1 cells derived from fibroblasts of African grivet, 293 cells derived from human renal cells, A431 cells derived from human epidermal cells, 205 cells derived from human colon cells, COP cells, MOP cells, WOP cells derived from murine fibroblasts, CHO cells, CHO DHFR cells derived from Chinese hamster cells, human HeLa cells, C127 cells derived from murine cells, NIH3T3 cells derived from murine cells, murine L cells, 9BHK, HL-60, U937, HaK and Jurkat cells, other cell lines obtained by transformation, normal diploid cells, cell lines induced from primary cultured tissue in vitro, and the like. Insect cells include cells using Spodoptera frugiperda (caterpillar), Aedes aegypti (mosquito), Aedes albopictus (mosquito), Drosophila melanogaster (fruitfly), silk worm larva or cultured cells, e.g., BM-N cells using silk worm (Bombyx mori nuclear polyhedrosis virus) or those derived therefrom or other suitable ones as the vector (for example, Luckow et al., Bio/Technology, 6:47-55, 1988; Setlow, J.K. et al., ed., Genetic Engineering, Vol. 8, pp. 277-279, Plenum Publishing, 1986; Maeda et al., Nature, 315:592-594, 1985). Utilizing Agrobacterium tumefaciens etc., it is possible to use plant cells as the host cells, which have been widely known along with vectors suitable therefor in the art.

In the gene engineering techniques of the present invention, it is possible to use restriction enzymes, reverse transcriptases known and widely used in the art, DNA modification enzymes, DNase, DNA polymerases, terminal nucleotidyltransferases, DNA ligases and the like to modify or convert DNA into a structure suitable for cloning the DNA fragments. For example, restriction enzymes include those described in., for example, Roberts, R. J., Nucleic Acids Res., 13: r165, 1985; Linn, S. et al., ed., Nucleases, p.109, Cold Spring Harbor Lab., Cold Spring Harbor, New York, 1982; Roberts, R. J., Macelis, D., Nucleic Acids Res., 19:Suppl. 2077, 1991 and the like. Reverse transcriptases include, for example, reverse transcriptases derived from mouse Moloney leukemia virus (MMLV), avian myeloblastosis virus (AMV), and the like. As the reverse transcriptase, the RNase H defective is preferably used, especially, the modified MMLV RT defective for RNase H is preferably used, and those with heat stability are preferable. The suitable reverse transcriptases include MMLV RT (Gibco-BRL), Superscript RT plus (Life Technologies), and the like.

DNA polymerases include, for example, E. coli DNA polymerase, the Klenow fragment which is a derivative thereof, E. coli phage T4 DNA polymerase, E. coli phage T7 DNA polymerase, heat resistant bacterial DNA polymerase and the like. Terminal nucleotidyltransferases include, for example, TdTase which adds deoxyribonucleotide (dNMP) to 3'-OH terminus described in Wu, R. et al., "Methods in Enzymology" Vol. 100, p96, Academic Press, New York, 1983, and the like. DNA modification enzymes and DNases include exonucleases and endonucleases and the like, and include, for example, snake venom phosphodiesterase, splenic phosphodiesterase, E. coli DNA exonuclease I, E. coli DNA exonuclease III, E. coli DNA exonuclease VII, λ exonuclease, DNase I, nuclease S1 micrococcus nuclease and the like. DNA ligases include, for example, E. coli DNA ligase, T4 DNA ligase and the like. Vectors suitable for constructing DNA libraries by cloning DNA genes include plasmids, λ phages, cosmids, P1 phage, F element, YAC and the like, and include preferably vectors derived from λ phages, and, for example, Charon 4A, Charon 21A, λ gt10, λ gt11, λ DASHII, λ FIXII, λ EMBL3, λ ZAPII™ (Stratagene) and the like.

Appropriate selected markers are used, if necessary, for the transformed cells transformed with the expression vector containing the nucleic acids encoding the protein of the present invention. A cell line stably having a high expression ability can be obtained by cloning repeatedly. For instance, when a dhfr gene is utilized as a selected marker in the transformed cell using animal cells as the host cells, cell lines with higher expression can be obtained by amplifying the DNA encoding the protein of the present invention by culturing with a gradual increase in MTX concentration and selecting the resistant cell lines. The transformed cells of the present invention can be cultured under a condition where the nucleic acids encoding the protein of the present invention can be expressed, and can produce and accumulate the target. The transformed cells can be cultured in media widely used in the art. For instance, the transformed cells of which hosts are prokaryotic cells such as E. coli and Bacillus subtilis, or yeast cells can suitably used with liquid media. The media contain carbon sources, nitrogen sources, minerals, and others essential for growth of the transformed cells. The carbon sources include, for example, glucose, dextrin, soluble starch, sucrose and the like, the nitrogen sources include, for example, inorganic or organic materials such as ammonium salts, nitrates, cornsteep, liquor, peptone, casein, meat extract, wheat germ extract, soy bean bran, potato extract solution and the like, and the inorganic materials include, for example, potassium chloride, potassium dihydrogen phosphate, magnesium chloride, calcium carbonate and the like. Also, yeast, vitamins, casamino acids, growth factors and the like may be added. Also, reagents such as 3β -indolyl acrylate can be added, if necessary, to operate the promoter efficiently. The range of pH in the media is preferably about 5 to 8.

The culture of , for example, E. coli is typically carried out at about 15 to 45°C for about 3 to 75 hrs, and ventilation and stirring can be added if necessary. When the transformed cells of which the hosts are animal cells are cultured, for example, MEM medium, PRMI 1640 medium, DMEM medium and the like containing about 5 to 20% fetal calf serum are used as the medium. The range of pH is preferably about 6 to 8. The culture is typically carried out at about 30 to 40°C for about 15 to 72 hrs, and ventilation and stirring are added if necessary. The obtained cells and media can be used just as they are.

When the desired product is extracted from the above cultured cells, methods can be appropriately used in which the bacteria or cells are collected by methods known in the art after the culture, suspended in an appropriate buffer followed by being disrupted by sonication, lysozyme and/or freezing/thawing and the like, and subsequently the crude extract solution is obtained by centrifugation or filtration. Protein denaturants such as urea, guanidine hydrochloride and the like and surfactants such as Triton X-100 (trade name), Tween-80 (trade name) and the like may be added in the buffer. When the target product is secreted in the culture medium, a supernatant is separated from the bacteria or cells after completion of the culture by methods known in the art, and the supernatant is collected. The target product contained in the culture supernatant or the extract solution obtained in such ways can be purified by appropriately combining the isolation and purification methods known in the art. For instance, the product can be obtained by purifying by the methods such as salting out ammonium sulfate precipitation, the gel filtration methods with Sephadex and the like, the ion exchanging chromatography methods using carriers such as diethylaminoethyl groups or carboxymethyl groups and the like, the hydrophobic chromatography methods using carriers having hydrophobic groups such as butyl, octyl, phenyl groups and the like, the dye gel chromatography methods, electrophoresis, dialysis, ultrafiltration, the affinity chromatography methods, the high performance liquid chromatography methods, and the like. Preferably, the product can be isolated and purified by treating by the polyacrylamide gel electrophoresis, the affinity chromatography immobilized antibodies such as monoclonal antibodies which react specifically with antigens, and the like. For example, included are gelatin-agarose affinity chromatography, heparin-agarose chromatography and the like.

According to the present invention, provided is a screening method for substances which inhibit formation of the complex formed of multiple MT1-MMP, e.g., the formation of homodimer of MT1-MMP, using a system in which MT1-MMP and proMMP-2 co-exist. For instance, substances which inhibit the formation of homodimer of MT1-MMP can be screened using a chimera molecule (including the transformed cells which express the molecule or the culture thereof) in which TM/CP of MT1-MMP is substituted with TM/CP of NGFR.

In the screening, for example, using the transformed cells which express the chimera molecule in which TM/CP of MT1-MMP is substituted with TM/CP of NGFR, or the culture thereof, phosphorylation of the chimera molecule is compared between (i) in the absence of the test sample and (ii) in the case of contacting with the test sample. In addition, in the above screening, the biological activities (for example, protease activity, activation of proMMP-2 induced by the homodimer formation of MT1-MMP, or cellular migration, invasion and/or metastasis attributed thereto) can be determined and compared.

The chimera molecules can be used in intact, or by labeling with fluorescence such as fluorescein, enzymes, radioisotopes and the like. Those which can be preferably used as appropriate tags are added using the DNA recombination techniques or labels are added by chemical techniques. The determination of the phosphorylation can be carried out by methods known in the art, which include the method detecting the phosphorylation of tyrosine residues induced at the site of TM/CP of NGFR, with anti-phosphotyrosine antibody and the like. The test samples include, for example, proteins, peptides, non-peptide compounds, synthetic compounds, fermented products, plant extracts, tissue extracts from animals, cellular extracts and the like. Examples of the test compounds used for the test sample may include preferably anti-MMP antibodies, MMP inhibitors, compounds and especially synthetic compounds having the inhibitory activity against MMP family members. In particular, preferably included are anti-PEX antibodies, pseudo-PEX compounds and the like. These compounds may be novel compounds or compounds known in the art. Screening can be carried out according to determination methods known in the art. Also, it can be done using various markers, buffer systems and other appropriate reagents and the like, or according to operations as described in the determination methods using the following antibodies. The measurement can be typically carried out in the buffer such as Tris-HCl buffer, phosphate buffer and the like which has no adverse effect, for example from pH4 to 10 (preferably about pH6 to 8).

In such respective screening methods, the determination systems may be constructed which are associated with the MT1-MMP complex formation of the present invention, by adding normal technical consideration of those skilled in the art to typical conditions and the operating method in the respective methods. Reviews and books can be cited for details of these general technical means [for example, see Methods in Enzymology, Vols. 1, 2, 5 & 6 (Preparation and Assay of Enzymes); ibid., Vol. 3 (Preparation and Assay of Substrates); ibid., Vol. 4 (Special Techniques for the Emzymologist); ibid., Vol. 19 (Proteolytic Enzymes); ibid., Vol. 45 (Proteolytic Enzymes, Part B); ibid., Vol. 80 (Proteolytic Enzymes, Part C), all published from Academic Press USA].

As used herein, the term "antibody" is used in the broadest sense and may cover a single species of desirable monoclonal antibodies against PEX domain protein fragments and antibody compositions having a specificity to various epitopes thereof, further monovalent or polyvalent antibodies and polyclonal and monoclonal antibodies, and also those which are intact molecules or fragments and derivatives thereof, including F(ab')₂, Fab' and Fab fragments, and also chimeric antibodies, hybrid antibodies each having at least two antigen or epitope binding sites, or bispecific recombinant antibodies (e.g., quadromes, triomes, etc.), interspecies hybrid antibodies, anti-idiotypic antibodies and those which have been chemically modified or treated and must be regarded as derivatives of these antibodies and further which may be produced either by adopting cell fusion or hybridoma techniques or antibody engineering or by using synthetical or semisynthetical techniques in known manner, which may be prepared either by the known conventional methods in view of antibody production or by recombinant DNA techniques, and which have neutralizing or binding properties with respect to the target antigen substances or target epitopes described and defined herein.

Monoclonal antibodies prepared against antigenic substances are produced by any method capable of providing production of antibody molecules by a series of cell lines in culture. The modifier "monoclonal" indicates the character of the antibody as being obtained from a substantially homogeneous population of antibodies, and is not to be construed as requiring production of the antibody by any particular method. The individual antibodies are those containing a population of identical antibodies except for possible naturally occurring mutations that may be present in minor amounts.
Monoclonal antibodies are highly specific, being directed against a single antigenic site. In contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The monoclonal antibodies included within the scope of the invention include hybrid and recombinant antibodies. They are obtainable by substituting a constant domain of an antibody for a variable domain (e.g., "humanized" antibodies), or a heavy chain for a light chain, by substituting a chain from one species with a chain from another species, or by fusing to heterogeneous proteins, regardless of species of origin or immunoglobulin class or subclass designation, so long as they exhibit the desired biological activity (e.g., U.S. Pat. No. 4,816,567; Monoclonal Antibody Production Techniques and Applications, pp.79-97, Marcel Dekker, Inc., New York, .1987; etc.).

Preferable techniques for producing monoclonal antibodies include, for example, the methods using hybridoma cells (G. Kohler and C. Milstein, Nature, 256: 495-497, 1975); the methods using human B cell hybridomas (Kozbor et al., Immunology Today, 4: 72-79, 1983; Kozbor, J. Immunol., 133: 3001, 1984; Brodeur et al., Monoclonal Antibody
Production Techniques and Applications, pp.51-63, Marcel Dekker, Inc., New York (1987); triome methods; EBV-hybridoma methods (Cole et al., Monoclonal Antibodies and Cancer Therapy, Alan R. Liss, Inc., pp.77-96 (1985))(techniques for production of human monoclonal antibodies); U.S. Pat. No. 4,946,778 (techniques for production of single-chain antibodies), as well as the following documents:
S. Biocca et al., EMBO J, 9: 101-108, 1990; R.E. Bird et al., Science, 242: 423-426, 1988; M.A. Boss et al., Nucl. Acids Res., 12: 3791-3806, 1984; J. Bukovsky et al., Hybridoma, 6: 219-228, 1987; M. DAINO et al., Anal. Biochem., 166: 223-229, 1987; J.S. Huston et al., Proc. Natl. Acad. Sci. USA, 85; 5879-5883, 1988; P.T. Jones et al., Nature, 321: 522-525, 1986; J.J. Langone et al. (ed.), "Methods in Enzymology", Vol. 121 (Immunochemical Techniques, Part I: Hybridoma Technology and Monoclonal Antibodies), Academic Press, New York (1986); S. Morrison et al., Proc. Natl. Acad. Sci. USA, 81: 6851-6855, 1984; V.T. Oi et al., BioTechniques, 4: 214-221, 1986; L. Riechmann et al., Nature, 332: 323-327, 1988; A. Tramontano et al., Proc. Natl. Acad. Sci. USA, 83: 6736-6740, 1986; C. Wood et al., Nature, 314: 446-449, 1985; Nature, 314: 452-454, 1985, or documents quoted therein (the disclosures of which are incorporated herein by reference).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they have the desirable biological activity (U.S. Pat. No. 4,816,567; Morrison et al., Proc. Natl. Acad. Sci. USA, 81: 6851-6855, 1984).

Described herein below is the production of antibodies, including embodiments of monoclonal antibodies.

It goes without saying that the monoclonal antibody to be used in the present invention may be a monoclonal antibody obtained by utilizing cell fusion techniques with myeloma cells.

The monoclonal antibody to be used in the present invention can be produced by the following processes:
1. Preparation of immunogenic antigens (immunogens)
2. Immunization of animals with immunogenic antigens
3. Preparation of myeloma cells
4. Cell fusion between antibody-producing cells and myeloma cells
5. Selection and cloning of hybridomas (hybrid cells)
6. Production of monoclonal antibodies

### 1. Preparation of immunogenic antigens

The antigen as used herein includes isolated MT1-MMP PEX protein fragments and derivatives thereof, produced, as disclosed above, and suitable synthetic oligopeptides, chemically synthesized, based on information on amino acid sequences for the PEX domain of MT1-MMP sequenced (GenBank™ /EMBL accession number: D26512). Although the antigen may be used to immunize animals after being mixed with a suitable adjuvant without any modifications, it can be used after formation of immunogenic conjugates. The antigen for such an immunogen may be selected from at least three consecutive amino acid residues from the PEX domain of MT1-MMP (e.g., the region ranging from amino acid residue 316 to acid residue 508 among the MT1-MMP amino acid sequence, or the region from amino acid residue 319 to amino acid residue 535 among the MT1-MMP amino acid sequence in some cases). For instance, the antigens may be a fragment derived from MT1-MMP PEX, or a synthetic polypeptide fragment obtained via selecting characteristic sequence areas based on the amino acid sequences of MT1-MMP PEX areas followed by design and chemical synthesis. The fragments may be coupled with various carrier proteins via suitable coupling agents to form immunogenic conjugates such as hapten-proteins. The immunogenic conjugates can be used to design monoclonal antibodies that can react with (or recognize) specific sequences exclusively. A cysteine residue or the like can be added to the polypeptide thus designed so as to prepare an immunogenic conjugate easily. To couple with a carrier protein or the like, the carrier protein is first activated. This activation may include incorporation of an activated binding group thereinto, etc. The activated binding groups include (1) active ester or active carboxyl groups such as a nitrophenyl ester group, a pentafluorophenyl ester group, a 1-benzotriazol ester group, and an N-succinimido ester group; (2) active dithio groups such as a 2-pyridyldithio group, etc. The carrier proteins include keyhole limpet haemocyanin (KLH), bovine serum albumin (BSA), ovalbumin, globulin, polypeptides such as polylysine, bacterial components such as BCG or the like.

### 2. Immunization of animals with immunogenic antigens

Animals can be immunized according to techniques as described in Shigeru MURAMATSU et al. ed., "Jikken Seibutsu Gaku Kouza 14, Men-eki Seibutsu Gaku (Lectures on Experimental Biology 14, Immunobiology)", Maruzen K.K., 1985; Nippon Seikagaku Kai (Biochemical Society of Japan) ed., "Zoku-Seikagaku Jikken Kouza 5, Men-eki Seikagaku Kenkyuho (Lectures on Biochemical Experiments (Second Series; 5), Methods for Immunological and Biochemical Study)", Tokyo Kagaku Dojin, Japan (1986); Nippon Seikagaku Kai (Biochemical Society of Japan) ed., "Shin-Seikagaku Jikken Kouza 12, Bunshi Men-eki Gaku III (Kougen-Koutai-Hotai) (New Lectures on Biochemical Experiments 12, Molecular Immunology III (Antigen-Antibody-Complement))", Tokyo Kagaku Dojin, Japan (1992); etc., the disclosures of which are hereby incorporated by reference. Immunization'can be performed in a mammal, for example, by one or more injections of an immunizing agent (and, if desired, an adjuvant). Typically, the immunizing agent and/or adjuvant will be injected in the mammal by multiple subcutaneous or intraperitoneal injections. The immunizing agent may include the aforementioned antigen peptides or recombinant MT1-MMP proteins or recombinant MT1-MMP PEX obtained by DNA cloning or their fragments obtained by enzymatic digestion thereof. It may be useful to conjugate the immunizing agent to a protein known to be immunogenic in the mammal being immunized. Examples of such immunogenic proteins which may be employed include the aforementioned carrier proteins. The adjuvant to be used with the antigen includes Freund's complete adjuvant, Ribi adjuvant, pertussis vaccine, BCG, liposome, aluminium hydroxide, silica, lipid A, synthetic trehalose dicorynomycolate (TDM) adjuvant, etc.

The Immunization is carried out with suitable animals, including mice such as BALB/c, hamsters, and others. The antigen dose is, for example, approximately 1 to 400µ g/animal for mice. Generally, the antigen is injected intraperitoneally or subcutaneously into a host animal, followed by additional immunization by repeated courses wherein intraperitoneal, subcutaneous or intravenous administrations are carried out approximately 2 to 10 times at 1- to 4-week intervals, preferably 1- to 2-week intervals. For immunization, BALB/c mice, as well as F1 mice between BALB/c mice and other mice, etc. can be used. As required, the levels of animal immunization can be assessed by constructing a system for measuring a titer of antibody and measuring the titer of an antibody. The antibody of the present invention may include those obtainable from such immunized animals, for example, anti-serum, polyclonal antibodies, etc.

### 3. Preparation of myeloma cells

Immortal cell strains (tumor cell lines) to be used for cell fusion can be selected from non-immunoglobulin-producing cell lines. The cell strains to be used for cell fusion may include, for example, P3-NS-1-Ag4-1 (NS-1, Eur. J. Immunol., 6: 511-519, 1976), SP-2/0-Ag14 (SP-2, Nature, 276: 269-270, 1978), mouse myeloma MOPC-21 cell line-derived P3-X63-Ag8-U1 (P3U1, Current topics Microbiol. Immunol., 81: 1-7, 1978), P3-X63-Ag8 (X63, Nature, 256: 495-497, 1975), P3-X63-Ag8-653 (653, J. Immunol., 123: 1548-1550, 1979), etc. 8-Azaguanine resistant mouse myeloma cell lines can be sub-cultured in a medium for cell culture, such as Dulbecco's modified Eagle's medium (DMEM) or RPMI-1640 medium, supplemented with antibiotics such as penicillin, amikacin or the like, fatal calf serum (FCS) or the like and 8-azaguanine (for example, 5 to 45µ g/ml). The specified number of cell lines can be prepared by passage the normal medium two or five days before cell fusion. The cell lines to be used may be cultured on the normal medium after the frozen and preserved strains have been completely thawed at approximately 37°C and have been washed on the normal medium such as RPMI-1640 three or more times, and the specified number of cell strains may be prepared.

### 4. Cell fusion between antibody-producing cells and myeloma cells

After animals such as mice are immunized according to the above step 2, their spleens are taken out in two to five days from final immunization, and the spleen cell suspension is obtained. In addition to the spleen cells, lymph node cells at various sites of organisms can be obtained and used for cell fusion. The spleen cell suspension thus obtained and the myeloma cell strains obtained by the above step 3 are placed in a medium such as minimum essential medium (MEM), DMEM and RPMI-1640 medium, followed by addition of an agent for cell fusion, such as polyethylene glycol. A widely-used agent for cell fusion can be used, including inactivated HVJ (Hemagglutinating virus of Japan, "Sendai virus") and the like. Preferably, 0.5 to 2 ml of 30 to 60% polyethylene glycol can be added. Polyethylene glycol with molecular weights from 1,000 to 8,000 can be employed, more preferably, polyethylene glycol with molecular weights from 1,000 to 4,000. The preferred concentration of polyethylene glycol in the fusion medium is from 30 to 60%. As required, a small amount of dimethyl sulfoxide or the like is added to promote fusion. The ratio of spleen cells (lymphocytes) : myeloma cell lines to be used for fusion is preferably 1:1 to 20:1, and preferably falls within 4:1 to 7:1. The fusion reaction is conducted for 1 to 10 min, before the addition of a medium such as RPMI-1640 medium. Fusion reaction can be done several times. After fusion reaction, cells are separated by a centrifuge, then transferred to the selection medium.

### 5. Selection and cloning of hybridomas (hybrid cells)

The selection media include conventionally known "HAT medium", i.e., FCS-containing MEM, RPMI-1640 medium, etc., supplemented with hypoxanthine, aminopterin, and thymidine. The replacement method for the selection medium is to replenish an amount equivalent to the capacity dispensed to the medium plate on the following day, after which the medium is replaced by half an amount in HAT medium every one to three days. The replacement can be modified depending on situations. Eight to sixteen days after fusion, the medium may be replaced every one to four days with conventionally known "HT medium" wherein aminopterin is excluded. As a feeder cell, for example, mouse thymocyte can be used, which is sometimes effective.

The supernatant of the culture well with vigorously growing hybridoma is screened by using a predetermined peptide fragment as an antigen or by using a labeled anti-mouse antibody for measuring target antibodies, with a measuring system such as radioimmunoassay (RIA), enzyme-linked immunosorbent assay (ELISA), fluorescence immunoassay (FIA) or by the fluorescence activated cell sorter (FACS), etc. The target antibody-producing hybridoma is cloned. Cloning is carried out by picking up colonies in the agar medium or by the limiting dilution. The limiting dilution is preferred. Cloning should be performed several times.

### 6. Production of monoclonal antibodies

The obtained hybridoma cells are cultured in a suitable growth medium such as FCS-containing MEM, RPMI-1640 medium or the like, and a desired monoclonal antibody can be obtained from the culture supernatant. Large amounts of monoclonal antibodies can be produced by propagating hybridomas as ascites tumors, etc. In this case, each hybridoma is implanted intraperitoneally in a histocompatible animal isogenic to an animal from which the myeloma cell is derived and is propagated. Or each hybridoma can be inoculated, for example, in nude mice, and propagated to produce the monoclonal antibody in the ascites of the animals. The produced monoclonal antibody can be collected from the ascetic fluid and obtained. Prior to implantation of hybridomas, the animal is pretreated intraperitoneally with mineral oils such as Pristane (2,6,10,14-tetramethylpentadecane). After the pretreatment, the hybridoma can be propagated therein and the ascitic fluid can be harvested. The ascitic fluid can be used as a monoclonal antibody without purification or after purification by conventionally known methods, including salting out such as precipitation with ammonium sulfate, gel filtration with Sephadex and the like, ion exchange chromatography, electrophoresis, dialysis, ultrafiltration, affinity chromatography, high-performance liquid chromatography, etc. The isolated or purified products can be employed as monoclonal antibodies. Preferably, the monoclonal antibody-containing ascitic fluid is fractionated with ammonium sulfate and separated and purified by treatments with anion exchange gel such as DEAE-Sepharose, an affinity column such as protein A column, etc. More preferably, it is treated with affinity chromatography using immobilized antigens or antigen fragments (for example, synthetic peptides, recombinant antigen proteins or peptides, portions which the antibody can specifically recognize, etc.); affinity chromatography with immobilized protein A; hydroxyapatite chromatography; etc.

In addition, it is possible to use transgenic mice and other organisms including other mammals for expressing antibodies such as humanized antibodies against the immunogenic polypeptide products of the present invention.

It is also possible to produce antibodies with recombinant DNA techniques wherein the antibody thus obtained in a large amount is sequenced and/or a nucleotide sequence coding for the antibody obtained from the hybridoma cell line is employed.

DNA coding for the monoclonal antibodies is readily isolated and sequenced using conventional procedures (e.g., by using oligonucleotide probes that are capable of binding specifically to genes encoding the heavy or light chain of murine antibodies). Once isolated, the DNA may be placed, according to the aforementioned techniques, into expression vectors, which are then transfected into host cells such as CHO cells or COS cells. The DNA may be modified, for example, by substituting the coding sequence for human heavy- and light-chain constant domains for the homologous murine sequences (Morrison et al., Proc. Natl. Acad. Sci. USA, 81: 6581, 1984). Thus, chimeric and hybrid antibodies having a desired binding specificity can be prepared. Further, antibodies can be modified, including preparations of chimeric or hybrid antibodies by adaptations of known methods in synthetic protein chemistry, including those involving coupling agents as listed hereinbelow.

Humanized antibodies are achievable by known techniques in the art (e.g., Jones et al., Nature, 321: 522-525, 1986; Riechmann et al., Nature, 332: 323-327, 1988; Verhoeyen etal., Science, 239: 1534-1536, 1988).

Human monoclonal antibodies can be achieved according to known techniques in the art. For producing human monoclonal antibodies, human myeloma cells and human-mouse hetero-myeloma cells are known in the art (Kozbor, J. Immunol., 133: 3001, 1984; Brodeur et al., Monoclonal Antibody Production Techniques and Applications, pp.51-63, Marcel Dekker, Inc., New York (1987)). Methods for making bispecific antibodies are known in the art (Millstein et al., Nature, 305: 537-539, 1983; WO, A, 93/08829; Traunecker et al., EMBO J., 10: 3655-3659, 1991; Suresh et al., "Methods in Enzymology", Vol. 121, p.210 (1986)).

These antibodies may be treated with enzymes such as trypsin, papain, pepsin and others and occasionally be subjected to reduction to produce antibody fragments including Fab, Fab', and F(ab')₂. These antibody fragments may be occasionally used.

When employed as pharmaceutical agents, the active components of the present invention (e.g., (a) PEX protein of MT1-MMP (MT1PEX), peptide fragments and salts thereof, or mutants, analogs, derivatives thereof, (b) nucleic acids (including DNA, etc.) each encoding the PEX domain of MT1-MMP, (c) the antibodies (including monoclonal antibodies) of the present invention or derivatives thereof, and (d) compounds, or salts thereof, which suppress or inhibit a biological activity such as homodimer formation of MT1-MMP, etc,) may be administered usually in the form of a pharmaceutical composition or preparation alone or in admixture with a variety of pharmaceutically acceptable aids. For example, MT1PEX or a salt thereof can be administered as such forms. Preferably they may be administered in the form of a convenient pharmaceutical composition or formulation suitable for oral, topical, parenteral application, or the like. Any of dosage forms (including those for inhalation and rectal administration) may be selected depending on purpose.

The active components of the present invention can also be used in admixture with anti-tumor agents (antineoplastic agents) and/or tumor-metastasis inhibitors. Any of the anti-tumor agents (antineoplastic agents) and/or tumor-metastasis inhibitors can be used without any limitation so long as they advantageously serve. Examples of such drugs are selected from drugs known in the art.

The parenteral administration includes topical, percutaneous, intravenous, intramuscular, subcutaneous, intracutaneous, and intraperitoneal routes. It is also possible to apply the drug directly to affected sites, and, in a certain case, the direct application is suitable. Preferably mammals including human can receive the drug orally or parenterally (e.g., intracellularly, intra-tissularly, intravenously, intramuscularly, subcutaneously, intracutaneously, intraperitoneally, intrapleurally, intraspinally, by instillation, enterally, per rectum, by instillation into the ear, eye, or nose, by swabbing or application on the teeth, skin or mucosa, etc.). Specific dosage forms of the pharmaceutical preparations and formulations include pharmaceutical solutions, pharmaceutical dispersions, semisolid preparations, particulate preparations, shaped preparations, extractives, etc. Examples of the dosage forms are tablets, coated tablets, sugar coated tablets, pills, troches, hard capsules, soft capsules, microcapsules, implants, powders, pulvises, granules, fine granules, injections, liquids and solutions, elixirs, emulsions, irrigations, syrups, aqueous mixtures, milks, suspensions, liniments, lotions, aerosols, sprays, inhalations, nebula, ointments, plasters, patches, pastes, cataplasms, creams, oleates, suppositories (e.g., rectal suppositories), tinctures, dermatologic waters, ophthalmic solutions, collunariums, auristillae, paints, transfusions, powders for injection solutions, lyophilized preparations, conditioned gels, etc.

The pharmaceutical compositions can be formulated in accordance with conventional techniques. For example, the pharmaceutical composition or formulation may comprise at least one of said compounds (active components) of the present invention or a salt alone or in admixture with physiologically allowable carriers, pharmaceutically acceptable carriers, adjuvants, vehicles, excipients, diluents, etc. The compound (active component) of the present invention or a salt thereof is usually admixed with a single member selected from the group consisting of physiologically allowable carriers, pharmaceutically acceptable carriers, adjuvants, vehicles, excipients, diluents, flavoring agents, perfuming agents, sweetening agents, expanders, antiseptics, stabilizers, binders, pH regulators, buffering agents, detergents (surfactants), bases, solvents, fillers, bulking agents, solution adjuvants, solubilizers, isotonizing agents, emulsifiers, suspending agents, dispersers, thickeners, gelling agents, hardeners, absorbents, adhesives, elastomers, plasticizers, disintegrants, aerosol propellants, preservatives, antioxidants, opacifying agents, humectants, emollients, charge protectors, soothing agents, etc., or suitably in a combination thereof, depending on necessity, to give a unit dose form which is required for generally approved pharmaceutical practices.

Formulations suitable for parenteral routes include aseptic solutions or suspensions containing at least one active component in admixture with water or other pharmaceutically acceptable media. Examples of such parenteral formulations are injections. Preferred liquid carriers for injection generally include water, saline, dextrose solution, other related saccharide solutions, ethanol, glycols such as propylene glycol and polyethylene glycol, etc. For the preparation of injections, the active component of the present invention is usually admixed with any of carriers such as distilled water, Ringer's solution, physiological saline, suitable dispersing agents, moistening agents, suspending agents, etc. to form injectable formulations including solutions, suspensions, emulsions, etc. by known techniques in the art.

Examples of aqueous liquids for the injection are a physiological saline and isotonic solutions containing glucose and other aids (e.g., D-sorbitol, D-mannitol, sodium chloride, etc.), etc. The aqueous liquid may be used in admixture with a suitable pharmaceutically acceptable solubilizing aid such as alcohol (e.g., ethanol, etc.), polyalcohol (e.g., propylene glycol, polyethylene glycol, etc.), nonionic surface-active agent (e.g., Polysorbate 80™, HCO-50, etc.), etc. The injectable oily liquids may include sesame oil, soybean oil, etc. Such oily liquids may be used in admixture with solubilizing aids such as benzyl benzoate, and benzyl alcohol. They may also be admixed with buffers (e.g., phosphate buffer, sodium acetate buffer, etc.) or agents for osmoregulation, analgesic agents (e.g., benzalkonium chloride, procaine hydrochloride, etc.), stabilizers (e.g., human serum albumin, polyethylene glycol, etc.), preservatives (e.g., benzyl alcohol, phenol, etc.), antioxidants such as ascorbic acid, absorbefacients, etc. The prepared injection solution is usually filled in suitable ampoules.

For parenteral administration, solution or suspension unit dosage forms are prepared in pharmaceutically acceptable sterile fluids such as water, ethanol, and oils, in admixture with or without detergent and other pharmaceutically acceptable aids. The oily vehicle and solvent used in the parenteral formulation may include natural, synthetic or semi-synthetic mono-, di-, or triglycerides; natural, semi-synthetic or synthetic fats and oils; and fatty acids. Examples of such oily vehicles and solvents are plant oils such as peanut oil, corn oil, soybean oil, and sesame oil. For example, this injection can usually be prepared to form unit doses each containing approximately from 0.1 to 10 parts of the compound of the present invention per 100 parts by weight of the dose composition.

The formulation suitable for topical use, such as buccal.or rectal application, includes mouthwashes and gargles, dentifrices, sprays for cavitas oris, inhalants, ointments (salves), dental fillers, dental coating agents, dental pastes, suppositories, etc. The mouthwashes and other dental preparations are produced by conventional techniques, using pharmaceutically acceptable carriers. For the sprays for cavitas oris and inhalants, the compound of the present invention can be applied to teeth, etc. in the form of an aerosol or solution for nebulizers in which it is dissolved alone or in combination with pharmaceutically acceptable inert carriers, or in the form of powders for inhalation. The ointments (salves) are prepared by conventional techniques, in admixture with conventionally employed pharmaceutical bases such as ointment bases (white petrolatum, paraffin, olive oil, macrogol 400, macrogol ointment, etc.).

The pharmaceutical drugs for topical application (including painting) to teeth and skin can be prepared in the form of a solution or suspension utilizing suitably sterilized water or non-aqueous vehicles. The additives used include buffering agents such as sodium bisulfite and disodium edetate; preservatives including antiseptic, antimicrobial and antifungal agents such as acetic acid, phenylmercuric nitrate, benzalkonium chloride and chlorhexidine; and thickeners such as hypromellose.

The suppositories can be prepared by conventional techniques utilizing carriers well known in the art, preferably suitable non-irritative excipients. The excipients are preferably those which are solid at room temperature but liquid at rectal temperature wherein such substances melt in the rectum to deliver a drug. Examples of the excipients are polyethylene glycols, lanolin, cacao butter, fatty acid triglycerides, etc. The suppositories are ordinarily prepared in the form of compositions containing the compound of the present invention at ranges of approximately from 0.1 to 95 wt%. The ingredients, depending on the vehicle and concentration used, can be either suspended or dissolved in the vehicle. Adjuvants such as local anesthetics, preservatives and buffering agents can be dissolved in the vehicle.

The formulations suitable for oral application include solid compositions such as tablets, pills, capsules, powders, granules, and troches; fluid compositions such as solutions, syrups, and suspensions; etc. In preparing oral formulations, pharmaceutical adjuvants known in the art are employed. The tablets and pills can be prepared further by enteric coating. When the unit dosage form is a capsule, fluid carriers such as fats and oils can be contained in addition to the aforementioned materials.

Further, for the therapeutic and/or prophylactic agents, as aforementioned, containing the nucleic acids (including DNA) of the present invention, said nucleic acid can be applied alone, or by ligation with suitable vectors used in genetic recombination techniques (or recombinant DNA techniques),including virus-derived vectors such as vectors derived from retrovirus. The nucleic acids (including DNA) of the present invention can be administered by conventional known techniques, in unmodified forms or in forms formulated in admixture with suitable aids or physiologically acceptable carriers, for example, to promote transfer into intracellular compartments. The nucleic acids (including DNA) of the present invention can be presented for administration to humans and animals in pharmaceutical composition or preparations as aforementioned. For administration of the nucleic acids (including DNA) of the present invention, techniques known as gene therapy can also be applied.

Dose levels of said active component according to the present invention may vary within a wide range. Specific dose levels and administration cycles for any particular patient will be employed depending upon a variety of factors including the activity of specific compounds employed, the sex, age, body weight, general health, diet, time of administration, route of administration, rate of excretion, drug combination, and the severity of the particular disease undergoing therapy, or other factors.

For the manufacture of pharmaceutical compositions and preparations, the additives, etc., preparation methods and the like can be suitably selected, depending on necessity, from those disclosed in Nippon Yakkyokuho Kaisetsusho Henshu Iinkai (ed.), "13th ed. Nippon Yakkyokuho Kaisetsusho (Commentary on The Pharmacopoeia of Japan, 13th ed.)", July 10, 1996, Hirokawa Pub. Co., Tokyo, Japan; Hisashi Ichibagade et al. (ed.), "Pharmaceutical Research and Development (Ikuo Suzuki, chief editor), Vol. 12 (Pharmaceutical Necessities 1)", October 15, 1990, Hirokawa Pub. Co., Tokyo, Japan; ibid., Vol. 12 (Pharmaceutical Necessities 2), October 28, 1990, Hirokawa Pub. Co., Tokyo, Japan; etc., all the disclosures of which are incorporated herein by reference.

The active components of the present invention are not limited as long as they serve as suppressors and/or inhibitors against biological activities such as the homodimer formation of MT1-MMP. Preferable examples of the active components include substances having an advantageous property. The active components of the present invention include for example, (a) PEX protein of MT1-MMP, fragments or salts thereof, mutants, analogs or derivatives thereof, (b) nucleic acids such as DNA etc., encoding the PEX domain of the MT1-MMP, (c) the antibodies (including monoclonal antibodies) of the present invention, partial fragments thereof or derivatives thereof, and (d) the compounds or salts thereof which suppress and/or inhibit biological activities such as the homodimer formation of MT1-MMP.

The active ingredient of the present invention is anticipated to be useful for suppressing or inhibiting the processing of proMMP-2 induced by the formation of the complex formed of multiple MT1-MMP and, for example, at least the process converting ProMMP-2 to the activated type by the homodimer formation of MT1-MMP. The active ingredient is useful for maintenance or recovery of a function which is affected and/or disappears by excess activation of proMMP-2, and is useful for prevention or treatment of disorders, abnormalities and/or diseases with which the processing of proMMP-2 induced by the formation of the complex formed of multiple MT1-MMP, for example, at least the activation of proMMP-2 by the homodimer formation of MT1-MMP is associated. Also, it is useful for suppressing or inhibiting the processing of proMMP-2 by MT1-MMP. Additionally, it is useful for controlling, for example suppressing the migration, invasion and/or metastasis of MT1-MMP gene-expressing cells, and is anticipated to be useful for the prevention or treatment of disorders, abnormalities and/or diseases induced by the migration, invasion and/or metastasis of the cells in association with the processing of proMMP-2 by MT1-MMP in the expressing cells. Furthermore, the active ingredients including MT1PEX protein and antibodies against PEX of the present invention are useful for controlling, for example, suppressing the migration, invasion and/or metastasis of MT1-MMP gene-expressing cells, and are useful for the prevention or treatment of disorders, abnormalities and/or diseases induced by the increased migration, invasion and/or metastasis of the cells in association with the activation of proMMP-2 by the homodimer formation of the MT1-MMP. In particular, they are useful for blocking and/or suppressing the migration, invasion and/or metastasis of tumor cells, such as cancers, and can be anticipated as anti-tumor agents and/or cancer metastasis suppressors.

### Kits

The present invention further relates to pharmaceutically-acceptable packs (and/or containers or packages) and kits comprising one or more containers filled with one or more of the components of the aforementioned compositions of the invention. Associated with such a single or plural containers can be a notice (attached document) in the form prescribed by a governmental agency regulating the manufacture, use or sale of pharmaceuticals or biological products, reflecting approval by the agency of the manufacture, use or sale of the product for human administration.

According to the present invention, the inventive drug design may be performed utilizing techniques applicable in the art, including for example the following steps:
selecting a site or region selected from the group consisting of those comprising at least 2 continuous amino acid residues selected from constituent amino acid residues for a sequence ranging from amino acid residues for the PEX domain of MT1-MMP, and next
   (i) substituting an isoster for a pharmacophore among them,
   (ii) replacing at least one of constituent amino acid residues with a D-amino acid residue,
   (iii) modifying a side chain of amino acid residues,
   (iv) arranging and linking an amino acid residue different from amino acid residues present in the said sequences, or
   (v) analyzing a stereostructure and designing mimics (e.g., Koichi Shudo (ed.), "Pharmaceutical Research and Development, Vol. 7 (Drug Design)", June 25, 1990, Hirokawa Pub. Co., Tokyo, Japan and documents & articles quoted therein, etc.). Part of such techniques includes those which are disclosed herein above.

For terms (words) and/or abbreviations used in the specification and in the drawings, they must conform with an "IUPAC-IUB Commission on Biochemical Nomenclature" or are based on the meanings of the terms which are commonly used in the art. Abbreviations as listed below are principally used hereinbelow:
bp: base pair(s);
BB-94: [4-(N-hydroxyamino)-2R-isobutyl-3S-(thiophen-2-yl thiomethyl)-succinyl]-L-phenylalanine-N-methylamide or batimastat
TIMP-2: tissue inhibitor of metalloproteinase-2
IPTG: isopropyl-l-thio-β -D-galactopyranoside
PCR: polymerase chain reaction
SDS: sodium dodecyl sulfate

For amino acid sequences in relation with proteins, peptides, etc.:
- A:: alanine (Ala)
- C:: cysteine (Cys)
- D:: aspartic acid (Asp)
- E:: glutamic acid (Glu)
- F:: phenylalanine (Phe)
- G:: glycine (Gly)
- H:: histidine (His)
- I:: isoleucine (Ile)
- K:: lysine (Lys)
- L:: leucine (Leu)

- M:: methionine (Met)
- N:: asparagine (Asn)
- P:: proline (Pro)
- Q:: glutamine (Gln)
- R:: arginine (Arg)
- S:: serine (Ser)
- T:: threonine (Thr)
- V:: valine (Val)
- W:: tryptophan (Trp)
- Y:: tyrosine (Tyr)

### For nucleotide sequences:

- A:: adenine
- C:: cytosine

- G:: guanine
- T:: thymine

### Examples

The present invention is specifically described by means of the following Examples which are provided only for illustrative purposes, and reference to specific embodiments of the present invention. Although these illustrative examples are provided for disclosing particular embodiments of the present invention, they should not be construed as limiting or restricting the scope of the present invention disclosed herein. It should be understood that various modes will be practicable based on the spirit of the present invention.

All the examples were or can be practiced using standard techniques well or conventionally known to those of ordinary skill in the art unless otherwise specified.

Molecular biological techniques generally employed in the following Examples can be carried out as described in a standard experimental manual, for example, J. Sambrook, E.F. Fritsch & T. Maniatis, Molecular Cloning: A Laboratory Manual (2nd ed.), Cold Spring Harbor Laboratory Press, Cold Spring Harbor, New York, 1989. All "parts" or amounts presented in the following Examples are according to weight basis unless defined and referred to in particular.

Size separation of fragments in the following Examples was carried out using a procedure described in the literature described above or in many other reference literature, e.g., Goeddel et al., Nucleic Acids Res., 8: 4057, 1980, namely, standard techniques such as agar and polyacrylamide gel electrophoresis ("PAGE"). Unless otherwise described, ligation was carried out with a standard buffer solution, incubation temperature and time, at an approximate amount of equivalent molar concentration to a DNA fragment to be ligated, using about 10 units of T4 DNA ligase ("ligase") per 0.5 µ g of DNA.

In the following Examples, unless particularly indicated, specific operation and conditions for treatment were according to:

J. Sambrook, E. F. Fritsch & T. Maniatis (ed.), "Molecular Cloning: A Laboratory Manual (2nd ed.)", Cold Spring Harbor Laboratory Press, Cold Spring Harbor, New York, 1989 and D. M. Glover et al. (ed.), "DNA Cloning", 2nd ed., Vol. 1 to 4, (The Practical Approach Series), IRL Press, Oxford University Press, 1995 and the like for DNA cloning;

R. Saiki et al., Science, 230: 1350, 1985; R. Saiki et al., Science, 239: 487, 1988; H. A. Erlich (ed.), PCR Technology, Stockton Press, 1989; D. M. Glover et al. (ed.), "DNA Cloning", 2nd ed., Vol. 1, (The Practical Approach Series), IRL Press, Oxford University Press, 1995; M. A. Innis et al. (ed.), "PCR Protocols: A Guide to Methods and Applications", Academic Press, New York, 1990; M. J. McPherson, P. Quirke and G. R. Taylor (ed.), PCR: A Practical Approach, IRL Press, Oxford, 1991; M. A. Frohman et al., Proc. Natl. Acad. Sci. USA, 85: 8998-9002, 1988 and the like for PCR in particular;

G. B. Fields (ed.), "Methods in Enzymology", Vol. 289 (Solid-Phase Peptide Synthesis), Academic Press, New York (1997) and the like for peptide synthesis. Furthermore, in instances where commercially available agents or kits were employed, protocols attached thereto or reagents and the like attached thereto were used.

Moreover, the following procedures were according to additional literature described below. Namely, these procedures can be carried out in accordance with a method described in:

L. Grossman et al. (ed.), "Methods in Enzymology", Vol. 12 (Part A & Part B: Nucleic Acids), Academic Press, New York, 1968; S. L. Berger et al. (ed.), "Methods in Enzymology", Vol. 152 (Guide to Molecular Cloning Techniques), Academic Press, New York, 1987; p. 33 & p. 215 and the like, for example, for isolation of mRNA;

R. Wu (ed.), "Methods in Enzymology", Vol. 68 (Recombinant DNA), Academic Press, New York, 1980; R. Wu et al. (ed.), "Methods in Enzymology", Vol. 100 (Recombinant DNA, Part B) & Vol. 101 (Recombinant DNA, Part C), Academic Press, New York, 1983; R. Wu et al. (ed.), "Methods in Enzymology", Vol. 153 (Recombinant DNA, Part D) & Vol. 154 (Recombinant DNA, Part E), Academic Press, New York, 1987; R. Wu (ed.), "Methods in Enzymology", Vol. 155 (Recombinant DNA, Part F), Academic Press, New York, 1987; J. H. Miller (ed.), "Methods in Enzymology", Vol. 204, Academic Press, New York, 1991; R. Wu (ed.), "Methods in Enzymology", Vol. 216 (Recombinant DNA, Part G), Vol. 217 (Recombinant DNA, Part H) & Vol. 218 (Recombinant DNA, Part I), Academic Press, New York, 1993; S. Weissman (ed.), "Methods in Enzymology", Vol. 303, Academic Press, New York, 1999; J. C. Glorioso et al. (ed.), "Methods in Enzymology", Vol. 306, Academic Press, New York, 1999; Japanese Biochemical Society (ed.), "Zoku Seikagaku Jikken Koza 1, Idenshi Kenkyu-ho II", Tokyo Kagaku-dojin Publishing Co., Inc., 1986; Japanese Biochemical Society (ed.), "Shin-Seikagaku Jikken Koza 2, Kakusan III (Recombinant DNA technique)", Tokyo Kagaku-dojin Publishing Co., Inc., 1992 and the like, for example, for DNA cloning;

L. Grossman et al. (ed.), "Methods in Enzymology", Vol. 29 (Nucleic Acids and Protein Synthesis, Part E), Academic Press, New York, 1974 and the like, for example, for hybridization. Alternatively, a method described in literature cited in the above literature or a method substantially equivalent to the same or a modified method thereof can be performed (Description in literature is incorporated in the disclosure of the present specification as reference.)

### Experimental materials and reagents used in the following Examples are as described hereinafter.

Dulbecco's modified Eagle's medium (DMEM) was manufactured by Nissui Pharmaceutical Co., Ltd (Tokyo, Japan). FuGENE 6™ was manufactured by Roche Molecular Biochemicals Co., Ltd., Basel, Switzerland. A mouse monoclonal anti-FLAG epitope M2 antibody and an alkaline phosphatase-conjugated goat anti-mouse IgG antibody were manufactured by Sigma Chemical Co., Ltd. (MO, USA). Monoclonal anti-phosphotyrosine antibody PY20 was manufactured by ICN Biochemicals, Inc., (Ohio, USA). Chemical cross linking agent DSG was manufactured by Pierce Chemical Co. (1L, USA). DQ™ gelatin was manufactured by Molecular Probes, Inc., (OR, USA). Matrigel Matrix was manufactured by Becton Dickinson Labware (MA, USA). Peptidyl hydroxamate MMP inhibitor BB-94 was provided by Dr. Peter D. Brown of British Biotech Pharmaceuticals Ltd. (Oxford, UK).

### Example 1

### A

### (1) Construction of PEX-deleted FLAG-tagged MT1-MMP (MT1Cat-F) Catalytic domain-deleted FLAG-tagged MT1-MMP (MT1PEX-F), MT1-F/NGFR, MT1PEX-F/NGFR, MT1Cat-F/NGF and MT4PEX-F/NGFR

A FLAG epitope (Asp-Tyr-Lys-Asp-Asp-Asp-Asp-Lys)-tagged MT1-MMP (MT1-F) expression construct was generated as described in Itoh, Y. et al., J. Biol. Chem. 274: 34260-34266, 1999. This construct was used as a template to generate cDNA fragments for MT1Cat-F (Δ Ile³¹⁸-Gly⁵³⁵) and MT1-PEX-F (Δ Tyr¹¹²-Pro³¹²) by the PCR extension method of Ho et al., (Ho, S. N. et al., Gene, 77: 51-59, 1989). The cDNA was subcloned into pSG5 (Stratagene, CA, USA).

Chimera mutants of the ectodomain of MT1-F, MT1Cat-F MT1PEX-F or MT4PEX-F, with the transmembrane (TM)/cytoplasmic (CP) domain of NGFR were also generated by the PCR method in the same manner, and subcloned into pSG5.

The mutant is derived from sequences corresponding to Met¹ to Asp⁵¹⁵ of MT1-MMP and Glu³⁸⁴ to Gly⁷⁹⁰ of NGFR. The other chimera mutants were also generated at the corresponding sites. All the PCR-generated fragments were confirmed by DNA sequencing.

### (2) Purification and Folding of the Expressed Products

MT1EAΔ TM, MT1-PEX and MT4-PEX expressed in E. coli were purified and folded as follows:

The cDNA fragments encoding Tyr¹¹² through Gly⁵³⁵ of Met plus MT1-MMP with the point mutation of Glu240 to Ala (MT1EAΔ TM), Cys³¹⁹ through Gly⁵³⁵ of Met plus MT1-MMP (MT1PEX), and Cys³³⁶ through Gly⁵⁵⁰ of Met plus mouse MT4-MMP were generated by PCR, and subcloned into a pET3a expression vector. All the PCR-generated DNAs were confirmed by DNA sequencing.

BL21(DE3)pLys S cells were transformed with these pET3a constructs, and protein expression was induced by 0.4 mM IPTG. The expressed protein products were purified and folded according to the method of Huang et al. (Huang, W. et al., FEBS Lett. 384, 155-161, 1996).

### (3) Purification of proMMP-2 and TIMP-2

Recombinant human proMMP-2 and TIMP-2 were expressed in HighFive insect cells (Invitrogen, CA, USA), respectively, infected with recombinant baculo viruses engineered to express human proMMP-2 and TIMP-2. Recombinant viruses were prepared using BAC-TO-BAC™ Baculovirus Expression Systems (Life Technologies, MD, USA).

Purification of proMMP-2 was conducted from the culture supernatant by Gelatin Sepharose (Amersham Pharmacia Biotech, Inc., Uppsala, Sweden) and gel-permeation on S-200 (Amersham Pharmacia Biotech, Inc.) according to the description in Itoh, Y. et al., Biochem. J., 308: 645-651, 1995. Purification of TIMP-2 from the culture supernatant was conducted by Green A Dyematrex (Millipore, Co., MA, USA) and gel-permeation on S-200.

### (4) Gel-Permeation Column Chromatography

MT1EAΔ TM, MT1PEX and MT4PEX were subjected to gel-permeation column chromatography on Superdex 200 or Superdex 75 equilibrated with 50 mM Tris-HCl buffer, pH 7.5 containing 150 mM Nac1, 10 mM cacl₂ and 0.02% NaN₃ and analyzed under the AKTA explorer 10S system (Amersham Pharmacia Biotech, Inc.).

### (5) Western Blotting

Cell lysate samples were subjected to separation on SDS-PAGE, followed by a transfer of the proteins in the gel to nitrocellulose membranes (Hybond-ECL, Amersham Pharmacia Biotech, Inc.). After blocking with a solution of 10% skim milk dissolved in TBS (20 mM Tris-HCl buffer, pH 7.5 containing 150 mM NaCl) the membrane was exposed to a monoclonal anti-FLAG M2 antibody (3 µ g/ml) for detecting FLAG-tagged MT1-MMP and its mutants. In order to visualize the immunoreactive bands, the membrane was further exposed to an alkaline phosphatase-conjugated goat anti-mouse IgG antibody. For the purpose of detecting a phosphotyrosine residue in chimera proteins, a monoclonal anti-phosphotyrosine antibody, PY20 (1 µ g/ml) was used.

### (6) Gelatin Zymography

Gelatin zymography was conducted using an SDS-polyacrylamide gel (PAGE) containing gelatin (0.8 mg/ml) (Itoh, Y. et al., J. Biol. Chem., 273: 24360-24367, 1998). The samples were mixed with a SDS-PAGE loading buffer without a reducing agent and subjected to electrophoretic analysis at room temperature. Enzyme activity was visualized as a negative staining area with Coomassie Brilliant Blue R-250.

### (7) Cell Culture and Transfection

COS1, HT1080 and CHO-K1 cells were maintained in a HAM F-12 medium or DMEM containing 10% fetal bovine serum in a humidified incubator at 37°C. At 16 hrs before the transfection was performed, the cells were seeded in 6-well plates at 1.5 x 10⁵/well.

Expression vectors for each protein were transfected using FuGENE^{6TM} (Roche Molecular Biochemicals) according to the manufacturer's instructions. At 48 hrs after the transfection was performed, the cells were collected for use in the experiments.

### (8) Construction of PEX-deleted MT1-MMP (MT1Cat) Catalytic domain-deleted MT1-MMP (MT1PEX), MT1-MT4PEX, MT1-F/NGFR, MT1PEX-F/NGFR, MT1Cat-F/NGFR and MT4PEX-F/NGFR

The cDNA of MT1-MMP was used as a template to generate DNA fragments for MTlCat (Ile³¹⁸-Gly⁵³⁵) and MT1PEX (Tyr¹¹²-Pro³¹²) by the PCR extension method (Ho et al., Gene, 77, 51-59 (1989)). A FLAG epitope (Asp-Tyr-Lys-Asp-Asp-Asp-Asp-Lys)-tagged MT1-MMP (MT1-F) expression vector was constructed according to the method of Itoh et al., Biochem. J.,. 308: 645-651, 1999 and used as a template to generate cDNA for FLAG-tagged MT1Cat (MT1Cat-F) and MT1PEX (MT1PEX-F). The cDNA was subcloned into pSG5 (Stratagene, CA, USA). For stable expression, the cDNA of MT1PEX-F was subcloned into pCEP4 (Invitrogen, Groningen, Netherlands) that bears EBNA-1 and hygromycin resistance gene expression cassettes.

The DNA corresponding to Cys³¹⁹ to Cys⁵⁰⁸ in MT1-MMP and MT1PEX-F was replaced with Cys³³⁶ to Cys⁵²⁷ of mouse MT4-MMP to generate MT1-MT4PEX and MT4PEX-F, respectively.

A c-Myc epitope (Glu-Gln-Lys-Leu-Ile-Ser-Glu-Glu-Asp-Leu)-tagged MT1-MMP (MT1-Myc) expression vector was generated by PCR in a manner similar to that described for MT1-F in Itoh et al., Biochem. J., 308: 645-651, 1999. A c-Myc tag was inserted between Arg¹¹¹-Tyr which is the same site for insertion of FLAG-tag in MT1-F.

Chimera mutants of the ectodomain of MT1-F, MT1Cat-F MT1PEX-F or MT4PEX-F with the transmembrane/cytoplasmic domain of NGFR (MT1-F/NGFR, MT1Cat-F/NGFR MT1PEX-P/NGFR, or MT4PEX-F/NGFR, respectively) were also generated by PCR and subcloned into pSG5. The mutants are derived from sequences corresponding to Met¹ through Asp⁵¹⁵ of MT1-MMP and Glu³⁸⁴ through Gly⁷⁹⁰ of NGFR. The other chimera mutants were also generated at the corresponding sites. All the PCR-generated fragments were confirmed by DNA sequencing analysis.

The expression vector for the constitutively active form of Rac1 (V17, Rac1DA) was kindly provided by Dr. Yoshimi Takai of Osaka University.

### (9) Cell Culture and Transfection

COS1 and HT1080 cells were maintained in Dulbecco's modified Eagle's medium (DMEM; Sigma Chemical Co., Ltd., MO, USA) containing 10% fetal bovine serum and kanamycin in a humidified 37°C incubator. At 16 hrs before the transfection was performed, the cells were seeded in 6-well plates at 1 x 10⁵/well. Each protein expression plasmid was transfected with FuGENE6™ (Roche Molecular Biochemicals, Basel, Switzerland) according to the manufacturer's instructions.

### (10) Generation of antibodies

An anti-(MT1-MMP catalytic domain) antibody (anti-Cat) was generated via immunization of rabbits with the purified MT1-MMP catalytic domain which was expressed in E. coli. The antibody was confirmed to recognize MT1-MMP specifically as it did not recognize other MT-MMPs including MT2-, MT3-, MT4-, MT5- and MT6-MMPs. A monoclonal mouse anti-(human MT1-MMP PEX domain) antibody (anti-PEX, 222-1D8) was generated via immunization of mice with a purified E. coli-expressed human MT1-MMP PEX domain. The antibody was confirmed to be specific to MT1-MMP.

### (11) Immunoprecipitation

Transfected COS1 cells were lysed in a RIPA buffer (50 mM Tris-HCl, pH 7.5; 150 mM NaCl; 1% Triton-X100; 1% sodium deoxycolate; 0.1% SDS; 1 mM EDTA; 1 mM PMSF; 10µ M E-64; 0.02% NaN₃) at room temperature for 1 hr. Cell lysates were centrifuged at 15,000 rpm in Eppendorf tubes, and the supernatant was reacted with anti-FLAG M2 antibody-conjugated beads (Sigma) at 4°C for 1 hr. The beads were washed three times with a RIPA buffer and once with Tris-buffered saline (pH 7.5) (TBS). Bound proteins were eluted by the FLAG peptide (Asp-Tyr-Lys-Asp-Asp-Asp-Asp-Lys: 200 µ g/ml) (Sigma) in TBS, and subjected to Western blot analysis using a monoclonal mouse anti-FLAG epitope M2 antibody (Sigma), a monoclonal mouse anti-c-Myc antibody (Ab-1) (Oncogene, MA, USA; 1 µ g/ml), a rabbit anti-(human MT1Cat antibody (1:2000), or a monoclonal mouse anti-human MT1PEX antibody (222-1D8) (0.5 p. g/ml).

### (12) Western blotting

Cell lysates were subjected to SDS/PAGE, followed by a transfer of the proteins to nitrocellulose membranes (Hybond-ECL, Amersham Pharmacia Biotech, Inc.).
MT1-MMP, MT1Cat MT1-MT4PEX and MT1-F/NGFR were detected with a polyclonal anti-CAT antibody (1:2000), while MT1-MMP, MT1PEX and MT1-F/NGFR with an anti-PEX antibody (222-1D8, 0.5µ g/ml). FLAG-tagged MT1-MMP and its mutants were detected with an anti-FLAG M2 antibody (3µ g/ml). Chimera proteins containing a phosphotyrosine residue were detected with a monoclonal mouse anti-phosphotyrosine antibody, PY20 (ICN Biochemicals, Inc., OH, USA; 1µ g/ml). Immunoreactive bands were visualized with an alkaline phosphatase-conjugated goat anti-mouse IgG or anti-rabbit IgG (Sigma).

### (13) Surface Biotinylation and Immunoprecipitation

Transfected COS1 cells were washed three times with chilled PBS containing 1 mM MgCI₂ and 0.1 mM CaCl₂. Cells were then incubated with 2 mg/ml sulfo-NHS-biotin (Pierce) in the same buffer at 4°C for 30 min. The reaction was terminated by further incubating the cells with 25 mM lysine in PBS.
The cells were lysed in a RIPA buffer, and the biotinylated proteins were precipitated with streptavidin-Sepharose beads (Amersham-Pharmacia). The samples were analyzed by Western blotting using an anti-MT1Cat or anti-PEX antibody.

### (14) In Situ Gelatin Degrading Assay

Two different in situ gelatin degrading assays were performed. One used DQ™ gelatin (Molecular Probes, Inc., OR, USA). DQ gelatin is a fluorescence-quenched gelatin substrate, and will fluoresce upon proteolytic cleavage. Glass cover slips were coated with 5% DQ™ gelatin in a 10 mg/ml unlabeled gelatin solution, and dried. Transfected cells were cultured on the cover slip in a humidified chamber for 16 hrs. The cells were then fixed with 3% para-formaldehyde and the degraded areas were visualized as those showing fluorescence.

The other assay used Alexa488-conjugated gelatin made with an Alexa488-labeling kit (Molecular Probes). The bottom surfaces of 4-well chamber slides (Nunc) were coated with Alexa488-conjugated gelatin according to the method of Yana, I. and Weiss, S. J., Mol. Biol. Cell, 11, 2387-2401, 2000. Transfected COS1 cells were cultured in the chamber slides for 16 hrs in a humidified culture incubator. The cells were then fixed with 3% para-formaldehyde in PBS, and the degraded area was analyzed under a fluorescence microscope. The degraded area was visualized as a dark, non-fluorescent area. Fluorescence images were analyzed using confocal microscopy (BioRad).

The activities of MT1-MMPs detected by the methods described above are confirmed to be inhibited by the peptidyl hydroxamate MMP inhibitor, BB94. BB94 was kindly provided by Dr. Peter D. Brown of British Biotech Pharmaceuticals Ltd. (Oxford, UK).

### (15) Indirect Immunofluorescence Staining

Transfected cos1 cells or HT1080 cells were re-seeded on gelatin-coated cover slips and cultured for 24 hrs.
The cells were then fixed with 3% para-formaldehyde in PBS containing 75 mM lysine. After blocking with 5% goat serum and 3% bovine serum albumin in TBS for 1 hr at room temperature, the cells were reacted with an anti-FLAG M1 antibody (5µ g/ml), PY20 (2µ g/ml), or anti-Cat antiserum (1:1,000) at room temperature for 2 hrs. To detect the phosphotyrosine signal, the cells were permeabilized with 0.1% Triton-X100 in TBS after fixation. 1 mM CaCl₂ was included throughout the procedure of washing and incubation for staining with the anti-FLAG M1 antibody. Cy3- or Alexa488-conjugated goat anti-mouse IgG was used to visualize the antigen signal. To visualize F-actin, the cells were further incubated with Alexa488- or Alexa594-conjugated phalloidin (Molecular Probes). The signals were analyzed by confocal microscopy (BioRad).

### (16) ProMMP-2 Binding Assay

Transfected COS1 cells were cultured in a 24-well plate (0.2 x 10⁴/well). Forty-eight hours after transfection, the cells were washed three times with serum-free DMEM, and incubated with purified proMMP-2 (2µ g/ml) in the serum-free DMEM at 25°C for 1 hr. The cells were then washed three times with serum-free DMEM containing 5% dimethyl sulfoxide (DMSO) to remove free proMMP-2, and lysed in 50 µ 1 of a non-reducing SDS-loading buffer. MMP-2 bound to the cells was analyzed by gelatin zymography.

### (17) Matrigel Invasion Assay

Matrigel Invasion Chambers were prepared according to the manufacturer's instructions (Becton Dickinson Labware, MA, USA) by coating, with 10µ g of Matrigel, the 8µ m-pore sized membrane of the culture inserts for 24-well plates (Falcon). A 0.5 ml aliquot of transfected HT1080 cell suspension at 1 x 10⁵/ml was seeded on the upper chamber and incubated at 37°C in a humidified chamber. Hepatocyte growth factor (Toyobo; 10 ng/ml) was added to the lower chamber as a chemo-attractant. After 14 hrs of incubation, cells remaining on the upper surface of the membrane were removed, and cells on the lower surface were fixed with 3% para-formaldehyde in PBS. Green fluorescent protein (GFP)-positive cells that had invaded to the lower surface were counted using fluorescence microscopy (x40).

### B

### The results are summarized as follows:

### (1) Elucidation of Functions for the Activation of ProMMP-2 by MT1-MMP on the Cell Surface through the Hemopexin-like (PEX) Domain

In order to reveal the importance of the PEX domain of MT1-MMP in the activation of proMMP-2 by an experiment, a plasmid was constructed which was capable of expressing PEX-deleted FLAG-tagged MT1-MMP (MT1Cat-F (Fig. 1). In Fig. 1, each symbol indicates the following:
Pro: propeptide;
FLAG: FLAG epitope;
CD: catalytic domain;
H: hinge;
PEX: hemopexin-like domain;
TM: transmembrane domain.

The expression construct for FLAG epitope (Asp-Tyr-Lys-Asp-Asp-Asp-Asp-Lys)-tagged MT1-MMP (MT1-F) described in Itoh, Y. et al., J. Biol. Chem., 274: 34260-34266, 1999 was used as a template to generate cDNA for MT1Cat- (Δ Ile³¹⁸-Gly⁵³⁵) by the PCR extension method of Ho et al., (Ho, S. N. et al., Gene, 77: 51-59, 1989). The cDNA was subcloned into pSG5 (Stratagene, CA, USA). The PCR-generated fragments were confirmed by DNA sequencing. COS1 cells were transfected with the expression vector for MT1-MMP mutant; MT1-F or MT1Cat-F or a vector alone (Mock). Expression vectors for each protein were transfected using FuGENE6™ (Roche Molecular Biochemicals) according to the manufacturer's instructions. COS1 cells were seeded in 6-well plates at 1.5 x 10⁵/well at 16 hrs before the transfection was performed. The COS1 cells were maintained in a HAM F-12 medium or DMEM containing 10% fetal bovine serum in a humidified incubator at 37°C. At 48 hrs after the transfection, the cells were collected for use in the experiments. The cells were incubated in the presence of purified proMMP-2 in a serum-free medium (0.25 µ g/ml) at 37°C for 18 hrs. Cell lysates were tested for the expression of the mutants, while culture supernatants were tested for the activation of proMMP-2.

The COS1 cells transfected with FLAG-tagged full length MT1-MMP activated exogenous proMMP-2 which was added to the culture supernatant while the cells transfected with MT1Cat-F did not activate at all (Fig. 2). The upper panel in Fig. 2 shows Western blotting analysis using a monoclonal anti-FLAG M2 antibody for cell lysates obtained from the transfected cells. The lower panel in Fig. 2 shows gelatin zymography of the culture medium.

MT1Cat-F is expressed on the cell surface, and has similar gelatin degrading activity as compared to MT1-F (see, Fig. 13, lower panel). Therefore, it is obvious that differences in the activation of proMMP-2 results from either its appearance manner on the cell surface or its proteolytic degradation activity. PEX is thought to decide presumably the arrangement of MT1-MMP on the cell surface through complex formation.

### (2) To Test Whether the Function of MT1-MMP Ectodomain is Present or not

In order to test if the ectodomain of MT1-MMP is involved in the complex formation, a catalytically-inactive soluble mutant (MT1EAΔ TM) wherein a propeptide was deleted was expressed in E. coli (Fig. 3). The cDNA fragments encoding Tyr¹¹² through Gly⁵³⁵ of Met plus MT1-MMP with the point mutation of Glu240 to Ala (MT1EAΔ TM) was prepared by PCR, and subcloned into a pET3a expression vector. The resulting PCR-generated DNA was confirmed by DNA sequencing. E. coli BL21(DE3)pLys S cells were transformed with the pET3a construct thus prepared, and protein expression was induced by 0.4 mM IPTG. The expressed protein product was purified and folded according to the method of Huang et al. (Huang, W. et al., FEBS Lett. 384, 155-161, 1996). This purified protein appeared to be more than 98% pure, as shown in Fig. 4.

Although the catalytically active site was mutated so as to avoid auto-degradation, this mutation should not create an overall structural alteration, as has been shown in other MMPs (Atkinson, S. J. et al., J. Biol. Chem., 270: 30479-30485, 1995). Furthermore, MT1EA Δ TM was subjected to Superdex 200 gel filtration column chromatography at three different concentrations. The inset in Fig. 4 shows the SDS-PAGE analyses of MT1EAΔ TM under reducing and non-reducing conditions. Bands were visualized with Coomassie Blue R-250.

MT1EAΔ TM demonstrated a different mobility under reducing and non-reducing SDS-PAGE, suggesting that the S-S bond was correctly formed. In addition, it was confirmed that MT1EAΔ TM had an ability to form a complex with TIMP-2, suggesting that this catalytic domain was correctly folded. For the purification step, it was noted that MT1EAΔ TM did not elute from the gel permeation column where expected for its size of 53 kDa, but rather at a volume suggesting a larger molecular mass (600 kDa or greater, and 100 kDa).

Accordingly, it was suggested that MT1-MMP formed a homophilic complex. This complex formation (in particular, the formation of a complex having a high molecular mass) is dependent on the concentration of MT1EAΔ TM (Fig. 4). At a concentration of 0.33µ M, a monomer peak at 50 kDa and a peak above 600kDa were observed. When the sample was added at 0.66µ M or Y.4µ M, the height of a dimer peak around 130 kDa increased, while the height of a peak corresponding to a higher molecular mass further increased (Fig. 4).
The appearance of a dimer, a tetramer, and additional polymers was also revealed for MT1EAΔ TM treated with a chemical crosslinking agent DSG (Fig. 5). Crosslinking of MT1EAΔ TM was conducted as follows:

Purified MT1EAΔ TM derived from E. coli was treated with 0.2 mM DSG in 50 mM HEPES containing 150 mM NaCl, 10 mM CaCl₂, 0.02% NaN₃ at 0°C. The reaction was terminated by adding a Tris-HCl buffer, pH 7.5 until its final concentration reached 10 mM.

### (3) Test for a Domain Involved in the Formation of a Complex

To determine whether either of the catalytic domain and PEX was involved in the complex formation, MT1EAΔ TM was treated with trypsin. Since trypsin is found to cleave the middle of the MT1EAΔ TM hinge region that contains several Arg and Lys, it is capable of separating the catalytic domain and PEX. The sample was then analyzed on gel permeation column chromatography with Superdex 75. Thus, MT1EAΔ TM (50 µ g/ml) was treated with trypsin (0.2 µ g/ml) at 37°C for 1 hr to cleave between the catalytic domain and PEX. Trypsin was deactivated by adding 2 mM PMSF to the mixture. Next, the sample was applied to a Superdex 75 gel filtration column. As shown in Fig. 6, the catalytic domain was eluted at a volume corresponding to around 17 kDa, but PEX was eluted at a volume corresponding to around 40 kDa. Since the catalytic domain and PEX migrate in SDS-PAGE under non-reducing conditions to 21 kDa and 24 kDa, respectively (Fig. 6), it is suggested that PEX but not the catalytic domain forms a homophilic complex in solution.

Moreover, to test if the complex formation of PEX is specific to MT1-MMP, Cys³¹⁹ through Gly⁵³⁵ of Met plus MT1-MMP (MT1PEX) and the PEX of MT4-MMP were expressed in E. coli. The cDNA fragments encoding MT1PEX, and Cys³³⁶ through Gly⁵⁵⁰ of Met plus mouse MT4-MMP (GenBank™ accession number AB021224) were prepared by PCR, and subcloned into a pET3a expression vector. The PCR-generated DNA was confirmed by DNA sequencing. Thereafter, E. coli BL21(DE3)pLys S cells were transformed with the pET3a constructs, and protein expression was induced by 0.4 mM IPTG. The expressed protein products were purified and folded according to the method of Huang et al. (Huang, W. et al., FEBS Lett. 384, 155-161, 1996).

As shown in Fig. 7, the apparent molecular mass of the PEX of MT4-MMP (MT4-PEX) is almost identical to that of the PEX of MT1-MMP (MT1-PEX) on SDS-PAGE under reducing and non-reducing conditions (Fig. 6). However, on gel-permeation chromatography, the PEX of MT4-MMP (MT4-PEX) eluted at approximately 24 kDa whereas the PEX of MT1-MMP (MT1-PEX) eluted at approximately 40 kDa, suggesting that dimer complex formation was specific for MT1-PEX. These results indicate that the ectodomain of MT1-MMP forms a homophilic complex through the interaction of its PEX at least in a solution.

In other words, the ectodomain of MT1-MMP forms a homophilic complex through the interaction of PEX thereof in vitro.

### (4) Test for a Site Where a Homophilic Complex is Formed

Next, it is tested whether MT1-MMP expressed in animal cells also forms a homophilic complex or not. For the test, the trk protooncogene (nerve growth factor receptor, NGFR) is utilized in order to monitor dimer formation on the cell surface.

The principle of assay is as follows:
NGFR forms a dimer through binding to its ligand. Subsequently, tyrosine residues in the cytoplasmic domain are trans-phosphorylated by its tyrosine kinase (Jing, S. et al., Neuron, 9: 1067-1079, 1992; Lemmon, M. A. et al., Trends Biochem. Sci., 19: 459-463, 1994). Thus, it was demonstrated that the fusion protein between (a) the cytoplasmic domain of NGFR and (b) BCR of the oncoprotein BCR-ABL induced constitutive phosphorylation of NGFR's tyrosine residues when BCR formed a tetramer (Maru-, Y. et al., Oncogene, 16: 2585-2595, 1998).

By utilizing the above principle, a chimeric protein is first constructed consisting of the ectodomain of MT1-MMP and the transmembrane (TM) and cytoplasmic (CP) domains of NGFR (Fig. 8) in order to test for direct complex formation. When the ectodomain of this chimeric protein forms a homophilic complex in a similar manner, tyrosine residues in the cytoplasmic domain will be phosphorylated.

Accordingly, to test for domain specific interactions of MT1-MMP, the following chimeras were constructed (Fig. 8):
MT1-F/NGFR; MT1PEX-F/NGFR; MT1Cat-F/NGFR MT4PEX-F/NGFR

In Fig. 8, each of the symbols other than those described above is as follows:
NGFR-TM/CP, transmembrane and cytoplasmic domains derived from NGFR;
TK, tyrosine kinase domain;
Y, tyrosine residues phosphorylated.

A FLAG-tagged MT1-MMP (MT1-F) expression construct as used herein was as described in Itoh, Y. et al., J. Biol. Chem. 274: 34260-34266, 1999. In addition, MT1-F was used as a template to generate cDNAs for MT1Cat-F(Δ Ile³¹⁸-Gly⁵³⁵) and MT1PEX-F (Δ Tyr¹¹²-Pro³¹²) by the PCR extension method of Ho et al. (Ho, S. N. et al., Gene, 77: 51-59, 1989). The cDNA was subcloned into pSG5 (Stratagene, CA, USA). Chimera mutants of the ectodomain of MT1-F, MT1Cat-F, MT1PEX-F or MT4PEX-F, with the transmembrane (TM)/cytoplasmic (CP) domain of NGFR were also generated by the PCR method in a similar manner, and subcloned into pSG5. The MT1-MMP-derived sequence corresponded to Met¹ through Asp⁵¹⁵ of MT1-MMP, while the NGFR-derived sequence corresponded to Glu³⁸⁴ through Gly⁷⁹⁰ of NGFR (GenBank™ accession number M23102). The other chimera mutants were also composed from these corresponding sequences. All the PCR-generated fragments were confirmed by DNA sequencing.

Expression vectors for the protein were transfected into COS1 cells, using FuGENE6™ (Roche Molecular Biochemicals) according to the manufacturer's instructions. COS1 cells were seeded in 6-well plates at 1.5 x 10⁵/well at 16 hrs before the transfection was performed. The COS1 cells were maintained in a HAM F-12 medium or DMEM containing 10% fetal bovine serum in a humidified incubator at 37°C. At 48 hrs after the transfection, the cells were collected for use in the experiments.

As shown in Fig. 9, these proteins expressed via the transfection of these constructs were recognized by an anti-FLAG M2 antibody (upper panel, Anti-FLAG). When identical samples were analyzed with an anti-phosphotyrosine antibody (Anti-PY), tyrosine residues of MT1-F/NGFR were phosphorylated (lower panel, Anti-PY, lane 1), suggesting that MT1-MMP expressed in animal cells also formed at least a dimer. Additionally, MT1PEX-F/NGFR was phosphorylated (lane 2) as well.

In contrast, sufficient phosphorylation was not observed for cells transfected with MT1Cat-F/NGF or MT4PEX-F/NGFR (lanes 3 and 4).

These results strongly support the complex formation through PEX as found in vitro. In other words, it is clear that MT1-MMP forms a homophilic complex on the cell surface.

### (5) Inhibition of Dimer Formation by Co-expression with MT1PEX-F

It is investigated whether co-expression of MT1PEX-F and MT1-F competes in the dimer formation of MT1-F/NGFR or not. For the test, the expression plasmids for MT1-F/NGFR and MT1PEX-F were co-transfected into COS1 cells. As shown in Fig. 10, phosphorylation of MT1-F/NGFR (lane 1) was inhibited depending on the expression level of MT1PEX-F (lanes 2-5).

### (6) Elucidation of the Significance of MT1-MMP Complex Formation on the Cell Surface

To investigate roles of dimer formation upon the activation of proMMP-2, MT1-F was introduced into COS1 cells together with MT1PEX-F. The co-introduced cells were reacted with exogenously added proMMP-2. Thus, COS1 cells were co-transfected with the MT1-F and MT1PEX-F at indicated DNA amount ratios. Next, purified proMMP-2 (0.25 µ g/ml) in a serum-free medium was reacted with the resulting cells at 37°C for 18 hrs. MMP-2 in the culture supernatant was analyzed by gelatin zymography, and the cell lysates were subjected to Western blotting analysis using a monoclonal anti-FLAG M2 antibody.

As shown in Figs. 11 and 28, the expression of MT1PEX-F inhibited the activation of proMMP-2 by MT1-F, in dependence upon the expression level. This observation suggests that the dimer formation is necessary for the activation of proMMP-2 on the cell surface, and that MT1PEX-F can inhibit the function of MT1-MMP in a dominant negative manner.

Next, to determine if MT1PEX-F affects the catalytic activity of MT1-MMP on the cell surface or not, tests for gelatin degrading activity and binding capacity to TIMP-2 were conducted using cells transfected in the same incubator. As shown in Figs. 12 through 14, CHO-K1 cells were transfected with an expression vector for MT1-F, MT1-F/MT1PEX-F or MT1Cat-F or a vector alone (Mock). The cells were then treated with trypsin, and separately seeded to: DQ™ gelatin-coated cover slips for testing the proteolytic activity (Fig. 13); and 24-well plates for TIMP-2 binding assay (Fig. 12) and for the activation of proMMP-2 (Fig. 14). Transfected cells on the DQ™ gelatin-coated cover slip as shown in Fig. 13 were incubated in a medium at 37°C for 18 hrs, and the generated fluorescence was analyzed using confocal microscopy. Transfected cells on the 24-well plate as shown in Fig. 12 were incubated with 25 nM ¹²⁵I-labeled TIMP-2 in the presence or absence of BB-94 (50 µ M) at 0°C for 1 hr. MT1-MMP specific binding was calculated by subtracting the datum obtained in the presence of BB-94 (50 µ M) from each datum derived from the corresponding experiments.
Identically transfected cells on the 24-well plate as shown in Fig. 14 were subjected to the reaction with purified proMMP-2 in a serum-free medium at 37°C for 18 hrs. The cells and culture supernatants were respectively subjected to Western blotting analysis using a monoclonal anti-FLAG M2 antibody, and gelatin zymography analysis. All results were derived from the identical transfection experiments. DQ™ gelatin is a fluorescence-quenched gelatin, which fluoresces upon proteolytic cleavage. This characteristic was utilized for the test.

As shown in Fig. 13, all of the cells transfected with MT1-F, MT1-F/MT1PEX-F, or MT1Cat-F exhibited the gelatin degrading activity, and fluoresced, suggesting that all of them retain proteolytic activity. Although the co-transfection of MTIPEX-F led to a 37% decrease in TIMP-2 binding on the cell surface as compared with MT1-F alone (Fig. 12), it could not be explained why these cells completely inhibited the activation of proMMP-2 (Fig. 14). MT1Cat-F-expressing cells exhibited an equivalent TIMP-2 binding capacity as compared to the cells bearing MT1-F (Fig. 12).

These results indicate that the dimer formation through the PEX of MT1-MMP is of importance in the activation of proMMP-2 on the cell surface.

To test if MT1PEX-F also functions in biological systems, Matrigel invasion by MT1-MMP-expressing HT1080 cells was examined.

Cells were transiently transfected with a vector for MT1PEX-F, together with an expression vector for green fluorescent protein (GFP), and GFP-positive invading cells were counted under fluorescence microscopy. HT1080 cells were co-transfected with expression vectors for MT1PEX-F and GFP, or with an expression vector for GFP alone. The cells were assayed for Matrigel invasion as described below. The modified Boiden-chamber method was carried out.

Matrigel Invasion Chambers were coated with 10 µ g of Matrigel per chamber according to the manufacturer's instructions (Becton Dickinson), and were placed on the 8 µ m pore-sized membrane of culture inserts (FALCON). A 0.5 ml aliquot of HT1080 cell suspension at 1 x 10⁵/ml was seeded on the upper chamber, and incubated at 37°C in a humidified chamber for 14 hrs. HGF (5 ng/ml) was added to the lower chamber as a chemo-attractant.

TIMP-2 at 0.5 µ g/ml was added to the upper and lower chambers. GFP-positive cells on the surface of the lower chamber were counted using fluorescence microscopy. The number of GFP-positive cells among mock-transfected cells was 1.31 x 10⁴/1 x 10⁵ cells. The number of GFP-positive cells among MT1PEX-F-transfected cells was 1.12 x 10⁴/1 x 10⁵ cells.

As shown in Fig. 15, exogenously added TIMP-2 inhibited by 50% the invasion by mock-transfected cells. In contrast, by transfection with the expression plasmid for MT1PEX-F, the invasion was suppressed by 70%. The addition of TIMP-2 to the MT1PEX-F-expressing cells produced no further reduction in the invasion. These results indicate that MT1PEX-F can inhibit not only the transiently expressed MT1-MMP activity but also the endogenous MT1-MMP activity. Accordingly, the expression of PEX in HT1080 cells significantly reduced Matrigel invasion activity.

In other words, these findings indicate the action mechanism of MT1-MMP to activate proMMP-2 on the cell surface, and also reveal that this mechanism finally promotes cell invasiveness in tissues. Thus, it is suggested that dimer formation of MT1-MMP is essential for the activation of proMMP-2, leading to the promotion of tumor cell invasion.

### (7) Homophilic Complex Formation of MT1-MMP

The activation of proMMP-2 by MT1-MMP on the cell surface requires the ternary complex formation of proMMP-2, TIMP-2 and MT1-MMP, and also the presence of TIMP-2-free MT1-MMP near the ternary complex. If TIMP-2-free MT1-MMP and the ternary complex are far apart, the activation should not occur. We thus hypothesized that MT1-MMP would form a homophilic complex on the cell surface to arrange the effective activation complex for MMP-2. To test this possibility, FLAG-tagged MT1-MMP (MT1-F) and c-Myc-tagged MT1-MMP (MT1-Myc) (Fig. 16) were co-expressed in COS1 cells, and MT1-F was immunoprecipitated with anti-FLAG M2 antibody-conjugated beads. As shown in Fig. 17, MT1-Myc was co-immunoprecipitated with MT1-F when co-expressed with MT1-F (lane 6, Anti-FLAG and Anti-Myc), whereas MT1-Myc alone did not lead to immunoprecipitation (lane 7, Anti-Myc). This indicates that the MT1-MMP molecules having different tags formed a complex with each other. Next, to test the domain requirement for the complex formation, MT1-F was co-expressed either (i) with MT1-MMP lacking the PEX domain (MT1Cat) or (ii) with MT1-MMP lacking the catalytic domain (MT1PEX) (Fig. 16), and subjected to immunoprecipitation with anti-FLAG beads. The samples were then subjected to Western blotting using anti-FLAG M2, anti-MT1PEX, and anti-MT1Ca to examine the species co-immunoprecipitated with MT1-F. As shown in Fig. 18, MT1PEX but not MT1Cat was co-immunoprecipitated with MT1-F (Anti-PEX, lane 9 and Anti-Cat, lane 8, respectively). This indicates that MT1-MMP forms a homophilic complex through the PEX domain.

### (8) Necessity of PEX in the Activation of ProMMP-2 by MT1-MMP on the Cell Surface

To confirm the importance of the MT1-MMP PEX domain in the activation of proMMP-2, the MT1-MMP PEX domain was exchanged with the one derived from MT4-MMP (Fig. 19, MT1-MT4PEX). As shown in Fig. 20, MT1-MT4PEX-expressing COS1 cells did not activate proMMP-2 (upper panel) although the expression level was similar to that of the wild type enzyme (second panel, Anti-Cat). MT1-MT4PEX is expressed on the cell surface, as the molecule was sensitive to surface biotinylation (lower panel, Surface Biotinylation). MT1-MT4PEX can form a ternary complex of MT1-MT4PEX/TIMP-2/proMMP-2 on the cell surface as the proMMP-2 bound to MT1-MT4PEX-expressing cells (Fig. 21, lanes 2 and 3). To examine if MT1-MT4PEX is proteolytically active on the cell surface, the transfected cells were cultured on DQ™ gelatin substrate. DQ™ gelatin is a fluorescence-quenched substrate that fluoresces upon proteolytic cleavage. As shown in Fig. 22, MT1-MMP- as well as MT1-MT4PEX-expressing cells showed proteolytic activity, whereas mock-transfected cells (Mock) did not. This activity was also completely inhibited by BB94. It has been confirmed that the PEX domain-deleted MT1-MMP (MT1Cat) cannot activate proMMP-2, although it retains its proteolytic activity as well as its ability to form the ternary complex of MT1Cat/TIMP-2/proMMP-2 on the cell surface, as does MT1-MT4PEX. Taken together, these data show that the dimer formation of MT1-MMP through the PEX domain on the cell surface is required to activate proMMP-2.

### (9) MT1-MMP Forms a Homophilic Complex on the Cell Surface

To monitor the dimer formation of the enzyme in the cells, another approach to detect the molecular interactions was executed. Growth factor receptors, including nerve growth factor receptor (NGFR), form a dimer or oligomer complex through the ectodomain to bind to their specific ligands. Subsequently, the cytoplasmic domains containing tyrosine kinase (TK) are brought into close proximity. It is thus possible to determine the specific homodimer interactions of a protein by making a chimeric protein with a receptor molecule. For example, the fusion protein between the cytoplasmic domain of NGFR and BCR of the oncoprotein BCR-ABL which forms a tetramer exhibited constitutive phosphorylation of tyrosine residues. Accordingly, a chimeric protein consisting of the ectodomain of MT1-MMP and the transmembrane and cytoplasmic domains of NGFR was constructed (Fig. 8, MT1-F/NGFR).

To test for domain-specific interactions in this system, the following chimeras were constructed:
MT1PEX-F/NGFR; MT1Cat-F/NGFR; and MT4PEX-F/NGFR (Fig. 8).

These constructs were expressed in COS1 cells at levels similar to MT1-F/NGFR expression as detected by an anti-FLAG M2 antibody (Fig. 9, upper panel, Anti-FLAG, lanes 2-4). When tyrosine phosphorylation in the identical samples was detected by PY, only MT1PEX-F/NGFR was shown to be strongly phosphorylated (lower panel, Anti-PY, lane 2). Phosphorylation levels of MT1Cat-F/NGF and MT4PEX-F/NGFR were extremely low (lanes 3 and 4). These results indicate that a homophilic complex was formed through PEX on the cell surface, supporting the foregoing data.

Next, the expression plasmids for MT1-F/NGFR and FLAG-tagged MT1PEX (MT1PEX-F), MT1Cat (MT1Cat-F) or MT1-F were co-transfected to investigate if the dimer formation of the chimera could be disrupted or not. As shown in Fig. 23, co-expression of MT1PEX-F and MT1-F effectively competed in the dimer formation of MT1-F/NGFR (lanes 2 and 4).

In contrast, the expression of MT1Cat-F did not decrease the intensity of the band (lane 3). Furthermore, 90 % or more inhibition of the phosphorylation was observed by increasing levels of MT1PEX-F expression (Fig. 10, lanes 1-5). This further suggests that MT1-MMP molecules interact through the PEX domains.

### (10) Dimer Formation of MT1-MMP Increases ProMMP-2 Activation

Using the chimera construct, it was investigated whether there are any regulatory factors that might regulate the dimer formation of MT1-MMP on the cell surface. Since it has been reported that MT1-MMP is localized at the lamellipodia structure in walking osteoclasts, it has been examined for the efficacy of a constitutively active form of the small GTPase Rac1 (V12Rac1, Rac1DA on the dimer formation as a GTP-bound form of Rac1 stimulates the generation of lamellipodia in cells. MT1-F/NGFR was co-expressed with Rac1DA in COS1 cells and the phosphotyrosine signal was monitored. As shown in Fig. 24, co-transfection of Rac1DA and MT1-F/NGFR resulted in an enhanced phosphotyrosine signal as compared to MT1-F/NGFR alone (Anti-PY, lanes 1 and 3). When MT1PEX-F was co-expressed, the signal decreased by 43%, suggesting that the increased phosphotyrosine signal was the result of enhanced dimer formation, but was not likely to be due to other tyrosine kinases which might be present downstream of the Rac1 signal, as MT1PEX-F inhibits dimer formation (Anti-PY, lanes 2 and 4, see also Figs. 9, 10 and 23). Immunostaining of MT1-F/NGFR-expressing cells for phosphotyrosine revealed that distinct signals were detected in the cells, especially where the F-actin was concentrated (Fig. 25, MT1-F/NGFR). Expression of Rac1DA generated ruffled membranes (Fig. 25, MT1-F/NGFR-Rac1DA and Rac1DA). Surprisingly, phosphotyrosine signals localized extensively at the edge of the ruffled membrane structure (MT1-F/NGFR-Rac1DA). These signals were confirmed to be generated by MT1-F/NGFR as they were co-localized with the staining pattern with the anti-FLAG M1 antibody, and no such signals were detected in Rac1DA- or control-transfected cells (Rac1DA and Mock). These results suggest that accelerated dimer formation occurs at the ruffled membrane edge. Then, the effect of Rac1DA expression on the proMMP-2 activation by MT1-MMP was investigated. As shown in Fig. 26, co-expression of MT1-MMP and Rac1DA clearly enhanced the processing of proMMP-2 to provide its active form. Immunolocalization of MT1-F in the transfected cells shows that MT1-F is distributed on the cell surface and is relatively concentrated at the lamellipodium structure where F-actin is concentrated (Fig. 27, Anti-FLAG, MT1-F). In contrast, co-expression of Rac1 with MT1-F reinforced the localization of MT1-F at the ruffled membrane edge (Anti-FLAG, MT1-P/Rac1DA). These data suggest that the expression of Rac1DA increases MT1-MMP dimer formation, and the activation of proMMP-2 by generating membrane ruffles that result in an increased concentration of MT1-MMP at the site of the plasma membrane.

### (11) Complex Formation of MT1-MMP on the Cell Surface is Important for MT1-MMP's Biological Activity

Next; the efficacy of MT1PEX-F on the activation of proMMP-2 was examined, since MT1PEX-F effectively inhibited the dimer formation of MT1-F/NGFR. Cost cells were transfected with the plasmids for MT1-MMP and MT1PEX-F in different ratios.

As shown in Fig. 29, proMMP-2 binding to the cell surface was also not affected by MT1PEX-F expression. Co-expression of MT1PEX-F did not decrease the amount of proMMP-2 bound to the cell surface, and no active form was detected. MT1PEX-F also did not affect the proteolytic activity of MT1-MMP on the cell surface as shown in Fig. 30, showing a similar gelatin degradation pattern and area. Therefore, MT1PEX-F inhibits the dimer formation of MT1-MMP and acts as a dominant negative form of MT1-MMP in the activation of proMMP-2 on the cell surface.

HT1080 cells are known to express MT1-MMP and MMP-2 endogenously. Thus, the efficacy of MT1PEX-F expression on the activation of endogenous proMMF-2 in HT1080 cells was next examined. The cells were transfected with a plasmid for MT1PEX-F or an empty vector bearing the hygromycin-resistant gene. The culture medium from the hygromycin-resistant cells was analyzed for produced MMP-2 by zymography. As shown in Fig. 31, control cells activate endogenous MMP-2 in a manner sensitive to BB94 (upper panel, lanes 1 and 2) while the MT1PEX-F-expressing cells did not effectively activate proMMP-2 (upper panel, lane 3). Western blotting analysis of the cell lysates indicated that HT1080 cells spontaneously produced MT1-MMP, and MT1-MMP was partially processed to a 43 kDa fragment as reported previously. MT1PEX-F did not affect the expression and processing of endogenous MT1-MMP. Co-expression of endogenous MT1-MMP and MT1PEX-P clearly indicated the co-localization of both molecules at the edge of the cells (Fig. 32).

In accordance with the present invention, it was reveled that MT1-MMP forms a homodimer through MT1-PEX, and that the complex formation is essential for the activation of progelatinase A (proMMP-2) on the cell surface. Similarly to the requirement of TIMP-2 for the activation of proMMP-2, it is clear that cell surface MT1-MMP requires the dimer formation as demonstrated in the present invention. TIMP-2 which is bound to MT1-MMP is necessary to concentrate proMMP-2 on the cell surface where the activation occurs. For activating proMMP-2 bound to TIMP-2/MT1-MMP complex, MT1-MMP which is not inhibited by TIMP-2 should be located close thereto.

As demonstrated in the present invention, MT1-MMP without PEX (MT1Cat-F) can no longer activate proMMP-2. Since Mt1Cat-F-expressin cells showed gelatinase activity and TIMP-2 binding capacity on their cell surface, it is clear that the said MT1Cat- defect of not being able to activate proMMP-2 is not due to the lack of proteolytic activity and of binding capacity to TIMP-2. Therefore, the activation is believed to result from random distribution of MT1Cat-F) on the cell surface.

Accordingly, it is conceivable that dimer formation of MT1-MMP through PEX on the cell surface allows a closer molecular arrangement between two enzyme molecules and that one of the MT1-MMP molecules in the dimer complex binds to TIMP-2 to act as a proMMP-2 receptor while the other MT1-MMP molecule cleaves the propeptide of proMMP-2 bound to the receptor.

MT1PEX-F creates a certain distance between the MT1-MMP/TIMP-2 complex and the catalytic MT1-MMP so that the reaction of proMMP-2 with the enzyme for its activation is prevented.

It was previously reported that a catalytic domain-deleted form of MT1-MMP was generated in HT1080 cells (Stanton, H. et al., J. Cell Sci., 111: 2789-2798, 1998; Lehti, K. et al., Biochem. J., 334: 345-353, 1998). This product is considered to be the result of a specific cleavage at the Ala²⁵⁵-Lle bond located at the end of the MT1-MMP catalytic domain by the action of either MT1-MMP itself or MMP-2 activated by MT1-MMP.

Since this deleted form retains PEX, it may have a similar effect to MT1PEX-F. Thus, this processing not only removes the activity of MT1-MMP, but may also inhibit adjacent MT1-MMP activity.

In accordance with the present invention, it has been revealed that PEX plays an important role in MT1-MMP dimer formation and further functions in facilitating effective activation of proMMP-2 on the cell surface. Therefore, it is conceivable that PEX is a uniquely preserved domain in matrixins and a general device involved in interactions with molecules that determine the fate of the enzyme in biological systems.

Accordingly, roles of proMMP-2 activation or MT1-MMP dimer formation in specific biological systems can be tested using MT1PEX-F. In addition, since Matrigel invasion activity owned by HT1080 cells capable of producing MT1-MMP and MMP-2 (Strongin, A. Y. et al., J. Biol. Chem., 270: 5331-5338, 1995) is effectively inhibited by MT1PEX-F, it can be utilized in developing and investigating means with intent to suppress and/or inhibit the invasion and/or metastasis of cancer cells. Furthermore, it has been shown that MT1PEX-F blocks the MT1-MMP functions without affecting MT1-MMP proteolytic activity. MT1-F/tfk having NGFR transmembrane/cytoplasmic domains forms a dimer complex, and this complex formation is inhibited by MT1PEX-F, suggesting that a sequence property of the transmembrane/cytoplasmic domains is not important for dimer formation. Moreover, GPI-anchored MT1PEX-F inhibits the activation of proMMP-2. Thus, the inhibitory efficacy of MT1PEX-F is truly a result of PEX interaction.

### Industrial Applicability

In accordance with the present invention, the activation mechanisms of proMMP-2 by MT1-MMP existing on the cell surface have been disclosed, thereby enabling the investigation and development of substances active in affecting the activation of proMMP-2. Moreover, the activation of proMMP-2 by MT1-MMP existing on the cell surface is suspected to participating in a variety of physiological or biological phenomena, for example, disorders, abnormal conditions and/or diseases in living organisms, including invasion or metastasis of cancer cells. Therefore, it paves the way to serve in prophylaxis and/or therapy of these phenomena.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. A membrane-type 1 matrix metalloproteinase (MT1-MMP) complex formation inhibitor which inhibits the formation of a complex comprised of plural MT1-MMPs.

2. A proMMP-2 activation inhibitor which inhibits the formation of a complex comprised of plural MT1-MMPs.

3. A blocker for cell migration, invasion and/or metastasis which inhibits the activation of proMMP-2 through inhibiting the formation of a complex comprised of plural MT1-MMPs.

4. The blocker according to claim 3, wherein the cell is a cancer cell.

5. The inhibitor or blocker according to any of claims 1 through 4, wherein the complex comprised of plural MT1-MMPs is an MT1-MMP homophilic dimer.

6. The inhibitor or blocker according to any of claims 1 through 5, wherein the complex formation is executed at least with MT1-MMP existing on a cell surface.

7. A method for inhibiting MT1-MMP complex formation which comprises inhibiting the formation of a complex comprised of plural MT1-MMPs.

8. A method for inhibiting the activation of proMMP-2 which comprises inhibiting the formation of a complex comprised of plural MT1-MMPs.

9. A method for blocking cell migration, invasion and/or metastasis which comprises inhibiting the activation of proMMP-2 through inhibiting the formation of a complex comprised of plural MT1-MMPs.

10. The method according to claim 9, wherein the cell is a cancer cell.

11. The method according to any of claims 7 through 10, wherein the complex comprised of plural MT1-MMPs is an MT1-MMP homophilic dimer.

12. The method according to any of claims 7 through 11, wherein the complex formation is executed at least with MT1-MMP existing on a cell surface.

13. A substance which is active in inhibiting the homophilic dimer formation of MT1-MMP that exists on a cell surface.

14. The substance according to claim 13 which is an MT1-MMP hemopexin-like domain (PEX) protein or a fragment thereof, or a substance which has substantially equivalent activity as compared to the same.

15. The substance according to claim 14, wherein the substance is from Cys³¹⁹ to Gly⁵³⁵ of Met plus MT1-MMP, or a protein having substantially equivalent activity as compared to the same.

16. A nucleic acid selected from the group consisting of:
(i) a nucleic acid coding for MT1-MMP PEX;
(ii) a nucleic acid coding for a fragment of MT1-MMP PEX;
(iii) a nucleic acid hybridizable to the nucleic acid as set forth in the above (i); and
(iv) a nucleic acid having substantially equivalent activity as compared to the nucleic acid as set forth in any of the above (i) through (iii).

17. The nucleic acid according to claim 16, wherein the nucleic acid is a chimeric molecule in which the transmembrane (TM)/cytoplasmic (CP) domains of MT1-MMP are substituted with nerve growth factor receptor (NGFR) TM/CP, or a molecule having substantially equivalent activity as compared to the same.

18. The nucleic acid according to claim 16, which is a nucleic acid coding for from Cys³¹⁹ to Gly⁵³⁵ of Met plus MT1-MMP, or a nucleic acid having substantially equivalent activity as compared to the same.

19. A vector comprising a nucleic acid according to any of claims 16 through 18.

20. A transformed cell comprising (i) a nucleic acid according to any of claims 16 through 18 or (ii) a vector according to claim 18.

21. The transformed cell according to claim 20 which expresses a chimeric molecule in which MT1-MMP TM/CP is substituted with NGFR TM/CP, or a molecule having substantially equivalent activity as compared to the same.

22. A gene therapeutic agent which comprises (i) a nucleic acid according to any of claims 16 or 18 or (ii) a vector according to claim 19.

23. An antibody against (i) an MT1-MMP PEX protein or a fragment thereof, or (ii) a polypeptide having substantially equivalent activity as compared to the same.

24. A substance capable of binding to MT1-MMP PEX.

25. The substance according to claim 22 which has MT1PEX activity or substantially equivalent activity as compared to MT1PEX.

26. A pharmaceutical or veterinary drug comprising the inhibitor or blocker according to any of claims 1 through 6, or the substance, nucleic acid, vector, transformed cell, agent or antibody according to any of clams 13 through 16, 18 through 20, and 22 through 25.

27. The pharmaceutical or veterinary drug according to claim 26 for suppressing and/or blocking cell migration, invasion and/or metastasis.

28. The pharmaceutical or veterinary drug according to claim 26, wherein the cell is a cancer cell.

29. A screening which comprises screening with using, as a marker, the formation of a complex comprised of plural MT1-MMPs for a substance which inhibits the formation of said complex comprised of said plural MT1-MMPs.

30. The screening according to claim 29, wherein the complex comprised of the plural MT1-MMPs is an MT1-MMP homophilic dimer.

31. The screening according to claim 29 or 30, wherein a chimeric molecule is used in which MT1-MMP transmembrane (TM)/cytoplasmic (CP) domains are substituted with nerve growth factor receptor (NGFR) TM/CP.

32. A substance which (i) inhibits the formation of a complex comprised of plural MT1-MMPs and (ii) is obtained by the screening according to any of claims 29 through 31.
